(19) Europäisches Patentamt
European Patent Office
Office européen des brevets

(11) **EP 3 943 511 A1**

(12) **EUROPEAN PATENT APPLICATION**
published in accordance with Art. 153(4) EPC

(43) Date of publication:
**26.01.2022 Bulletin 2022/04**

(21) Application number: **20772799.1**

(22) Date of filing: **17.03.2020**

(51) International Patent Classification (IPC):
*C07K 16/46* [(2006.01)]   *C07K 16/22* [(2006.01)]
*C07K 16/28* [(2006.01)]   *C12N 15/13* [(2006.01)]
*C12N 5/10* [(2006.01)]   *A61K 39/395* [(2006.01)]
*A61P 35/00* [(2006.01)]   *A61P 27/02* [(2006.01)]

(52) Cooperative Patent Classification (CPC):
**A61K 39/395; A61P 27/02; A61P 35/00;
C07K 16/22; C07K 16/28; C07K 16/46; C12N 5/10**

(86) International application number:
**PCT/CN2020/079690**

(87) International publication number:
**WO 2020/187202 (24.09.2020 Gazette 2020/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.03.2019 CN 201910204246**

(71) Applicants:
• **Jiangsu Hengrui Medicine Co., Ltd.
Lianyungang, Jiangsu 222047 (CN)**
• **Shanghai Hengrui Pharmaceutical Co., Ltd.
Shanghai 200245 (CN)**

(72) Inventors:
• **YING, Hua
Shanghai 200245 (CN)**
• **SHI, Jinping
Shanghai 200245 (CN)**
• **MAO, Langyong
Shanghai 200245 (CN)**
• **GE, Hu
Shanghai 200245 (CN)**
• **YANG, Xiaoying
Shanghai 200245 (CN)**
• **TAO, Weikang
Shanghai 200245 (CN)**

(74) Representative: **Sonzogni, Laura Gabriella et al
Dragotti & Associati S.r.l.
Via Nino Bixio, 7
20129 Milano (IT)**

(54) **BISPECIFIC ANTIBODY SPECIFICALLY BOUND TO VEGF AND ANG2**

(57) The present disclosure provides a bispecific antibody specifically bound to VEGF and ANG2, comprising an anti-VEGF antibody or antigen-binding fragment thereof that specifically binds to VEGF, and an anti-ANG2 single-domain antibody that specifically binds to ANG2, wherein the anti-ANG2 single domain antibody is directly or indirectly connected to the anti-VEGF antibody or an antigen-binding fragment thereof. The present disclosure further provides an anti-ANG2 single domain antibody and an antigen-binding fragment thereof, as well as a preparation and application of said antibody.

```
<110>

<120>    VEGFANG2

<130>    702011CPCT

<160>    59

<170>    SIPOSequenceListing 1.0

<210>    1
<211>    723
<212>    PRT
<213>    (Artificial Sequence)

<220>
<221>    PEPTIDE
<223>    FcANG2


<400>    1
Tyr Asn Asn Phe Arg Lys Ser Met Asp Ser Ile Gly Lys Lys Gln Tyr
1               5                   10                  15
Gln Val Gln His Gly Ser Cys Ser Tyr Thr Phe Leu Leu Pro Glu Met
            20                  25                  30
Asp Asn Cys Arg Ser Ser Ser Ser Pro Tyr Val Ser Asn Ala Val Gln
        35                  40                  45
Arg Asp Ala Pro Leu Glu Tyr Asp Asp Ser Val Gln Arg Leu Gln Val
    50                  55                  60
Leu Glu Asn Ile Met Glu Asn Asn Thr Gln Trp Leu Met Lys Leu Glu
65                  70                  75                  80
Asn Tyr Ile Gln Asp Asn Met Lys Lys Glu Met Val Glu Ile Gln Gln
            85                  90                  95
Asn Ala Val Gln Asn Gln Thr Ala Val Met Ile Glu Ile Gly Thr Asn
            100                 105                 110
Leu Leu Asn Gln Thr Ala Glu Gln Thr Arg Lys Leu Thr Asp Val Glu
        115                 120                 125
Ala Gln Val Leu Asn Gln Thr Thr Arg Leu Glu Leu Gln Leu Leu Glu
    130                 135                 140
His Ser Leu Ser Thr Asn Lys Leu Glu Lys Gln Ile Leu Asp Gln Thr
145                 150                 155                 160
Ser Glu Ile Asn Lys Leu Gln Asp Lys Asn Ser Phe Leu Glu Lys Lys
                165                 170                 175
Val Leu Ala Met Glu Asp Lys His Ile Ile Gln Leu Gln Ser Ile Lys
            180                 185                 190
Glu Glu Lys Asp Gln Leu Gln Val Leu Val Ser Lys Gln Asn Ser Ile
            195                 200                 205
Ile Glu Glu Leu Glu Lys Lys Ile Val Thr Ala Thr Val Asn Asn Ser
        210                 215                 220
Val Leu Gln Lys Gln Gln His Asp Leu Met Glu Thr Val Asn Asn Leu
225                 230                 235                 240
Leu Thr Met Met Ser Thr Ser Asn Ser Ala Lys Asp Pro Thr Val Ala
                245                 250                 255
Lys Glu Glu Gln Ile Ser Phe Arg Asp Cys Ala Glu Val Phe Lys Ser
                260                 265                 270
Gly His Thr Thr Asn Gly Ile Tyr Thr Leu Thr Phe Pro Asn Ser Thr
            275                 280                 285
Glu Glu Ile Lys Ala Tyr Cys Asp Met Glu Ala Gly Gly Gly Gly Trp
        290                 295                 300
Thr Ile Ile Gln Arg Arg Glu Asp Gly Ser Val Asp Phe Gln Arg Thr
```

```
            305                      310                      315                      320
      Trp Lys Glu Tyr Lys Val Gly Phe Gly Asn Pro Ser Gly Glu Tyr Trp
                        325                      330                      335
      Leu Gly Asn Glu Phe Val Ser Gln Leu Thr Asn Gln Gln Arg Tyr Val
                        340                      345                      350
      Leu Lys Ile His Leu Lys Asp Trp Glu Gly Asn Glu Ala Tyr Ser Leu
                        355                      360                      365
      Tyr Glu His Phe Tyr Leu Ser Ser Glu Glu Leu Asn Tyr Arg Ile His
          370                      375                      380
      Leu Lys Gly Leu Thr Gly Thr Ala Gly Lys Ile Ser Ser Ile Ser Gln
      385                      390                      395                      400
      Pro Gly Asn Asp Phe Ser Thr Lys Asp Gly Asp Asn Asp Lys Cys Ile
                        405                      410                      415
      Cys Lys Cys Ser Gln Met Leu Thr Gly Gly Trp Trp Phe Asp Ala Cys
                        420                      425                      430
      Gly Pro Ser Asn Leu Asn Gly Met Tyr Tyr Pro Gln Arg Gln Asn Thr
                        435                      440                      445
      Asn Lys Phe Asn Gly Ile Lys Trp Tyr Tyr Trp Lys Gly Ser Gly Tyr
          450                      455                      460
      Ser Leu Lys Ala Thr Thr Met Met Ile Arg Pro Ala Asp Phe Asp Ile
      465                      470                      475                      480
      Glu Gly Arg Met Asp Glu Pro Lys Ser Ser Asp Lys Thr His Thr Cys
                        485                      490                      495
      Pro Pro Cys Pro Ala Pro Glu Leu Leu Gly Gly Pro Ser Val Phe Leu
                        500                      505                      510
      Phe Pro Pro Lys Pro Lys Asp Thr Leu Met Ile Ser Arg Thr Pro Glu
                        515                      520                      525
      Val Thr Cys Val Val Val Asp Val Ser His Glu Asp Pro Glu Val Lys
                        530                      535                      540
      Phe Asn Trp Tyr Val Asp Gly Val Glu Val His Asn Ala Lys Thr Lys
      545                      550                      555                      560
      Pro Arg Glu Glu Gln Tyr Asn Ser Thr Tyr Arg Val Val Ser Val Leu
                        565                      570                      575
      Thr Val Leu His Gln Asp Trp Leu Asn Gly Lys Glu Tyr Lys Cys Lys
                        580                      585                      590
      Val Ser Asn Lys Ala Leu Pro Ala Pro Ile Glu Lys Thr Ile Ser Lys
                        595                      600                      605
      Ala Lys Gly Gln Pro Arg Glu Pro Gln Val Tyr Thr Leu Pro Pro Ser
          610                      615                      620
      Arg Asp Glu Leu Thr Lys Asn Gln Val Ser Leu Thr Cys Leu Val Lys
      625                      630                      635                      640
      Gly Phe Tyr Pro Ser Asp Ile Ala Val Glu Trp Glu Ser Asn Gly Gln
                        645                      650                      655
      Pro Glu Asn Asn Tyr Lys Thr Thr Pro Pro Val Leu Asp Ser Asp Gly
                        660                      665                      670
      Ser Phe Phe Leu Tyr Ser Lys Leu Thr Val Asp Lys Ser Arg Trp Gln
                        675                      680                      685
      Gln Gly Asn Val Phe Ser Cys Ser Val Met His Glu Ala Leu His Asn
          690                      695                      700
      His Tyr Thr Gln Lys Ser Leu Ser Leu Ser Pro Gly Lys His His His
      705                      710                      715                      720
      His His His
```

```
<210>   2
<211>   953
<212>   PRT
<213>   (Artificial Sequence)

<220>
<221>   PEPTIDE
<223>   FcTie2
```

```
<400>   2
Ala Met Asp Leu Ile Leu Ile Asn Ser Leu Pro Leu Val Ser Asp Ala
1               5                   10                  15
Glu Thr Ser Leu Thr Cys Ile Ala Ser Gly Trp Arg Pro His Glu Pro
            20                  25                  30
Ile Thr Ile Gly Arg Asp Phe Glu Ala Leu Met Asn Gln His Gln Asp
        35                  40                  45
Pro Leu Glu Val Thr Gln Asp Val Thr Arg Glu Trp Ala Lys Lys Val
    50                  55                  60
Val Trp Lys Arg Glu Lys Ala Ser Lys Ile Asn Gly Ala Tyr Phe Cys
65                  70                  75                  80
Glu Gly Arg Val Arg Gly Glu Ala Ile Arg Ile Arg Thr Met Lys Met
                85                  90                  95
Arg Gln Gln Ala Ser Phe Leu Pro Ala Thr Leu Thr Met Thr Val Asp
            100                 105                 110
Lys Gly Asp Asn Val Asn Ile Ser Phe Lys Lys Val Leu Ile Lys Glu
            115                 120                 125
Glu Asp Ala Val Ile Tyr Lys Asn Gly Ser Phe Ile His Ser Val Pro
    130                 135                 140
Arg His Glu Val Pro Asp Ile Leu Glu Val His Leu Pro His Ala Gln
145                 150                 155                 160
Pro Gln Asp Ala Gly Val Tyr Ser Ala Arg Tyr Ile Gly Gly Asn Leu
            165                 170                 175
Phe Thr Ser Ala Phe Thr Arg Leu Ile Val Arg Arg Cys Glu Ala Gln
            180                 185                 190
Lys Trp Gly Pro Glu Cys Asn His Leu Cys Thr Ala Cys Met Asn Asn
            195                 200                 205
Gly Val Cys His Glu Asp Thr Gly Glu Cys Ile Cys Pro Pro Gly Phe
210                 215                 220
Met Gly Arg Thr Cys Glu Lys Ala Cys Glu Leu His Thr Phe Gly Arg
225                 230                 235                 240
Thr Cys Lys Glu Arg Cys Ser Gly Gln Glu Gly Cys Lys Ser Tyr Val
                245                 250                 255
Phe Cys Leu Pro Asp Pro Tyr Gly Cys Ser Cys Ala Thr Gly Trp Lys
            260                 265                 270
Gly Leu Gln Cys Asn Glu Ala Cys His Pro Gly Phe Tyr Gly Pro Asp
            275                 280                 285
Cys Lys Leu Arg Cys Ser Cys Asn Asn Gly Glu Met Cys Asp Arg Phe
    290                 295                 300
Gln Gly Cys Leu Cys Ser Pro Gly Trp Gln Gly Leu Gln Cys Glu Arg
305                 310                 315                 320
Glu Gly Ile Pro Arg Met Thr Pro Lys Ile Val Asp Leu Pro Asp His
            325                 330                 335
Ile Glu Val Asn Ser Gly Lys Phe Asn Pro Ile Cys Lys Ala Ser Gly
            340                 345                 350
Trp Pro Leu Pro Thr Asn Glu Glu Met Thr Leu Val Lys Pro Asp Gly
            355                 360                 365
Thr Val Leu His Pro Lys Asp Phe Asn His Thr Asp His Phe Ser Val
    370                 375                 380
Ala Ile Phe Thr Ile His Arg Ile Leu Pro Pro Asp Ser Gly Val Trp
385                 390                 395                 400
Val Cys Ser Val Asn Thr Val Ala Gly Met Val Glu Lys Pro Phe Asn
            405                 410                 415
Ile Ser Val Lys Val Leu Pro Lys Pro Leu Asn Ala Pro Asn Val Ile
            420                 425                 430
Asp Thr Gly His Asn Phe Ala Val Ile Asn Ile Ser Ser Glu Pro Tyr
        435                 440                 445
Phe Gly Asp Gly Pro Ile Lys Ser Lys Lys Leu Leu Tyr Lys Pro Val
    450                 455                 460
Asn His Tyr Glu Ala Trp Gln His Ile Gln Val Thr Asn Glu Ile Val
465                 470                 475                 480
Thr Leu Asn Tyr Leu Glu Pro Arg Thr Glu Tyr Glu Leu Cys Val Gln
```

```
                        485                     490                     495
     Leu Val Arg Arg Gly Glu Gly Gly Glu Gly His Pro Gly Pro Val Arg
                    500                     505                     510
     Arg Phe Thr Thr Ala Ser Ile Gly Leu Pro Pro Pro Arg Gly Leu Asn
                    515                     520                     525
     Leu Leu Pro Lys Ser Gln Thr Thr Leu Asn Leu Thr Trp Gln Pro Ile
                    530                     535                     540
     Phe Pro Ser Ser Glu Asp Asp Phe Tyr Val Glu Val Glu Arg Arg Ser
     545                 550                     555                     560
     Val Gln Lys Ser Asp Gln Gln Asn Ile Lys Val Pro Gly Asn Leu Thr
                    565                     570                     575
     Ser Val Leu Leu Asn Asn Leu His Pro Arg Glu Gln Tyr Val Val Arg
                    580                     585                     590
     Ala Arg Val Asn Thr Lys Ala Gln Gly Glu Trp Ser Glu Asp Leu Thr
                    595                     600                     605
     Ala Trp Thr Leu Ser Asp Ile Leu Pro Pro Gln Pro Glu Asn Ile Lys
                    610                     615                     620
     Ile Ser Asn Ile Thr His Ser Ser Ala Val Ile Ser Trp Thr Ile Leu
     625                 630                     635                     640
     Asp Gly Tyr Ser Ile Ser Ser Ile Thr Ile Arg Tyr Lys Val Gln Gly
                    645                     650                     655
     Lys Asn Glu Asp Gln His Val Asp Val Lys Ile Lys Asn Ala Thr Ile
                    660                     665                     670
     Thr Gln Tyr Gln Leu Lys Gly Leu Glu Pro Glu Thr Ala Tyr Gln Val
                    675                     680                     685
     Asp Ile Phe Ala Glu Asn Asn Ile Gly Ser Ser Asn Pro Ala Phe Ser
                    690                     695                     700
     His Glu Leu Val Thr Leu Pro Glu Ser Gln Ala Pro Ala Asp Leu Gly
     705                 710                     715                     720
     Gly Gly Lys Glu Pro Lys Ser Cys Asp Lys Thr His Thr Cys Pro Pro
                    725                     730                     735
     Cys Pro Ala Pro Glu Leu Leu Gly Gly Pro Ser Val Phe Leu Phe Pro
                    740                     745                     750
     Pro Lys Pro Lys Asp Thr Leu Met Ile Ser Arg Thr Pro Glu Val Thr
                    755                     760                     765
     Cys Val Val Val Asp Val Ser His Glu Asp Pro Glu Val Lys Phe Asn
     770                 775                     780
     Trp Tyr Val Asp Gly Val Glu Val His Asn Ala Lys Thr Lys Pro Arg
     785                 790                     795                     800
     Glu Glu Gln Tyr Asn Ser Thr Tyr Arg Val Val Ser Val Leu Thr Val
                    805                     810                     815
     Leu His Gln Asp Trp Leu Asn Gly Lys Glu Tyr Lys Cys Lys Val Ser
                    820                     825                     830
     Asn Lys Ala Leu Pro Ala Pro Ile Glu Lys Thr Ile Ser Lys Ala Lys
                    835                     840                     845
     Gly Gln Pro Arg Glu Pro Gln Val Tyr Thr Leu Pro Pro Ser Arg Asp
                    850                     855                     860
     Glu Leu Thr Lys Asn Gln Val Ser Leu Thr Cys Leu Val Lys Gly Phe
     865                 870                     875                     880
     Tyr Pro Ser Asp Ile Ala Val Glu Trp Glu Ser Asn Gly Gln Pro Glu
                    885                     890                     895
     Asn Asn Tyr Lys Thr Thr Pro Pro Val Leu Asp Ser Asp Gly Ser Phe
                    900                     905                     910
     Phe Leu Tyr Ser Lys Leu Thr Val Asp Lys Ser Arg Trp Gln Gln Gly
                    915                     920                     925
     Asn Val Phe Ser Cys Ser Val Met His Glu Ala Leu His Asn His Tyr
                    930                     935                     940
     Thr Gln Lys Ser Leu Ser Leu Ser Pro
     945                 950
```

<210> 3
<211> 115
<212> PRT

<213> (Artificial Sequence)

<220>
<221> DOMAIN
<223> nano14

<400> 3
Asp Val Gln Leu Gln Glu Ser Gly Gly Gly Leu Val Gln Pro Gly Gly
1                   5                   10                  15
Ser Leu Arg Leu Ser Cys Ala Ala Ser Gly Phe Thr Phe Asp Asp Phe
            20                  25                  30
Gly Met Ser Trp Val Arg Gln Ala Pro Gly Lys Gly Leu Glu Trp Val
            35                  40                  45
Ser Ser Ile Thr Trp Asn Gly Gly Ser Thr Tyr Tyr Ala Asp Ser Val
            50                  55                  60
Lys Gly Arg Phe Thr Ile Ser Arg Asp Asn Ala Lys Asn Thr Val Tyr
65                  70                  75                  80
Leu Gln Met Asn Ser Leu Lys Pro Glu Asp Thr Ala Ile Tyr Tyr Cys
                85                  90                  95
Asn Ala Asp His Pro Gln Gly Tyr Trp Gly Gln Gly Thr Gln Val Thr
                100                 105                 110
Val Ser Ser
            115

<210> 4
<211> 115
<212> PRT
<213> (Artificial Sequence)

<220>
<221> DOMAIN
<223> nano15

<400> 4
Asp Val Gln Leu Gln Glu Ser Gly Gly Gly Leu Val Gln Pro Gly Gly
1                   5                   10                  15
Ser Leu Arg Leu Ser Cys Ala Ala Ser Gly Phe Thr Phe Asn Ser Tyr
            20                  25                  30
Ala Met Ser Trp Val Arg Gln Ala Pro Gly Lys Gly Leu Glu Trp Val
            35                  40                  45
Ser Thr Ile Asn Ser Gly Gly Gly Arg Thr Gly Tyr Ala Asp Ser Val
            50                  55                  60
Lys Gly Arg Phe Thr Ile Ser Arg Asp Asn Ala Lys Asn Thr Leu Tyr
65                  70                  75                  80
Leu Gln Met Asn Ser Leu Lys Pro Glu Asp Thr Ala Ile Tyr Tyr Cys
                85                  90                  95
Asn Ala Asp His Pro Gln Gly Tyr Trp Gly Gln Gly Thr Gln Val Thr
                100                 105                 110
Val Ser Ser
            115

<210> 5
<211> 5
<212> PRT
<213> (Artificial Sequence)

<220>
<221> DOMAIN
<223> nano14 CDR1

<400> 5
Asp Phe Gly Met Ser
1               5


<210> 6
<211> 17
<212> PRT
<213> (Artificial Sequence)


<220>
<221> DOMAIN
<223> nano14 CDR2


<400> 6
Ser Ile Thr Trp Asn Gly Gly Ser Thr Tyr Tyr Ala Asp Ser Val Lys
1               5                   10                  15
Gly


<210> 7
<211> 6
<212> PRT
<213> (Artificial Sequence)


<220>
<221> DOMAIN
<223> nano14 CDR3


<400> 7
Asp His Pro Gln Gly Tyr
1               5


<210> 8
<211> 5
<212> PRT
<213> (Artificial Sequence)


<220>
<221> DOMAIN
<223> nano15 CDR1


<400> 8
Ser Tyr Ala Met Ser
1               5


<210> 9
<211> 17
<212> PRT
<213> (Artificial Sequence)


<220>
<221> DOMAIN
<223> nano15 CDR2


<400> 9
Thr Ile Asn Ser Gly Gly Gly Arg Thr Gly Tyr Ala Asp Ser Val Lys
1               5                   10                  15
Gly

<210> 10
<211> 17
<212> PRT
<213> (Artificial Sequence)

<220>
<221> DOMAIN
<223> nano14.1 CDR2


<400> 10
Ser Ile Thr Trp Gly Gly Gly Ser Thr Tyr Tyr Ala Asp Ser Val Lys
1               5                   10                  15
Gly


<210> 11
<211> 17
<212> PRT
<213> (Artificial Sequence)

<220>
<221> DOMAIN
<223> nano14.2  CDR2


<400> 11
Ser Ile Thr Trp Ser Gly Gly Ser Thr Tyr Tyr Ala Asp Ser Val Lys
1               5                   10                  15
Gly


<210> 12
<211> 115
<212> PRT
<213> (Artificial Sequence)

<220>
<221> DOMAIN
<223> nano14.1


<400> 12
Asp Val Gln Leu Gln Glu Ser Gly Gly Gly Leu Val Gln Pro Gly Gly
1               5                   10                  15
Ser Leu Arg Leu Ser Cys Ala Ala Ser Gly Phe Thr Phe Asp Asp Phe
            20                  25                  30
Gly Met Ser Trp Val Arg Gln Ala Pro Gly Lys Gly Leu Glu Trp Val
            35                  40                  45
Ser Ser Ile Thr Trp Gly Gly Gly Ser Thr Tyr Tyr Ala Asp Ser Val
        50                  55                  60
Lys Gly Arg Phe Thr Ile Ser Arg Asp Asn Ala Lys Asn Thr Val Tyr
65                  70                  75                  80
Leu Gln Met Asn Ser Leu Lys Pro Glu Asp Thr Ala Ile Tyr Tyr Cys
                85                  90                  95
Asn Ala Asp His Pro Gln Gly Tyr Trp Gly Gln Gly Thr Gln Val Thr
            100                 105                 110
Val Ser Ser
        115


<210> 13
<211> 115

```
<212>    PRT
<213>    (Artificial Sequence)


<220>
<221>    DOMAIN
<223>    nano14.2


<400>    13
Asp Val Gln Leu Gln Glu Ser Gly Gly Gly Leu Val Gln Pro Gly Gly
1               5                   10                  15
Ser Leu Arg Leu Ser Cys Ala Ala Ser Gly Phe Thr Phe Asp Asp Phe
            20                  25                  30
Gly Met Ser Trp Val Arg Gln Ala Pro Gly Lys Gly Leu Glu Trp Val
            35                  40                  45
Ser Ser Ile Thr Trp Ser Gly Gly Ser Thr Tyr Tyr Ala Asp Ser Val
        50                  55                  60
Lys Gly Arg Phe Thr Ile Ser Arg Asp Asn Ala Lys Asn Thr Val Tyr
65                  70                  75                  80
Leu Gln Met Asn Ser Leu Lys Pro Glu Asp Thr Ala Ile Tyr Tyr Cys
                85                  90                  95
Asn Ala Asp His Pro Gln Gly Tyr Trp Gly Gln Gly Thr Gln Val Thr
                100                 105                 110
Val Ser Ser
        115


<210>    14
<211>    5
<212>    PRT
<213>    (Artificial Sequence)


<220>
<221>    UNSURE
<223>    CDR1


<220>
<221>    UNSURE
<222>    (1)..(1)
<223>    XaaAspSer.


<220>
<221>    UNSURE
<222>    (2)..(2)
<223>    XaaPheTyr.


<220>
<221>    UNSURE
<222>    (3)..(3)
<223>    XaaGlyAla.


<400>    14
Xaa Xaa Xaa Met Ser
1               5


<210>    15
<211>    17
<212>    PRT
<213>    (Artificial Sequence)
```

```
<220>
<221>   DOMAIN
<223>   CDR2


<220>
<221>   UNSURE
<222>   (1)..(1)
<223>   XaaSerThr.


<220>
<221>   UNSURE
<222>   (3)..(3)
<223>   XaaThrAsn.


<220>
<221>   UNSURE
<222>   (4)..(4)
<223>   XaaTrpSer.


<220>
<221>   UNSURE
<222>   (5)..(5)
<223>   XaaAsn,GlySer.


<220>
<221>   UNSURE
<222>   (8)..(8)
<223>   XaaArgSer.


<220>
<221>   UNSURE
<222>   (10)..(10)
<223>   XaaTyrGly.


<400>   15
Xaa Ile Xaa Xaa Xaa Gly Gly Xaa Thr Xaa Tyr Ala Asp Ser Val Lys
1               5                   10                  15
Gly


<210>   16
<211>   115
<212>   PRT
<213>   (Artificial Sequence)

<220>
<221>   DOMAIN
<223>   ANG2


<220>
<221>   UNSURE
<222>   (30)..(30)
<223>   XaaAspAsn.
```

```
<220>
<221>  UNSURE
<222>  (31)..(31)
<223>  XaaAspSer.


<220>
<221>  UNSURE
<222>  (32)..(32)
<223>  XaaPheTyr.


<220>
<221>  UNSURE
<222>  (33)..(33)
<223>  XaaGlyAla.


<220>
<221>  UNSURE
<222>  (50)..(50)
<223>  XaaSerThr.


<220>
<221>  UNSURE
<222>  (52)..(52)
<223>  XaaThrAsn.


<220>
<221>  UNSURE
<222>  (53)..(53)
<223>  XaaTrpSer.


<220>
<221>  UNSURE
<222>  (54)..(54)
<223>  XaaAsn,GlySer.


<220>
<221>  UNSURE
<222>  (57)..(57)
<223>  XaaArgSer.


<220>
<221>  UNSURE
<222>  (59)..(59)
<223>  XaaTyrGly.


<220>
<221>  UNSURE
<222>  (79)..(79)
<223>  XaaValLeu.


<400>  16
Asp Val Gln Leu Gln Glu Ser Gly Gly Gly Leu Val Gln Pro Gly Gly
1                   5                   10                  15
```

```
Ser Leu Arg Leu Ser Cys Ala Ala Ser Gly Phe Thr Phe Xaa Xaa Xaa
        20                  25                  30
Xaa Met Ser Trp Val Arg Gln Ala Pro Gly Lys Gly Leu Glu Trp Val
        35                  40                  45
Ser Xaa Ile Xaa Xaa Xaa Gly Xaa Thr Xaa Tyr Ala Asp Ser Val
        50                  55                  60
Lys Gly Arg Phe Thr Ile Ser Arg Asp Asn Ala Lys Asn Thr Xaa Tyr
65                  70                  75                  80
Leu Gln Met Asn Ser Leu Lys Pro Glu Asp Thr Ala Ile Tyr Tyr Cys
                85                  90                  95
Asn Ala Asp His Pro Gln Gly Tyr Trp Gly Gln Gly Thr Gln Val Thr
            100                 105                 110
Val Ser Ser
        115
```

```
<210>   17
<211>   115
<212>   PRT
<213>   (Artificial Sequence)

<220>
<221>   DOMAIN
<223>   hu14.A


<400>   17
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Phe
        20                  25                  30
Gly Met Ser Trp Val Arg Gln Ala Pro Gly Lys Gly Leu Glu Trp Val
        35                  40                  45
Ser Ser Ile Thr Trp Asn Gly Gly Ser Thr Tyr Tyr Ala Asp Ser Val
        50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Asn Ala Asp His Pro Gln Gly Tyr Trp Gly Gln Gly Thr Thr Val Thr
            100                 105                 110
Val Ser Ser
        115
```

```
<210>   18
<211>   115
<212>   PRT
<213>   (Artificial Sequence)

<220>
<221>   DOMAIN
<223>   hu14.1A


<400>   18
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Phe
        20                  25                  30
Gly Met Ser Trp Val Arg Gln Ala Pro Gly Lys Gly Leu Glu Trp Val
        35                  40                  45
Ser Ser Ile Thr Trp Gly Gly Ser Thr Tyr Tyr Ala Asp Ser Val
        50                  55                  60
Lys Gly Arg Phe Thr Ile Ser Arg Asp Asn Ser Lys Asn Thr Leu Tyr
```

```
                      65                       70                       75                       80
Leu Gln Met Asn Ser Leu Arg Ala Glu Asp Thr Ala Val Tyr Tyr Cys
                         85                       90                       95
Asn Ala Asp His Pro Gln Gly Tyr Trp Gly Gln Gly Thr Thr Val Thr
                         100                      105                      110
Val Ser Ser
                         115


<210>   19
<211>   115
<212>   PRT
<213>   (Artificial Sequence)


<220>
<221>   DOMAIN
<223>   hu14.2A


<400>   19
Glu Val Gln Leu Val Glu Ser Gly Gly Gly Leu Val Gln Pro Gly Gly
1                       5                       10                       15
Ser Leu Arg Leu Ser Cys Ala Ala Ser Gly Phe Thr Phe Ser Asp Phe
                         20                       25                       30
Gly Met Ser Trp Val Arg Gln Ala Pro Gly Lys Gly Leu Glu Trp Val
                         35                       40                       45
Ser Ser Ile Thr Trp Ser Gly Gly Ser Thr Tyr Tyr Ala Asp Ser Val
                         50                       55                       60
Lys Gly Arg Phe Thr Ile Ser Arg Asp Asn Ser Lys Asn Thr Leu Tyr
65                       70                       75                       80
Leu Gln Met Asn Ser Leu Arg Ala Glu Asp Thr Ala Val Tyr Tyr Cys
                         85                       90                       95
Asn Ala Asp His Pro Gln Gly Tyr Trp Gly Gln Gly Thr Thr Val Thr
                         100                      105                      110
Val Ser Ser
                         115


<210>   20
<211>   115
<212>   PRT
<213>   (Artificial Sequence)


<220>
<221>   DOMAIN
<223>   hu14.1B


<400>   20
Glu Val Gln Leu Val Glu Ser Gly Gly Gly Leu Val Gln Pro Gly Gly
1                       5                       10                       15
Ser Leu Arg Leu Ser Cys Ala Ala Ser Gly Phe Thr Phe Ser Asp Phe
                         20                       25                       30
Gly Met Ser Trp Val Arg Gln Ala Pro Gly Lys Gly Leu Glu Trp Val
                         35                       40                       45
Ser Ser Ile Thr Trp Gly Gly Gly Ser Thr Tyr Tyr Ala Asp Ser Val
                         50                       55                       60
Lys Gly Arg Phe Thr Ile Ser Arg Asp Asn Ser Lys Asn Thr Leu Tyr
65                       70                       75                       80
Leu Gln Met Asn Ser Leu Lys Pro Glu Asp Thr Ala Val Tyr Tyr Cys
                         85                       90                       95
Asn Ala Asp His Pro Gln Gly Tyr Trp Gly Gln Gly Thr Thr Val Thr
                         100                      105                      110
Val Ser Ser
                         115
```

<210> 21
<211> 115
<212> PRT
<213> (Artificial Sequence)

<220>
<221> DOMAIN
<223> hu14.2B

<400> 21

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Phe
            20                  25                  30
Gly Met Ser Trp Val Arg Gln Ala Pro Gly Lys Gly Leu Glu Trp Val
            35                  40                  45
Ser Ser Ile Thr Trp Ser Gly Gly Ser Thr Tyr Tyr Ala Asp Ser Val
        50                  55                  60
Lys Gly Arg Phe Thr Ile Ser Arg Asp Asn Ser Lys Asn Thr Leu Tyr
65                  70                  75                  80
Leu Gln Met Asn Ser Leu Lys Pro Glu Asp Thr Ala Val Tyr Tyr Cys
                85                  90                  95
Asn Ala Asp His Pro Gln Gly Tyr Trp Gly Gln Gly Thr Thr Val Thr
                100                 105                 110
Val Ser Ser
            115
```

<210> 22
<211> 115
<212> PRT
<213> (Artificial Sequence)

<220>
<221> DOMAIN
<223> hu15.A

<400> 22

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
            35                  40                  45
Ser Thr Ile Asn Ser Gly Gly Gly Arg Thr Gly Tyr Ala Asp Ser Val
        50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asp His Pro Gln Gly Tyr Trp Gly Gln Gly Thr Thr Val Thr
                100                 105                 110
Val Ser Ser
            115
```

<210> 23
<211> 115
<212> PRT
<213> (Artificial Sequence)

<220>

<221> DOMAIN
<223> hu15.B


<400> 23

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Thr Ile Asn Ser Gly Gly Gly Arg Thr Gly Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Asn Ala Asp His Pro Gln Gly Tyr Trp Gly Gln Gly Thr Thr Val Thr
                100                 105                 110
Val Ser Ser
            115
```

<210> 24
<211> 115
<212> PRT
<213> (Artificial Sequence)

<220>
<221> DOMAIN
<223> hu15.C


<400> 24

```
Glu Val Gln Leu Gln Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Thr Ile Asn Ser Gly Gly Gly Arg Thr Gly Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Asn Ala Asp His Pro Gln Gly Tyr Trp Gly Gln Gly Thr Thr Val Thr
                100                 105                 110
Val Ser Ser
            115
```

<210> 25
<211> 115
<212> PRT
<213> (Artificial Sequence)

<220>
<221> DOMAIN
<223> hu15.D


<400> 25

```
Glu Val Gln Leu Gln Glu Ser Gly Gly Gly Leu Val Gln Pro Gly Gly
1               5                   10                  15
```

56

```
       Ser Leu Arg Leu Ser Cys Ala Ala Ser Gly Phe Thr Phe Asn Ser Tyr
               20              25              30
       Ala Met Ser Trp Val Arg Gln Ala Pro Gly Lys Gly Leu Glu Trp Val
               35              40              45
       Ser Thr Ile Asn Ser Gly Gly Gly Arg Thr Gly Tyr Ala Asp Ser Val
               50              55              60
       Lys Gly Arg Phe Thr Ile Ser Arg Asp Asn Ser Lys Asn Thr Leu Tyr
       65              70              75              80
       Leu Gln Met Asn Ser Leu Arg Ala Glu Asp Thr Ala Val Tyr Tyr Cys
                       85              90              95
       Asn Ala Asp His Pro Gln Gly Tyr Trp Gly Gln Gly Thr Thr Val Thr
                       100             105             110
       Val Ser Ser
                       115


       <210>  26
       <211>  115
       <212>  PRT
       <213>  (Artificial Sequence)


       <220>
       <221>  DOMAIN
       <223>  hu15.E


       <400>  26
       Glu Val Gln Leu Val Glu Ser Gly Gly Gly Leu Val Gln Pro Gly Gly
       1               5               10              15
       Ser Leu Arg Leu Ser Cys Ala Ala Ser Gly Phe Thr Phe Ser Ser Tyr
               20              25              30
       Ala Met Ser Trp Val Arg Gln Ala Pro Gly Lys Gly Leu Glu Trp Val
               35              40              45
       Ser Thr Ile Asn Ser Gly Gly Gly Arg Thr Gly Tyr Ala Asp Ser Val
               50              55              60
       Lys Gly Arg Phe Thr Ile Ser Arg Asp Asn Ser Lys Asn Thr Leu Tyr
       65              70              75              80
       Leu Gln Met Asn Ser Leu Arg Pro Glu Asp Thr Ala Val Tyr Tyr Cys
                       85              90              95
       Asn Ala Asp His Pro Gln Gly Tyr Trp Gly Gln Gly Thr Thr Val Thr
                       100             105             110
       Val Ser Ser
                       115


       <210>  27
       <211>  115
       <212>  PRT
       <213>  (Artificial Sequence)


       <220>
       <221>  DOMAIN
       <223>


       <220>
       <221>  UNSURE
       <222>  (5)..(5)
       <223>  XaaValGln.


       <220>
       <221>  UNSURE
       <222>  (30)..(30)
       <223>  XaaSerAsn.
```

57

```
<220>
<221>  UNSURE
<222>  (31)..(31)
<223>  XaaAspSer.


<220>
<221>  UNSURE
<222>  (32)..(32)
<223>  XaaPheTyr.


<220>
<221>  UNSURE
<222>  (33)..(33)
<223>  XaaGlyAla.


<220>
<221>  UNSURE
<222>  (50)..(50)
<223>  XaaSerThr.


<220>
<221>  UNSURE
<222>  (52)..(52)
<223>  XaaThrAsn.


<220>
<221>  UNSURE
<222>  (53)..(53)
<223>  XaaTrpSer.


<220>
<221>  UNSURE
<222>  (54)..(54)
<223>  XaaAsn,GlySer.


<220>
<221>  UNSURE
<222>  (57)..(57)
<223>  XaaArgSer.


<220>
<221>  UNSURE
<222>  (59)..(59)
<223>  XaaTyrGly.


<220>
<221>  UNSURE
<222>  (87)..(87)
<223>  XaaArgLys.


<220>
```

<221> UNSURE
<222> (88)..(88)
<223> XaaAlaPro.


<220>
<221> UNSURE
<222> (97)..(97)
<223> XaaAlaAsn.


<220>
<221> UNSURE
<222> (98)..(98)
<223> XaaLysAla.


<400> 27
Glu Val Gln Leu Xaa Glu Ser Gly Gly Gly Leu Val Gln Pro Gly Gly
1               5                   10                  15
Ser Leu Arg Leu Ser Cys Ala Ala Ser Gly Phe Thr Phe Xaa Xaa Xaa
            20                  25                  30
Xaa Met Ser Trp Val Arg Gln Ala Pro Gly Lys Gly Leu Glu Trp Val
            35                  40                  45
Ser Xaa Ile Xaa Xaa Xaa Gly Gly Xaa Thr Xaa Tyr Ala Asp Ser Val
        50                  55                  60
Lys Gly Arg Phe Thr Ile Ser Arg Asp Asn Ser Lys Asn Thr Leu Tyr
65                  70                  75                  80
Leu Gln Met Asn Ser Leu Xaa Xaa Glu Asp Thr Ala Val Tyr Tyr Cys
                85                  90                  95
Xaa Xaa Asp His Pro Gln Gly Tyr Trp Gly Gln Gly Thr Thr Val Thr
            100                 105                 110
Val Ser Ser
        115


<210> 28
<211> 107
<212> PRT
<213> (Artificial Sequence)

<220>
<221> DOMAIN
<223>


<400> 28
Asp Ile Gln Leu Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Gly Lys Ala Pro Lys Val Leu Ile
            35                  40                  45
Tyr Phe Thr Ser Ser Leu His Ser Gly Val Pro Ser Arg Phe Ser Gly
        50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
                85                  90                  95
Thr Phe Gly Gln Gly Thr Lys Val Glu Ile Lys
            100                 105


<210> 29
<211> 214

<210> 29 ... (not shown)

<212> PRT
<213> (Artificial Sequence)

<220>
<221> CHAIN
<223>

<400> 29

```
Asp Ile Gln Leu Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Gly Lys Ala Pro Lys Val Leu Ile
        35                  40                  45
Tyr Phe Thr Ser Ser Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
            85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
            165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
        210
```

<210> 30
<211> 123
<212> PRT
<213> (Artificial Sequence)

<220>
<221> DOMAIN
<223>

<400> 30

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
            85                  90                  95
Ala Lys Tyr Pro Tyr Tyr Tyr Gly Thr Ser His Trp Tyr Phe Asp Val
        100                 105                 110
```

```
Trp Gly Gln Gly Thr Leu Val Thr Val Ser Ser
        115                 120


<210>   31
<211>   451
<212>   PRT
<213>   (Artificial Sequence)


<220>
<221>   CHAIN
<223>   +IgG1


<400>   31
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
            195                 200                 205
Asn His Lys Pro Ser Asn Thr Lys Val Asp Lys Lys Val Glu Pro Lys
        210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
        290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
            355                 360                 365
Val Ser Leu Thr Cys Leu Val Lys Gly Phe Tyr Pro Ser Asp Ile Ala
370                 375                 380
Val Glu Trp Glu Ser Asn Gly Gln Pro Glu Asn Asn Tyr Lys Thr Thr
```

```
385                         390                    395                       400
Pro Pro Val Leu Asp Ser Asp Gly Ser Phe Phe Leu Tyr Ser Lys Leu
                405                    410                    415
Thr Val Asp Lys Ser Arg Trp Gln Gln Gly Asn Val Phe Ser Cys Ser
                420                    425                    430
Val Met His Glu Ala Leu His Asn His Tyr Thr Gln Lys Ser Leu Ser
                435                    440                    445
Leu Ser Pro
        450
```

```
<210>   32
<211>   5
<212>   PRT
<213>   (Artificial Sequence)

<220>
<221>   DOMAIN
<223>   lucentis HCDR1


<400>   32
His Tyr Gly Met Asn
1                   5


<210>   33
<211>   17
<212>   PRT
<213>   (Artificial Sequence)

<220>
<221>   DOMAIN
<223>   lucentis HCDR2


<400>   33
Trp Ile Asn Thr Tyr Thr Gly Glu Pro Thr Tyr Ala Ala Asp Phe Lys
1                   5                   10                      15
Arg


<210>   34
<211>   14
<212>   PRT
<213>   (Artificial Sequence)

<220>
<221>   DOMAIN
<223>   lucentis HCDR3


<400>   34
Tyr Pro Tyr Tyr Tyr Gly Thr Ser His Trp Tyr Phe Asp Val
1                   5                   10


<210>   35
<211>   11
<212>   PRT
<213>   (Artificial Sequence)

<220>
<221>   DOMAIN
<223>   lucentis LCDR1
```

```
<400>  35
Ser Ala Ser Gln Asp Ile Ser Asn Tyr Leu Asn
1               5                   10


<210>  36
<211>  7
<212>  PRT
<213>  (Artificial Sequence)


<220>
<221>  DOMAIN
<223>  lucentis LCDR2



<400>  36
Phe Thr Ser Ser Leu His Ser
1               5


<210>  37
<211>  10
<212>  PRT
<213>  (Artificial Sequence)


<220>
<221>  DOMAIN
<223>  lucentis LCDR3



<400>  37
Gln Gln Tyr Ser Thr Val Pro Trp Thr Phe
1               5                   10


<210>  38
<211>  569
<212>  PRT
<213>  (Artificial Sequence)


<220>
<221>  DOMAIN
<223>  hu14.A-V



<400>  38
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
            35                  40                  45
Gly Trp Ile Asn Thr Tyr Thr Gly Glu Pro Thr Tyr Ala Ala Asp Phe
        50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
            115                 120                 125
Pro Ser Val Phe Pro Leu Ala Pro Ser Ser Lys Ser Thr Ser Gly Gly
            130                 135                 140
Thr Ala Ala Leu Gly Cys Leu Val Lys Asp Tyr Phe Pro Glu Pro Val
```

```
                145                   150                   155                   160
Thr Val Ser Trp Asn Ser Gly Ala Leu Thr Ser Gly Val His Thr Phe
                    165                   170                   175
Pro Ala Val Leu Gln Ser Ser Gly Leu Tyr Ser Leu Ser Ser Val Val
                180                   185                   190
Thr Val Pro Ser Ser Ser Leu Gly Thr Gln Thr Tyr Ile Cys Asn Val
            195                   200                   205
Asn His Lys Pro Ser Asn Thr Lys Val Asp Lys Lys Val Glu Pro Lys
    210                   215                   220
Ser Cys Asp Lys Thr His Thr Cys Pro Pro Cys Pro Ala Pro Glu Leu
225                   230                   235                   240
Leu Gly Gly Pro Ser Val Phe Leu Phe Pro Pro Lys Pro Lys Asp Thr
                245                   250                   255
Leu Met Ile Ser Arg Thr Pro Glu Val Thr Cys Val Val Val Asp Val
                260                   265                   270
Ser His Glu Asp Pro Glu Val Lys Phe Asn Trp Tyr Val Asp Gly Val
            275                   280                   285
Glu Val His Asn Ala Lys Thr Lys Pro Arg Glu Glu Gln Tyr Asn Ser
    290                   295                   300
Thr Tyr Arg Val Val Ser Val Leu Thr Val Leu His Gln Asp Trp Leu
305                   310                   315                   320
Asn Gly Lys Glu Tyr Lys Cys Lys Val Ser Asn Lys Ala Leu Pro Ala
                325                   330                   335
Pro Ile Glu Lys Thr Ile Ser Lys Ala Lys Gly Gln Pro Arg Glu Pro
                340                   345                   350
Gln Val Tyr Thr Leu Pro Pro Ser Arg Asp Glu Leu Thr Lys Asn Gln
                355                   360                   365
Val Ser Leu Thr Cys Leu Val Lys Gly Phe Tyr Pro Ser Asp Ile Ala
370                   375                   380
Val Glu Trp Glu Ser Asn Gly Gln Pro Glu Asn Asn Tyr Lys Thr Thr
385                   390                   395                   400
Pro Pro Val Leu Asp Ser Asp Gly Ser Phe Phe Leu Tyr Ser Lys Leu
                405                   410                   415
Thr Val Asp Lys Ser Arg Trp Gln Gln Gly Asn Val Phe Ser Cys Ser
                420                   425                   430
Val Met His Glu Ala Leu His Asn His Tyr Thr Gln Lys Ser Leu Ser
                435                   440                   445
Leu Ser Pro Gly Gly Gly Glu Val Gln Leu Val Glu Ser Gly Gly Gly
            450                   455                   460
Leu Val Gln Pro Gly Gly Ser Leu Arg Leu Ser Cys Ala Ala Ser Gly
465                   470                   475                   480
Phe Thr Phe Ser Asp Phe Gly Met Ser Trp Val Arg Gln Ala Pro Gly
                485                   490                   495
Lys Gly Leu Glu Trp Val Ser Ser Ile Thr Trp Asn Gly Gly Ser Thr
                500                   505                   510
Tyr Tyr Ala Asp Ser Val Lys Gly Arg Phe Thr Ile Ser Arg Asp Asn
            515                   520                   525
Ser Lys Asn Thr Leu Tyr Leu Gln Met Asn Ser Leu Arg Ala Glu Asp
    530                   535                   540
Thr Ala Val Tyr Tyr Cys Asn Ala Asp His Pro Gln Gly Tyr Trp Gly
545                   550                   555                   560
Gln Gly Thr Thr Val Thr Val Ser Ser
                565
```

<210> 39
<211> 569
<212> PRT
<213> (Artificial Sequence)

<220>
<221> DOMAIN
<223> hu14.1A-V

```
<400>  39
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
            195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
    275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
            355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
            435                 440                 445
Leu Ser Pro Gly Gly Gly Glu Val Gln Leu Val Glu Ser Gly Gly Gly
    450                 455                 460
Leu Val Gln Pro Gly Gly Ser Leu Arg Leu Ser Cys Ala Ala Ser Gly
465                 470                 475                 480
Phe Thr Phe Ser Asp Phe Gly Met Ser Trp Val Arg Gln Ala Pro Gly
```

```
                          485                    490                    495
         Lys Gly Leu Glu Trp Val Ser Ser Ile Thr Trp Gly Gly Gly Ser Thr
                     500                    505                    510
         Tyr Tyr Ala Asp Ser Val Lys Gly Arg Phe Thr Ile Ser Arg Asp Asn
                     515                    520                    525
         Ser Lys Asn Thr Leu Tyr Leu Gln Met Asn Ser Leu Arg Ala Glu Asp
             530                    535                    540
         Thr Ala Val Tyr Tyr Cys Asn Ala Asp His Pro Gln Gly Tyr Trp Gly
         545                    550                    555                    560
         Gln Gly Thr Thr Val Thr Val Ser Ser
                             565
```

```
<210>  40
<211>  569
<212>  PRT
<213>  (Artificial Sequence)
```

```
<220>
<221>  DOMAIN
<223>  hu14.2A-V
```

```
<400>  40
Glu Val Gln Leu Val Glu Ser Gly Gly Gly Leu Val Gln Pro Gly Gly
1                   5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
            195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
            245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
    275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
```

```
Asn Gly Lys Glu Tyr Lys Cys Lys Val Ser Asn Lys Ala Leu Pro Ala
            325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
            355                 360                 365
Val Ser Leu Thr Cys Leu Val Lys Gly Phe Tyr Pro Ser Asp Ile Ala
        370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Gly Gly Glu Val Gln Leu Val Glu Ser Gly Gly Gly
        450                 455                 460
Leu Val Gln Pro Gly Gly Ser Leu Arg Leu Ser Cys Ala Ala Ser Gly
465                 470                 475                 480
Phe Thr Phe Ser Asp Phe Gly Met Ser Trp Val Arg Gln Ala Pro Gly
                485                 490                 495
Lys Gly Leu Glu Trp Val Ser Ser Ile Thr Trp Ser Gly Gly Ser Thr
            500                 505                 510
Tyr Tyr Ala Asp Ser Val Lys Gly Arg Phe Thr Ile Ser Arg Asp Asn
        515                 520                 525
Ser Lys Asn Thr Leu Tyr Leu Gln Met Asn Ser Leu Arg Ala Glu Asp
        530                 535                 540
Thr Ala Val Tyr Tyr Cys Asn Ala Asp His Pro Gln Gly Tyr Trp Gly
545                 550                 555                 560
Gln Gly Thr Thr Val Thr Val Ser Ser
                565
```

```
<210>   41
<211>   569
<212>   PRT
<213>   (Artificial Sequence)

<220>
<221>   DOMAIN
<223>   hu14.1B-V

<400>   41
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
        50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
            115                 120                 125
Pro Ser Val Phe Pro Leu Ala Pro Ser Ser Lys Ser Thr Ser Gly Gly
        130                 135                 140
Thr Ala Ala Leu Gly Cys Leu Val Lys Asp Tyr Phe Pro Glu Pro Val
```

```
                145                           150                           155                           160
                Thr Val Ser Trp Asn Ser Gly Ala Leu Thr Ser Gly Val His Thr Phe
                                165                           170                           175
                Pro Ala Val Leu Gln Ser Ser Gly Leu Tyr Ser Leu Ser Ser Val Val
                                180                           185                           190
                Thr Val Pro Ser Ser Ser Leu Gly Thr Gln Thr Tyr Ile Cys Asn Val
                                195                           200                           205
                Asn His Lys Pro Ser Asn Thr Lys Val Asp Lys Lys Val Glu Pro Lys
                210                           215                           220
                Ser Cys Asp Lys Thr His Thr Cys Pro Pro Cys Pro Ala Pro Glu Leu
                225                           230                           235                           240
                Leu Gly Gly Pro Ser Val Phe Leu Phe Pro Pro Lys Pro Lys Asp Thr
                                245                           250                           255
                Leu Met Ile Ser Arg Thr Pro Glu Val Thr Cys Val Val Val Asp Val
                                260                           265                           270
                Ser His Glu Asp Pro Glu Val Lys Phe Asn Trp Tyr Val Asp Gly Val
                                275                           280                           285
                Glu Val His Asn Ala Lys Thr Lys Pro Arg Glu Glu Gln Tyr Asn Ser
                290                           295                           300
                Thr Tyr Arg Val Val Ser Val Leu Thr Val Leu His Gln Asp Trp Leu
                305                           310                           315                           320
                Asn Gly Lys Glu Tyr Lys Cys Lys Val Ser Asn Lys Ala Leu Pro Ala
                                325                           330                           335
                Pro Ile Glu Lys Thr Ile Ser Lys Ala Lys Gly Gln Pro Arg Glu Pro
                                340                           345                           350
                Gln Val Tyr Thr Leu Pro Pro Ser Arg Asp Glu Leu Thr Lys Asn Gln
                                355                           360                           365
                Val Ser Leu Thr Cys Leu Val Lys Gly Phe Tyr Pro Ser Asp Ile Ala
                370                           375                           380
                Val Glu Trp Glu Ser Asn Gly Gln Pro Glu Asn Asn Tyr Lys Thr Thr
                385                           390                           395                           400
                Pro Pro Val Leu Asp Ser Asp Gly Ser Phe Phe Leu Tyr Ser Lys Leu
                                405                           410                           415
                Thr Val Asp Lys Ser Arg Trp Gln Gln Gly Asn Val Phe Ser Cys Ser
                                420                           425                           430
                Val Met His Glu Ala Leu His Asn His Tyr Thr Gln Lys Ser Leu Ser
                                435                           440                           445
                Leu Ser Pro Gly Gly Gly Glu Val Gln Leu Val Glu Ser Gly Gly Gly
                                450                           455                           460
                Leu Val Gln Pro Gly Gly Ser Leu Arg Leu Ser Cys Ala Ala Ser Gly
                465                           470                           475                           480
                Phe Thr Phe Ser Asp Phe Gly Met Ser Trp Val Arg Gln Ala Pro Gly
                                485                           490                           495
                Lys Gly Leu Glu Trp Val Ser Ser Ile Thr Trp Gly Gly Gly Ser Thr
                                500                           505                           510
                Tyr Tyr Ala Asp Ser Val Lys Gly Arg Phe Thr Ile Ser Arg Asp Asn
                                515                           520                           525
                Ser Lys Asn Thr Leu Tyr Leu Gln Met Asn Ser Leu Lys Pro Glu Asp
                530                           535                           540
                Thr Ala Val Tyr Tyr Cys Asn Ala Asp His Pro Gln Gly Tyr Trp Gly
                545                           550                           555                           560
                Gln Gly Thr Thr Val Thr Val Ser Ser
                                565
```

<210>    42
<211>    569
<212>    PRT
<213>    (Artificial Sequence)

<220>
<221>    DOMAIN
<223>    hu14.2B-V

<400> 42

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
            195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
    275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
            355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Gly Gly Glu Val Gln Leu Val Glu Ser Gly Gly Gly
    450                 455                 460
Leu Val Gln Pro Gly Gly Ser Leu Arg Leu Ser Cys Ala Ala Ser Gly
465                 470                 475                 480
Phe Thr Phe Ser Asp Phe Gly Met Ser Trp Val Arg Gln Ala Pro Gly
```

```
                              485                      490                        495
          Lys Gly Leu Glu Trp Val Ser Ser Ile Thr Trp Ser Gly Gly Ser Thr
                      500                      505                      510
          Tyr Tyr Ala Asp Ser Val Lys Gly Arg Phe Thr Ile Ser Arg Asp Asn
                      515                      520                      525
          Ser Lys Asn Thr Leu Tyr Leu Gln Met Asn Ser Leu Lys Pro Glu Asp
                      530                      535                      540
          Thr Ala Val Tyr Tyr Cys Asn Ala Asp His Pro Gln Gly Tyr Trp Gly
          545                      550                      555                      560
          Gln Gly Thr Thr Val Thr Val Ser Ser
                              565


          <210>  43
          <211>  569
          <212>  PRT
          <213>  (Artificial Sequence)


          <220>
          <221>  DOMAIN
          <223>  hu15.A-V


          <400>  43
          Glu Val Gln Leu Val Glu Ser Gly Gly Gly Leu Val Gln Pro Gly Gly
          1                   5                   10                      15
          Ser Leu Arg Leu Ser Cys Ala Ala Ser Gly Tyr Asp Phe Thr His Tyr
                      20                      25                      30
          Gly Met Asn Trp Val Arg Gln Ala Pro Gly Lys Gly Leu Glu Trp Val
                      35                      40                      45
          Gly Trp Ile Asn Thr Tyr Thr Gly Glu Pro Thr Tyr Ala Ala Asp Phe
          50                      55                      60
          Lys Arg Arg Phe Thr Phe Ser Leu Asp Thr Ser Lys Ser Thr Ala Tyr
          65                      70                      75                      80
          Leu Gln Met Asn Ser Leu Arg Ala Glu Asp Thr Ala Val Tyr Tyr Cys
                      85                      90                      95
          Ala Lys Tyr Pro Tyr Tyr Tyr Gly Thr Ser His Trp Tyr Phe Asp Val
                      100                     105                     110
          Trp Gly Gln Gly Thr Leu Val Thr Val Ser Ser Ala Ser Thr Lys Gly
                      115                     120                     125
          Pro Ser Val Phe Pro Leu Ala Pro Ser Ser Lys Ser Thr Ser Gly Gly
                      130                     135                     140
          Thr Ala Ala Leu Gly Cys Leu Val Lys Asp Tyr Phe Pro Glu Pro Val
          145                     150                     155                     160
          Thr Val Ser Trp Asn Ser Gly Ala Leu Thr Ser Gly Val His Thr Phe
                      165                     170                     175
          Pro Ala Val Leu Gln Ser Ser Gly Leu Tyr Ser Leu Ser Ser Val Val
                      180                     185                     190
          Thr Val Pro Ser Ser Ser Leu Gly Thr Gln Thr Tyr Ile Cys Asn Val
                      195                     200                     205
          Asn His Lys Pro Ser Asn Thr Lys Val Asp Lys Lys Val Glu Pro Lys
                      210                     215                     220
          Ser Cys Asp Lys Thr His Thr Cys Pro Pro Cys Pro Ala Pro Glu Leu
          225                     230                     235                     240
          Leu Gly Gly Pro Ser Val Phe Leu Phe Pro Pro Lys Pro Lys Asp Thr
                      245                     250                     255
          Leu Met Ile Ser Arg Thr Pro Glu Val Thr Cys Val Val Val Asp Val
                      260                     265                     270
          Ser His Glu Asp Pro Glu Val Lys Phe Asn Trp Tyr Val Asp Gly Val
                      275                     280                     285
          Glu Val His Asn Ala Lys Thr Lys Pro Arg Glu Glu Gln Tyr Asn Ser
                      290                     295                     300
          Thr Tyr Arg Val Val Ser Val Leu Thr Val Leu His Gln Asp Trp Leu
          305                     310                     315                     320
```

```
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Gly Gly Glu Val Gln Leu Val Glu Ser Gly Gly Gly
        450                 455                 460
Leu Val Gln Pro Gly Gly Ser Leu Arg Leu Ser Cys Ala Ala Ser Gly
465                 470                 475                 480
Phe Thr Phe Ser Ser Tyr Ala Met Ser Trp Val Arg Gln Ala Pro Gly
                485                 490                 495
Lys Gly Leu Glu Trp Val Ser Thr Ile Asn Ser Gly Gly Gly Arg Thr
            500                 505                 510
Gly Tyr Ala Asp Ser Val Lys Gly Arg Phe Thr Ile Ser Arg Asp Asn
        515                 520                 525
Ser Lys Asn Thr Leu Tyr Leu Gln Met Asn Ser Leu Arg Ala Glu Asp
        530                 535                 540
Thr Ala Val Tyr Tyr Cys Ala Lys Asp His Pro Gln Gly Tyr Trp Gly
545                 550                 555                 560
Gln Gly Thr Thr Val Thr Val Ser Ser
                565
```

```
<210>   44
<211>   569
<212>   PRT
<213>   (Artificial Sequence)


<220>
<221>   DOMAIN
<223>   hu15.B-V


<400>   44
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
            35                  40                  45
Gly Trp Ile Asn Thr Tyr Thr Gly Glu Pro Thr Tyr Ala Ala Asp Phe
        50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
        130                 135                 140
Thr Ala Ala Leu Gly Cys Leu Val Lys Asp Tyr Phe Pro Glu Pro Val
```

```
                145                      150                      155                      160
        Thr Val Ser Trp Asn Ser Gly Ala Leu Thr Ser Gly Val His Thr Phe
                            165                      170                      175
        Pro Ala Val Leu Gln Ser Ser Gly Leu Tyr Ser Leu Ser Ser Val Val
                            180                      185                      190
        Thr Val Pro Ser Ser Ser Leu Gly Thr Gln Thr Tyr Ile Cys Asn Val
                            195                      200                      205
        Asn His Lys Pro Ser Asn Thr Lys Val Asp Lys Lys Val Glu Pro Lys
                210                      215                      220
        Ser Cys Asp Lys Thr His Thr Cys Pro Pro Cys Pro Ala Pro Glu Leu
        225                      230                      235                      240
        Leu Gly Gly Pro Ser Val Phe Leu Phe Pro Pro Lys Pro Lys Asp Thr
                            245                      250                      255
        Leu Met Ile Ser Arg Thr Pro Glu Val Thr Cys Val Val Val Asp Val
                            260                      265                      270
        Ser His Glu Asp Pro Glu Val Lys Phe Asn Trp Tyr Val Asp Gly Val
                            275                      280                      285
        Glu Val His Asn Ala Lys Thr Lys Pro Arg Glu Glu Gln Tyr Asn Ser
                290                      295                      300
        Thr Tyr Arg Val Val Ser Val Leu Thr Val Leu His Gln Asp Trp Leu
        305                      310                      315                      320
        Asn Gly Lys Glu Tyr Lys Cys Lys Val Ser Asn Lys Ala Leu Pro Ala
                            325                      330                      335
        Pro Ile Glu Lys Thr Ile Ser Lys Ala Lys Gly Gln Pro Arg Glu Pro
                            340                      345                      350
        Gln Val Tyr Thr Leu Pro Pro Ser Arg Asp Glu Leu Thr Lys Asn Gln
                            355                      360                      365
        Val Ser Leu Thr Cys Leu Val Lys Gly Phe Tyr Pro Ser Asp Ile Ala
        370                      375                      380
        Val Glu Trp Glu Ser Asn Gly Gln Pro Glu Asn Asn Tyr Lys Thr Thr
        385                      390                      395                      400
        Pro Pro Val Leu Asp Ser Asp Gly Ser Phe Phe Leu Tyr Ser Lys Leu
                            405                      410                      415
        Thr Val Asp Lys Ser Arg Trp Gln Gln Gly Asn Val Phe Ser Cys Ser
                            420                      425                      430
        Val Met His Glu Ala Leu His Asn His Tyr Thr Gln Lys Ser Leu Ser
                            435                      440                      445
        Leu Ser Pro Gly Gly Gly Glu Val Gln Leu Val Glu Ser Gly Gly Gly
                            450                      455                      460
        Leu Val Gln Pro Gly Gly Ser Leu Arg Leu Ser Cys Ala Ala Ser Gly
        465                      470                      475                      480
        Phe Thr Phe Ser Ser Tyr Ala Met Ser Trp Val Arg Gln Ala Pro Gly
                            485                      490                      495
        Lys Gly Leu Glu Trp Val Ser Thr Ile Asn Ser Gly Gly Gly Arg Thr
                            500                      505                      510
        Gly Tyr Ala Asp Ser Val Lys Gly Arg Phe Thr Ile Ser Arg Asp Asn
                            515                      520                      525
        Ser Lys Asn Thr Leu Tyr Leu Gln Met Asn Ser Leu Arg Ala Glu Asp
                530                      535                      540
        Thr Ala Val Tyr Tyr Cys Asn Ala Asp His Pro Gln Gly Tyr Trp Gly
        545                      550                      555                      560
        Gln Gly Thr Thr Val Thr Val Ser Ser
                            565
```

```
        <210>   45
        <211>   569
        <212>   PRT
        <213>   (Artificial Sequence)

        <220>
        <221>   DOMAIN
        <223>   hu15.C-V
```

```
<400>   45
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
            195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
                260                 265                 270
Ser His Glu Asp Pro Glu Val Lys Phe Asn Trp Tyr Val Asp Gly Val
    275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
            355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
            405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
            435                 440                 445
Leu Ser Pro Gly Gly Gly Glu Val Gln Leu Gln Glu Ser Gly Gly Gly
            450                 455                 460
Leu Val Gln Pro Gly Gly Ser Leu Arg Leu Ser Cys Ala Ala Ser Gly
465                 470                 475                 480
Phe Thr Phe Ser Ser Tyr Ala Met Ser Trp Val Arg Gln Ala Pro Gly
```

```
                         485                    490                     495
       Lys Gly Leu Glu Trp Val Ser Thr Ile Asn Ser Gly Gly Gly Arg Thr
                     500                    505                    510
       Gly Tyr Ala Asp Ser Val Lys Gly Arg Phe Thr Ile Ser Arg Asp Asn
                     515                    520                    525
       Ser Lys Asn Thr Leu Tyr Leu Gln Met Asn Ser Leu Arg Ala Glu Asp
                     530                    535                    540
       Thr Ala Val Tyr Tyr Cys Asn Ala Asp His Pro Gln Gly Tyr Trp Gly
       545                    550                    555                    560
       Gln Gly Thr Thr Val Thr Val Ser Ser
                                 565


       <210>   46
       <211>   569
       <212>   PRT
       <213>   (Artificial Sequence)


       <220>
       <221>   DOMAIN
       <223>   hu15.D-V


       <400>   46
       Glu Val Gln Leu Val Glu Ser Gly Gly Gly Leu Val Gln Pro Gly Gly
       1               5                   10                  15
       Ser Leu Arg Leu Ser Cys Ala Ala Ser Gly Tyr Asp Phe Thr His Tyr
                     20                  25                  30
       Gly Met Asn Trp Val Arg Gln Ala Pro Gly Lys Gly Leu Glu Trp Val
                 35                  40                  45
       Gly Trp Ile Asn Thr Tyr Thr Gly Glu Pro Thr Tyr Ala Ala Asp Phe
           50                  55                  60
       Lys Arg Arg Phe Thr Phe Ser Leu Asp Thr Ser Lys Ser Thr Ala Tyr
       65                  70                  75                  80
       Leu Gln Met Asn Ser Leu Arg Ala Glu Asp Thr Ala Val Tyr Tyr Cys
                         85                  90                  95
       Ala Lys Tyr Pro Tyr Tyr Tyr Gly Thr Ser His Trp Tyr Phe Asp Val
                     100                 105                 110
       Trp Gly Gln Gly Thr Leu Val Thr Val Ser Ser Ala Ser Thr Lys Gly
                 115                 120                 125
       Pro Ser Val Phe Pro Leu Ala Pro Ser Ser Lys Ser Thr Ser Gly Gly
           130                 135                 140
       Thr Ala Ala Leu Gly Cys Leu Val Lys Asp Tyr Phe Pro Glu Pro Val
       145                 150                 155                 160
       Thr Val Ser Trp Asn Ser Gly Ala Leu Thr Ser Gly Val His Thr Phe
                         165                 170                 175
       Pro Ala Val Leu Gln Ser Ser Gly Leu Tyr Ser Leu Ser Ser Val Val
                     180                 185                 190
       Thr Val Pro Ser Ser Ser Leu Gly Thr Gln Thr Tyr Ile Cys Asn Val
                     195                 200                 205
       Asn His Lys Pro Ser Asn Thr Lys Val Asp Lys Lys Val Glu Pro Lys
           210                 215                 220
       Ser Cys Asp Lys Thr His Thr Cys Pro Pro Cys Pro Ala Pro Glu Leu
       225                 230                 235                 240
       Leu Gly Gly Pro Ser Val Phe Leu Phe Pro Pro Lys Pro Lys Asp Thr
                         245                 250                 255
       Leu Met Ile Ser Arg Thr Pro Glu Val Thr Cys Val Val Val Asp Val
                     260                 265                 270
       Ser His Glu Asp Pro Glu Val Lys Phe Asn Trp Tyr Val Asp Gly Val
                     275                 280                 285
       Glu Val His Asn Ala Lys Thr Lys Pro Arg Glu Glu Gln Tyr Asn Ser
           290                 295                 300
       Thr Tyr Arg Val Val Ser Val Leu Thr Val Leu His Gln Asp Trp Leu
       305                 310                 315                 320
```

```
Asn Gly Lys Glu Tyr Lys Cys Lys Val Ser Asn Lys Ala Leu Pro Ala
                325                     330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
            355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
            435                 440                 445
Leu Ser Pro Gly Gly Gly Glu Val Gln Leu Gln Glu Ser Gly Gly Gly
            450                 455                 460
Leu Val Gln Pro Gly Gly Ser Leu Arg Leu Ser Cys Ala Ala Ser Gly
465                 470                 475                 480
Phe Thr Phe Asn Ser Tyr Ala Met Ser Trp Val Arg Gln Ala Pro Gly
                485                 490                 495
Lys Gly Leu Glu Trp Val Ser Thr Ile Asn Ser Gly Gly Gly Arg Thr
            500                 505                 510
Gly Tyr Ala Asp Ser Val Lys Gly Arg Phe Thr Ile Ser Arg Asp Asn
            515                 520                 525
Ser Lys Asn Thr Leu Tyr Leu Gln Met Asn Ser Leu Arg Ala Glu Asp
            530                 535                 540
Thr Ala Val Tyr Tyr Cys Asn Ala Asp His Pro Gln Gly Tyr Trp Gly
545                 550                 555                 560
Gln Gly Thr Thr Val Thr Val Ser Ser
                565
```

```
<210>  47
<211>  569
<212>  PRT
<213>  (Artificial Sequence)


<220>
<221>  DOMAIN
<223>  hu15.E-V
```

```
<400>  47
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
            35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
            115                 120                 125
Pro Ser Val Phe Pro Leu Ala Pro Ser Ser Lys Ser Thr Ser Gly Gly
            130                 135                 140
Thr Ala Ala Leu Gly Cys Leu Val Lys Asp Tyr Phe Pro Glu Pro Val
```

```
         145                    150                    155                    160
         Thr Val Ser Trp Asn Ser Gly Ala Leu Thr Ser Gly Val His Thr Phe
                         165                    170                    175
         Pro Ala Val Leu Gln Ser Ser Gly Leu Tyr Ser Leu Ser Ser Val Val
                         180                    185                    190
         Thr Val Pro Ser Ser Ser Leu Gly Thr Gln Thr Tyr Ile Cys Asn Val
                         195                    200                    205
         Asn His Lys Pro Ser Asn Thr Lys Val Asp Lys Lys Val Glu Pro Lys
         210                    215                    220
         Ser Cys Asp Lys Thr His Thr Cys Pro Pro Cys Pro Ala Pro Glu Leu
         225                    230                    235                    240
         Leu Gly Gly Pro Ser Val Phe Leu Phe Pro Pro Lys Pro Lys Asp Thr
                         245                    250                    255
         Leu Met Ile Ser Arg Thr Pro Glu Val Thr Cys Val Val Val Asp Val
                         260                    265                    270
         Ser His Glu Asp Pro Glu Val Lys Phe Asn Trp Tyr Val Asp Gly Val
                         275                    280                    285
         Glu Val His Asn Ala Lys Thr Lys Pro Arg Glu Glu Gln Tyr Asn Ser
         290                    295                    300
         Thr Tyr Arg Val Val Ser Val Leu Thr Val Leu His Gln Asp Trp Leu
         305                    310                    315                    320
         Asn Gly Lys Glu Tyr Lys Cys Lys Val Ser Asn Lys Ala Leu Pro Ala
                         325                    330                    335
         Pro Ile Glu Lys Thr Ile Ser Lys Ala Lys Gly Gln Pro Arg Glu Pro
                         340                    345                    350
         Gln Val Tyr Thr Leu Pro Pro Ser Arg Asp Glu Leu Thr Lys Asn Gln
                         355                    360                    365
         Val Ser Leu Thr Cys Leu Val Lys Gly Phe Tyr Pro Ser Asp Ile Ala
         370                    375                    380
         Val Glu Trp Glu Ser Asn Gly Gln Pro Glu Asn Asn Tyr Lys Thr Thr
         385                    390                    395                    400
         Pro Pro Val Leu Asp Ser Asp Gly Ser Phe Phe Leu Tyr Ser Lys Leu
                         405                    410                    415
         Thr Val Asp Lys Ser Arg Trp Gln Gln Gly Asn Val Phe Ser Cys Ser
                         420                    425                    430
         Val Met His Glu Ala Leu His Asn His Tyr Thr Gln Lys Ser Leu Ser
                         435                    440                    445
         Leu Ser Pro Gly Gly Gly Glu Val Gln Leu Val Glu Ser Gly Gly Gly
                         450                    455                    460
         Leu Val Gln Pro Gly Gly Ser Leu Arg Leu Ser Cys Ala Ala Ser Gly
         465                    470                    475                    480
         Phe Thr Phe Ser Ser Tyr Ala Met Ser Trp Val Arg Gln Ala Pro Gly
                         485                    490                    495
         Lys Gly Leu Glu Trp Val Ser Thr Ile Asn Ser Gly Gly Gly Arg Thr
                         500                    505                    510
         Gly Tyr Ala Asp Ser Val Lys Gly Arg Phe Thr Ile Ser Arg Asp Asn
                         515                    520                    525
         Ser Lys Asn Thr Leu Tyr Leu Gln Met Asn Ser Leu Arg Pro Glu Asp
         530                    535                    540
         Thr Ala Val Tyr Tyr Cys Asn Ala Asp His Pro Gln Gly Tyr Trp Gly
         545                    550                    555                    560
         Gln Gly Thr Thr Val Thr Val Ser Ser
                         565
```

<210> 48
<211> 463
<212> PRT
<213> (Artificial Sequence)

<220>
<221> CHAIN
<223> crossmab anti-ANG2

<400> 48
```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Gly Tyr
            20                  25                  30
Tyr Met His Trp Val Arg Gln Ala Pro Gly Gln Gly Leu Glu Trp Met
        35                  40                  45
Gly Trp Ile Asn Pro Asn Ser Gly Gly Thr Asn Tyr Ala Gln Lys Phe
    50                  55                  60
Gln Gly Arg Val Thr Met Thr Arg Asp Thr Ser Ile Ser Thr Ala Tyr
65                  70                  75                  80
Met Glu Leu Ser Arg Leu Arg Ser Asp Asp Thr Ala Val Tyr Tyr Cys
            85                  90                  95
Ala Arg Ser Pro Asn Pro Tyr Tyr Tyr Asp Ser Ser Gly Tyr Tyr Tyr
            100                 105                 110
Pro Gly Ala Phe Asp Ile Trp Gly Gln Gly Thr Met Val Thr Val Ser
        115                 120                 125
Ser Ala Ser Val Ala Ala Pro Ser Val Phe Ile Phe Pro Pro Ser Asp
    130                 135                 140
Glu Gln Leu Lys Ser Gly Thr Ala Ser Val Val Cys Leu Leu Asn Asn
145                 150                 155                 160
Phe Tyr Pro Arg Glu Ala Lys Val Gln Trp Lys Val Asp Asn Ala Leu
            165                 170                 175
Gln Ser Gly Asn Ser Gln Glu Ser Val Thr Glu Gln Asp Ser Lys Asp
            180                 185                 190
Ser Thr Tyr Ser Leu Ser Ser Thr Leu Thr Leu Ser Lys Ala Asp Tyr
    195                 200                 205
Glu Lys His Lys Val Tyr Ala Cys Glu Val Thr His Gln Gly Leu Ser
210                 215                 220
Ser Pro Val Thr Lys Ser Phe Asn Arg Gly Glu Cys Asp Lys Thr His
225                 230                 235                 240
Thr Cys Pro Pro Cys Pro Ala Pro Glu Leu Leu Gly Gly Pro Ser Val
            245                 250                 255
Phe Leu Phe Pro Pro Lys Pro Lys Asp Thr Leu Met Ile Ser Arg Thr
            260                 265                 270
Pro Glu Val Thr Cys Val Val Val Asp Val Ser His Glu Asp Pro Glu
        275                 280                 285
Val Lys Phe Asn Trp Tyr Val Asp Gly Val Glu Val His Asn Ala Lys
    290                 295                 300
Thr Lys Pro Arg Glu Glu Gln Tyr Asn Ser Thr Tyr Arg Val Val Ser
305                 310                 315                 320
Val Leu Thr Val Leu His Gln Asp Trp Leu Asn Gly Lys Glu Tyr Lys
            325                 330                 335
Cys Lys Val Ser Asn Lys Ala Leu Pro Ala Pro Ile Glu Lys Thr Ile
            340                 345                 350
Ser Lys Ala Lys Gly Gln Pro Arg Glu Pro Gln Val Cys Thr Leu Pro
            355                 360                 365
Pro Ser Arg Asp Glu Leu Thr Lys Asn Gln Val Ser Leu Ser Cys Ala
    370                 375                 380
Val Lys Gly Phe Tyr Pro Ser Asp Ile Ala Val Glu Trp Glu Ser Asn
385                 390                 395                 400
Gly Gln Pro Glu Asn Asn Tyr Lys Thr Thr Pro Pro Val Leu Asp Ser
            405                 410                 415
Asp Gly Ser Phe Phe Leu Val Ser Lys Leu Thr Val Asp Lys Ser Arg
        420                 425                 430
Trp Gln Gln Gly Asn Val Phe Ser Cys Ser Val Met His Glu Ala Leu
        435                 440                 445
His Asn His Tyr Thr Gln Lys Ser Leu Ser Leu Ser Pro Gly Lys
    450                 455                 460
```

<210> 49
<211> 213

<212> PRT
<213> (Artificial Sequence)

<220>
<221> CHAIN
<223> crossmab anti-ANG2


<400> 49
Gln Pro Gly Leu Thr Gln Pro Pro Ser Val Ser Val Ala Pro Gly Gln
1               5                   10                  15
Thr Ala Arg Ile Thr Cys Gly Gly Asn Ile Gly Ser Lys Ser Val
            20              25                  30
His Trp Tyr Gln Gln Lys Pro Gly Gln Ala Pro Val Leu Val Val Tyr
        35                  40                  45
Asp Asp Ser Asp Arg Pro Ser Gly Ile Pro Glu Arg Phe Ser Gly Ser
    50                  55                  60
Asn Ser Gly Asn Thr Ala Thr Leu Thr Ile Ser Arg Val Glu Ala Gly
65                  70                  75                  80
Asp Glu Ala Asp Tyr Tyr Cys Gln Val Trp Asp Ser Ser Ser Asp His
                85                  90                  95
Tyr Val Phe Gly Thr Gly Thr Lys Val Thr Val Leu Ser Ser Ala Ser
            100                 105                 110
Thr Lys Gly Pro Ser Val Phe Pro Leu Ala Pro Ser Ser Lys Ser Thr
        115                 120                 125
Ser Gly Gly Thr Ala Ala Leu Gly Cys Leu Val Lys Asp Tyr Phe Pro
    130                 135                 140
Glu Pro Val Thr Val Ser Trp Asn Ser Gly Ala Leu Thr Ser Gly Val
145                 150                 155                 160
His Thr Phe Pro Ala Val Leu Gln Ser Ser Gly Leu Tyr Ser Leu Ser
            165                 170                 175
Ser Val Val Thr Val Pro Ser Ser Ser Leu Gly Thr Gln Thr Tyr Ile
            180                 185                 190
Cys Asn Val Asn His Lys Pro Ser Asn Thr Lys Val Asp Lys Lys Val
        195                 200                 205
Glu Pro Lys Ser Cys
        210


<210> 50
<211> 453
<212> PRT
<213> (Artificial Sequence)

<220>
<221> CHAIN
<223> crossmab anti-VEGF


<400> 50
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Thr Phe Thr Asn Tyr
            20              25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
        50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro His Tyr Tyr Gly Ser Ser His Trp Tyr Phe Asp Val
            100                 105                 110

```
Trp Gly Gln Gly Thr Leu Val Thr Val Ser Ser Ala Ser Thr Lys Gly
    115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
                180                 185                 190
Thr Val Pro Ser Ser Ser Leu Gly Thr Gln Thr Tyr Ile Cys Asn Val
    195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
                260                 265                 270
Ser His Glu Asp Pro Glu Val Lys Phe Asn Trp Tyr Val Asp Gly Val
    275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
                340                 345                 350
Gln Val Tyr Thr Leu Pro Pro Cys Arg Asp Glu Leu Thr Lys Asn Gln
    355                 360                 365
Val Ser Leu Trp Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
                420                 425                 430
Val Met His Glu Ala Leu His Asn His Tyr Thr Gln Lys Ser Leu Ser
    435                 440                 445
Leu Ser Pro Gly Lys
    450
```

```
<210>   51
<211>   214
<212>   PRT
<213>   (Artificial Sequence)

<220>
<221>   CHAIN
<223>   crossmab anti-VEGF


<400>   51
Asp Ile Gln Met Thr Gln Ser Pro Ser Ser Leu Ser Ala Ser Val Gly
1                 5                 10                  15
Asp Arg Val Thr Ile Thr Cys Ser Ala Ser Gln Asp Ile Ser Asn Tyr
                20                  25                  30
Leu Asn Trp Tyr Gln Gln Lys Pro Gly Lys Ala Pro Lys Val Leu Ile
            35                  40                  45
Tyr Phe Thr Ser Ser Leu His Ser Gly Val Pro Ser Arg Phe Ser Gly
```

EP 3 943 511 A1

```
            50                    55                        60
Ser Gly Ser Gly Thr Asp Phe Thr Leu Thr Ile Ser Ser Leu Gln Pro
65                    70                  75                    80
Glu Asp Phe Ala Thr Tyr Tyr Cys Gln Gln Tyr Ser Thr Val Pro Trp
                    85                  90                    95
Thr Phe Gly Gln Gly Thr Lys Val Glu Ile Lys Arg Thr Val Ala Ala
            100                   105                   110
Pro Ser Val Phe Ile Phe Pro Pro Ser Asp Glu Gln Leu Lys Ser Gly
            115                   120                   125
Thr Ala Ser Val Val Cys Leu Leu Asn Asn Phe Tyr Pro Arg Glu Ala
            130                   135                   140
Lys Val Gln Trp Lys Val Asp Asn Ala Leu Gln Ser Gly Asn Ser Gln
145                   150                   155                   160
Glu Ser Val Thr Glu Gln Asp Ser Lys Asp Ser Thr Tyr Ser Leu Ser
                    165                   170                   175
Ser Thr Leu Thr Leu Ser Lys Ala Asp Tyr Glu Lys His Lys Val Tyr
                    180                   185                   190
Ala Cys Glu Val Thr His Gln Gly Leu Ser Ser Pro Val Thr Lys Ser
            195                   200                   205
Phe Asn Arg Gly Glu Cys
            210


<210>  52
<211>  452
<212>  PRT
<213>  (Artificial Sequence)


<220>
<221>  CHAIN
<223>


<400>  52
Glu Val Gln Leu Val Glu Ser Gly Gly Gly Leu Val Gln Pro Gly Gly
1                   5                   10                    15
Ser Leu Arg Leu Ser Cys Ala Ala Ser Gly Tyr Thr Phe Thr Asn Tyr
                    20                  25                    30
Gly Met Asn Trp Val Arg Gln Ala Pro Gly Lys Gly Leu Glu Trp Val
            35                  40                  45
Gly Trp Ile Asn Thr Tyr Thr Gly Glu Pro Thr Tyr Ala Ala Asp Phe
            50                  55                  60
Lys Arg Arg Phe Thr Phe Ser Leu Asp Thr Ser Lys Ser Thr Ala Tyr
65                    70                  75                    80
Leu Gln Met Asn Ser Leu Arg Ala Glu Asp Thr Ala Val Tyr Tyr Cys
                    85                  90                    95
Ala Lys Tyr Pro His Tyr Tyr Gly Ser Ser His Trp Tyr Phe Asp Val
            100                   105                   110
Trp Gly Gln Gly Thr Leu Val Thr Val Ser Ser Ala Ser Thr Lys Gly
            115                   120                   125
Pro Ser Val Phe Pro Leu Ala Pro Ser Ser Lys Ser Thr Ser Gly Gly
            130                   135                   140
Thr Ala Ala Leu Gly Cys Leu Val Lys Asp Tyr Phe Pro Glu Pro Val
145                   150                   155                   160
Thr Val Ser Trp Asn Ser Gly Ala Leu Thr Ser Gly Val His Thr Phe
                    165                   170                   175
Pro Ala Val Leu Gln Ser Ser Gly Leu Tyr Ser Leu Ser Ser Val Val
            180                   185                   190
Thr Val Pro Ser Ser Ser Leu Gly Thr Gln Thr Tyr Ile Cys Asn Val
            195                   200                   205
Asn His Lys Pro Ser Asn Thr Lys Val Asp Lys Lys Val Glu Pro Lys
            210                   215                   220
Ser Cys Asp Lys Thr His Thr Cys Pro Pro Cys Pro Ala Pro Glu Leu
225                   230                   235                   240
```

80

Leu Gly Gly Pro Ser Val Phe Leu Phe Pro Pro Lys Pro Lys Asp Thr
                245                     250             255

Leu Met Ile Ser Arg Thr Pro Glu Val Thr Cys Val Val Val Asp Val
            260                 265             270

Ser His Glu Asp Pro Glu Val Lys Phe Asn Trp Tyr Val Asp Gly Val
        275                 280             285

Glu Val His Asn Ala Lys Thr Lys Pro Arg Glu Glu Gln Tyr Asn Ser
    290                 295             300

Thr Tyr Arg Val Val Ser Val Leu Thr Val Leu His Gln Asp Trp Leu
305             310             315                         320

Asn Gly Lys Glu Tyr Lys Cys Lys Val Ser Asn Lys Ala Leu Pro Ala
            325             330             335

Pro Ile Glu Lys Thr Ile Ser Lys Ala Lys Gly Gln Pro Arg Glu Pro
            340             345             350

Gln Val Tyr Thr Leu Pro Pro Ser Arg Glu Glu Met Thr Lys Asn Gln
            355             360             365

Val Ser Leu Thr Cys Leu Val Lys Gly Phe Tyr Pro Ser Asp Ile Ala
    370             375             380

Val Glu Trp Glu Ser Asn Gly Gln Pro Glu Asn Asn Tyr Lys Thr Thr
385             390             395                         400

Pro Pro Val Leu Asp Ser Asp Gly Ser Phe Phe Leu Tyr Ser Lys Leu
            405             410                         415

Thr Val Asp Lys Ser Arg Trp Gln Gln Gly Val Phe Ser Cys Ser Val
            420             425             430

Met His Glu Ala Leu His Asn His Tyr Thr Gln Lys Ser Leu Ser Leu
        435             440                 445

Ser Pro Gly Lys
        450


<210> 53
<211> 214
<212> PRT
<213> (Artificial Sequence)


<220>
<221> CHAIN
<223>


<400> 53
Asp Ile Gln Met Thr Gln Ser Pro Ser Ser Leu Ser Ala Ser Val Gly
1               5               10              15

Asp Arg Val Thr Ile Thr Cys Ser Ala Ser Gln Asp Ile Ser Asn Tyr
            20              25              30

Leu Asn Trp Tyr Gln Gln Lys Pro Gly Lys Ala Pro Lys Val Leu Ile
        35              40              45

Tyr Phe Thr Ser Ser Leu His Ser Gly Val Pro Ser Arg Phe Ser Gly
    50              55              60

Ser Gly Ser Gly Thr Asp Phe Thr Leu Thr Ile Ser Ser Leu Gln Pro
65              70              75                          80

Glu Asp Phe Ala Thr Tyr Tyr Cys Gln Gln Tyr Ser Thr Val Pro Trp
            85              90              95

Thr Phe Gly Gln Gly Thr Lys Val Glu Ile Lys Arg Thr Val Ala Ala
            100             105             110

Pro Ser Val Phe Ile Phe Pro Pro Ser Asp Glu Gln Leu Lys Ser Gly
            115             120             125

Thr Ala Ser Val Val Cys Leu Leu Asn Asn Phe Tyr Pro Arg Glu Ala
    130             135             140

Lys Val Gln Trp Lys Val Asp Asn Ala Leu Gln Ser Gly Asn Ser Gln
145             150             155                         160

Glu Ser Val Thr Glu Gln Asp Ser Lys Asp Ser Thr Tyr Ser Leu Ser
            165             170                         175

Ser Thr Leu Thr Leu Ser Lys Ala Asp Tyr Glu Lys His Lys Val Tyr

```
                      180                       185                       190
          Ala Cys Glu Val Thr His Gln Gly Leu Ser Ser Pro Val Thr Lys Ser
              195                       200                       205
          Phe Asn Arg Gly Glu Cys
              210


          <210>  54
          <211>  451
          <212>  PRT
          <213>  (Artificial Sequence)


          <220>
          <221>  CHAIN
          <223>  + IgG1


          <400>  54
          Glu Val Gln Leu Val Glu Ser Gly Gly Gly Leu Val Gln Pro Gly Gly
          1               5                   10                  15
          Ser Leu Arg Leu Ser Cys Ala Ala Ser Gly Tyr Asp Phe Thr His Tyr
                      20                  25                  30
          Gly Met Asn Trp Val Arg Gln Ala Pro Gly Lys Gly Leu Glu Trp Val
                      35                  40                  45
          Gly Trp Ile Asn Thr Tyr Thr Gly Glu Pro Thr Tyr Ala Ala Asp Phe
              50                  55                  60
          Lys Arg Arg Phe Thr Phe Ser Leu Asp Thr Ser Lys Ser Thr Ala Tyr
          65                  70                  75                  80
          Leu Gln Met Asn Ser Leu Arg Ala Glu Asp Thr Ala Val Tyr Tyr Cys
                      85                  90                  95
          Ala Lys Tyr Pro Tyr Tyr Tyr Gly Thr Ser His Trp Tyr Phe Asp Val
                      100                 105                 110
          Trp Gly Gln Gly Thr Leu Val Thr Val Ser Ser Ala Ser Thr Lys Gly
                      115                 120                 125
          Pro Ser Val Phe Pro Leu Ala Pro Ser Ser Lys Ser Thr Ser Gly Gly
              130                 135                 140
          Thr Ala Ala Leu Gly Cys Leu Val Lys Asp Tyr Phe Pro Glu Pro Val
          145                 150                 155                 160
          Thr Val Ser Trp Asn Ser Gly Ala Leu Thr Ser Gly Val His Thr Phe
                      165                 170                 175
          Pro Ala Val Leu Gln Ser Ser Gly Leu Tyr Ser Leu Ser Ser Val Val
                      180                 185                 190
          Thr Val Pro Ser Ser Ser Leu Gly Thr Gln Thr Tyr Ile Cys Asn Val
                      195                 200                 205
          Asn His Lys Pro Ser Asn Thr Lys Val Asp Lys Lys Val Glu Pro Lys
              210                 215                 220
          Ser Cys Asp Lys Thr His Thr Cys Pro Pro Cys Pro Ala Pro Glu Ala
          225                 230                 235                 240
          Ala Gly Gly Pro Ser Val Phe Leu Phe Pro Pro Lys Pro Lys Asp Thr
                      245                 250                 255
          Leu Met Ala Ser Arg Thr Pro Glu Val Thr Cys Val Val Val Asp Val
                      260                 265                 270
          Ser His Glu Asp Pro Glu Val Lys Phe Asn Trp Tyr Val Asp Gly Val
                      275                 280                 285
          Glu Val His Asn Ala Lys Thr Lys Pro Arg Glu Glu Gln Tyr Asn Ser
              290                 295                 300
          Thr Tyr Arg Val Val Ser Val Leu Thr Val Leu Ala Gln Asp Trp Leu
          305                 310                 315                 320
          Asn Gly Lys Glu Tyr Lys Cys Lys Val Ser Asn Lys Ala Leu Pro Ala
                      325                 330                 335
          Pro Ile Glu Lys Thr Ile Ser Lys Ala Lys Gly Gln Pro Arg Glu Pro
                      340                 345                 350
          Gln Val Tyr Thr Leu Pro Pro Ser Arg Asp Glu Leu Thr Lys Asn Gln
                      355                 360                 365
```

```
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn Ala Tyr Thr Gln Lys Ser Leu Ser
            435                 440                 445
Leu Ser Pro
        450
```

```
<210>  55
<211>  569
<212>  PRT
<213>  (Artificial Sequence)

<220>
<221>  PEPTIDE
<223>  hu15.E-V1


<400>  55
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
            195                 200                 205
Asn His Lys Pro Ser Asn Thr Lys Val Asp Lys Lys Val Glu Pro Lys
        210                 215                 220
Ser Cys Asp Lys Thr His Thr Cys Pro Pro Cys Pro Ala Pro Glu Ala
225                 230                 235                 240
Ala Gly Gly Pro Ser Val Phe Leu Phe Pro Pro Lys Pro Lys Asp Thr
                245                 250                 255
Leu Met Ala Ser Arg Thr Pro Glu Val Thr Cys Val Val Val Asp Val
            260                 265                 270
Ser His Glu Asp Pro Glu Val Lys Phe Asn Trp Tyr Val Asp Gly Val
            275                 280                 285
Glu Val His Asn Ala Lys Thr Lys Pro Arg Glu Glu Gln Tyr Asn Ser
            290                 295                 300
Thr Tyr Arg Val Val Ser Val Leu Thr Val Leu Ala Gln Asp Trp Leu
```

83

```
                   305                   310                   315                   320
Asn Gly Lys Glu Tyr Lys Cys Lys Val Ser Asn Lys Ala Leu Pro Ala
                325                   330                   335
Pro Ile Glu Lys Thr Ile Ser Lys Ala Lys Gly Gln Pro Arg Glu Pro
                340                   345                   350
Gln Val Tyr Thr Leu Pro Pro Ser Arg Asp Glu Leu Thr Lys Asn Gln
                355                   360                   365
Val Ser Leu Thr Cys Leu Val Lys Gly Phe Tyr Pro Ser Asp Ile Ala
                370                   375                   380
Val Glu Trp Glu Ser Asn Gly Gln Pro Glu Asn Asn Tyr Lys Thr Thr
385                   390                   395                   400
Pro Pro Val Leu Asp Ser Asp Gly Ser Phe Leu Tyr Ser Lys Leu
                405                   410                   415
Thr Val Asp Lys Ser Arg Trp Gln Gln Gly Asn Val Phe Ser Cys Ser
                420                   425                   430
Val Met His Glu Ala Leu His Asn Ala Tyr Thr Gln Lys Ser Leu Ser
                435                   440                   445
Leu Ser Pro Gly Gly Gly Glu Val Gln Leu Val Glu Ser Gly Gly Gly
                450                   455                   460
Leu Val Gln Pro Gly Gly Ser Leu Arg Leu Ser Cys Ala Ala Ser Gly
465                   470                   475                   480
Phe Thr Phe Ser Ser Tyr Ala Met Ser Trp Val Arg Gln Ala Pro Gly
                485                   490                   495
Lys Gly Leu Glu Trp Val Ser Thr Ile Asn Ser Gly Gly Gly Arg Thr
                500                   505                   510
Gly Tyr Ala Asp Ser Val Lys Gly Arg Phe Thr Ile Ser Arg Asp Asn
                515                   520                   525
Ser Lys Asn Thr Leu Tyr Leu Gln Met Asn Ser Leu Arg Pro Glu Asp
                530                   535                   540
Thr Ala Val Tyr Tyr Cys Asn Ala Asp His Pro Gln Gly Tyr Trp Gly
545                   550                   555                   560
Gln Gly Thr Thr Val Thr Val Ser Ser
                565
```

```
<210>   56
<211>   569
<212>   PRT
<213>   (Artificial Sequence)

<220>
<221>   PEPTIDE
<223>   hu14.2B-V1


<400>   56
Glu Val Gln Leu Val Glu Ser Gly Gly Gly Leu Val Gln Pro Gly Gly
1                   5                   10                   15
Ser Leu Arg Leu Ser Cys Ala Ala Ser Gly Tyr Asp Phe Thr His Tyr
                20                   25                   30
Gly Met Asn Trp Val Arg Gln Ala Pro Gly Lys Gly Leu Glu Trp Val
                35                   40                   45
Gly Trp Ile Asn Thr Tyr Thr Gly Glu Pro Thr Tyr Ala Ala Asp Phe
                50                   55                   60
Lys Arg Arg Phe Thr Phe Ser Leu Asp Thr Ser Lys Ser Thr Ala Tyr
65                   70                   75                   80
Leu Gln Met Asn Ser Leu Arg Ala Glu Asp Thr Ala Val Tyr Tyr Cys
                85                   90                   95
Ala Lys Tyr Pro Tyr Tyr Tyr Gly Thr Ser His Trp Tyr Phe Asp Val
                100                   105                   110
Trp Gly Gln Gly Thr Leu Val Thr Val Ser Ser Ala Ser Thr Lys Gly
                115                   120                   125
Pro Ser Val Phe Pro Leu Ala Pro Ser Ser Lys Ser Thr Ser Gly Gly
                130                   135                   140
```

```
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Val Val
                180                 185                 190
Thr Val Pro Ser Ser Ser Leu Gly Thr Gln Thr Tyr Ile Cys Asn Val
            195                 200                 205
Asn His Lys Pro Ser Asn Thr Lys Val Asp Lys Lys Val Glu Pro Lys
        210                 215                 220
Ser Cys Asp Lys Thr His Thr Cys Pro Pro Cys Pro Ala Pro Glu Ala
225                 230                 235                 240
Ala Gly Gly Pro Ser Val Phe Leu Phe Pro Pro Lys Pro Lys Asp Thr
                245                 250                 255
Leu Met Ala Ser Arg Thr Pro Glu Val Thr Cys Val Val Val Asp Val
                260                 265                 270
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
        290                 295                 300
Thr Tyr Arg Val Val Ser Val Leu Thr Val Leu Ala Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
                340                 345                 350
Gln Val Tyr Thr Leu Pro Pro Ser Arg Asp Glu Leu Thr Lys Asn Gln
            355                 360                 365
Val Ser Leu Thr Cys Leu Val Lys Gly Phe Tyr Pro Ser Asp Ile Ala
370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
                420                 425                 430
Val Met His Glu Ala Leu His Asn Ala Tyr Thr Gln Lys Ser Leu Ser
            435                 440                 445
Leu Ser Pro Gly Gly Gly Glu Val Gln Leu Val Glu Ser Gly Gly Gly
        450                 455                 460
Leu Val Gln Pro Gly Gly Ser Leu Arg Leu Ser Cys Ala Ala Ser Gly
465                 470                 475                 480
Phe Thr Phe Ser Asp Phe Gly Met Ser Trp Val Arg Gln Ala Pro Gly
                485                 490                 495
Lys Gly Leu Glu Trp Val Ser Ser Ile Thr Trp Ser Gly Gly Ser Thr
                500                 505                 510
Tyr Tyr Ala Asp Ser Val Lys Gly Arg Phe Thr Ile Ser Arg Asp Asn
            515                 520                 525
Ser Lys Asn Thr Leu Tyr Leu Gln Met Asn Ser Leu Lys Pro Glu Asp
        530                 535                 540
Thr Ala Val Tyr Tyr Cys Asn Ala Asp His Pro Gln Gly Tyr Trp Gly
545                 550                 555                 560
Gln Gly Thr Thr Val Thr Val Ser Ser
                565
```

```
<210>   57
<211>   453
<212>   PRT
<213>   (Artificial Sequence)

<220>
<221>   CHAIN
```

<223> RG7716 -VEGF-Knob)


<400> 57

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65              70                  75                  80
Leu Gln Met Asn Ser Leu Arg Ala Glu Asp Thr Ala Val Tyr Tyr Cys
            85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
        130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
            165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Ala
225             230                 235                 240
Ala Gly Gly Pro Ser Val Phe Leu Phe Pro Pro Lys Pro Lys Asp Thr
            245                 250                 255
Leu Met Ala Ser Arg Thr Pro Glu Val Thr Cys Val Val Val Asp Val
            260                 265                 270
Ser His Glu Asp Pro Glu Val Lys Phe Asn Trp Tyr Val Asp Gly Val
    275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu Ala Gln Asp Trp Leu
305             310                 315                 320
Asn Gly Lys Glu Tyr Lys Cys Lys Val Ser Asn Lys Ala Leu Gly Ala
            325                 330                 335
Pro Ile Glu Lys Thr Ile Ser Lys Ala Lys Gly Gln Pro Arg Glu Pro
            340                 345                 350
Gln Val Tyr Thr Leu Pro Pro Cys Arg Asp Glu Leu Thr Lys Asn Gln
        355                 360                 365
Val Ser Leu Trp Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385             390                 395                 400
Pro Pro Val Leu Asp Ser Asp Gly Ser Phe Leu Tyr Ser Lys Leu
            405                 410                 415
Thr Val Asp Lys Ser Arg Trp Gln Gln Gly Asn Val Phe Ser Cys Ser
            420                 425                 430
Val Met His Glu Ala Leu His Asn Ala Tyr Thr Gln Lys Ser Leu Ser
        435                 440                 445
Leu Ser Pro Gly Lys
        450
```

<210> 58
<211> 463
<212> PRT
<213> (Artificial Sequence)

<220>
<221> CHAIN
<223> RG7716 -Ang2 Hole

<400> 58

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Gly Tyr
            20                  25                  30
Tyr Met His Trp Val Arg Gln Ala Pro Gly Gln Gly Leu Glu Trp Met
        35                  40                  45
Gly Trp Ile Asn Pro Asn Ser Gly Gly Thr Asn Tyr Ala Gln Lys Phe
    50                  55                  60
Gln Gly Arg Val Thr Met Thr Arg Asp Thr Ser Ile Ser Thr Ala Tyr
65                  70                  75                  80
Met Glu Leu Ser Arg Leu Arg Ser Asp Asp Thr Ala Val Tyr Tyr Cys
            85                  90                  95
Ala Arg Ser Pro Asn Pro Tyr Tyr Tyr Asp Ser Ser Gly Tyr Tyr Tyr
            100                 105                 110
Pro Gly Ala Phe Asp Ile Trp Gly Gln Gly Thr Met Val Thr Val Ser
        115                 120                 125
Ser Ala Ser Val Ala Ala Pro Ser Val Phe Ile Phe Pro Pro Ser Asp
        130                 135                 140
Glu Gln Leu Lys Ser Gly Thr Ala Ser Val Val Cys Leu Leu Asn Asn
145                 150                 155                 160
Phe Tyr Pro Arg Glu Ala Lys Val Gln Trp Lys Val Asp Asn Ala Leu
            165                 170                 175
Gln Ser Gly Asn Ser Gln Glu Ser Val Thr Glu Gln Asp Ser Lys Asp
            180                 185                 190
Ser Thr Tyr Ser Leu Ser Ser Thr Leu Thr Leu Ser Lys Ala Asp Tyr
        195                 200                 205
Glu Lys His Lys Val Tyr Ala Cys Glu Val Thr His Gln Gly Leu Ser
        210                 215                 220
Ser Pro Val Thr Lys Ser Phe Asn Arg Gly Glu Cys Asp Lys Thr His
225                 230                 235                 240
Thr Cys Pro Pro Cys Pro Ala Pro Glu Ala Ala Gly Gly Pro Ser Val
            245                 250                 255
Phe Leu Phe Pro Pro Lys Pro Lys Asp Thr Leu Met Ala Ser Arg Thr
            260                 265                 270
Pro Glu Val Thr Cys Val Val Val Asp Val Ser His Glu Asp Pro Glu
            275                 280                 285
Val Lys Phe Asn Trp Tyr Val Asp Gly Val Glu Val His Asn Ala Lys
        290                 295                 300
Thr Lys Pro Arg Glu Glu Gln Tyr Asn Ser Thr Tyr Arg Val Val Ser
305                 310                 315                 320
Val Leu Thr Val Leu Ala Gln Asp Trp Leu Asn Gly Lys Glu Tyr Lys
            325                 330                 335
Cys Lys Val Ser Asn Lys Ala Leu Gly Ala Pro Ile Glu Lys Thr Ile
            340                 345                 350
Ser Lys Ala Lys Gly Gln Pro Arg Glu Pro Gln Val Cys Thr Leu Pro
        355                 360                 365
Pro Ser Arg Asp Glu Leu Thr Lys Asn Gln Val Ser Leu Ser Cys Ala
        370                 375                 380
Val Lys Gly Phe Tyr Pro Ser Asp Ile Ala Val Glu Trp Glu Ser Asn
385                 390                 395                 400
Gly Gln Pro Glu Asn Asn Tyr Lys Thr Thr Pro Pro Val Leu Asp Ser
            405                 410                 415
```

```
Asp Gly Ser Phe Phe Leu Val Ser Lys Leu Thr Val Asp Lys Ser Arg
        420             425             430
Trp Gln Gln Gly Asn Val Phe Ser Cys Ser Val Met His Glu Ala Leu
        435             440             445
His Asn Ala Tyr Thr Gln Lys Ser Leu Ser Leu Ser Pro Gly Lys
        450             455             460
```

<210> 59
<211> 213
<212> PRT
<213> (Artificial Sequence)

<220>
<221> CHAIN
<223> RG7716 -Ang2

<400> 59

```
Ser Tyr Val Leu Thr Gln Pro Pro Ser Val Ser Val Ala Pro Gly Gln
1           5               10              15
Thr Ala Arg Ile Thr Cys Gly Gly Asn Asn Ile Gly Ser Lys Ser Val
        20              25              30
His Trp Tyr Gln Gln Lys Pro Gly Gln Ala Pro Val Leu Val Val Tyr
        35              40              45
Asp Asp Ser Asp Arg Pro Ser Gly Ile Pro Glu Arg Phe Ser Gly Ser
        50              55              60
Asn Ser Gly Asn Thr Ala Thr Leu Thr Ile Ser Arg Val Glu Ala Gly
65              70              75              80
Asp Glu Ala Asp Tyr Tyr Cys Gln Val Trp Asp Ser Ser Ser Asp His
            85              90              95
Trp Val Phe Gly Gly Gly Thr Lys Leu Thr Val Leu Ser Ser Ala Ser
        100             105             110
Thr Lys Gly Pro Ser Val Phe Pro Leu Ala Pro Ser Ser Lys Ser Thr
        115             120             125
Ser Gly Gly Thr Ala Ala Leu Gly Cys Leu Val Lys Asp Tyr Phe Pro
        130             135             140
Glu Pro Val Thr Val Ser Trp Asn Ser Gly Ala Leu Thr Ser Gly Val
145             150             155             160
His Thr Phe Pro Ala Val Leu Gln Ser Ser Gly Leu Tyr Ser Leu Ser
            165             170             175
Ser Val Val Thr Val Pro Ser Ser Ser Leu Gly Thr Gln Thr Tyr Ile
        180             185             190
Cys Asn Val Asn His Lys Pro Ser Asn Thr Lys Val Asp Lys Lys Val
        195             200             205
Glu Pro Lys Ser Cys
        210
```

**Description**

**FIELD OF THE INVENTION**

The present disclosure belongs to the field of biopharmaceuticals. Specifically, the present disclosure relates to anti-ANG2 single domain antibodies or antigen-binding fragment thereof, anti-VEGF antibodies or antigen-binding fragment thereof, as well as the preparation and application of the bispecific antibodies formed by fusion of the anti-ANG2 single domain antibody and the anti-VEGF antibody.

**BACKGROUND OF THE INVENTION**

The statements herein only provide background information related to the present disclosure and do not necessarily constitute the prior art.

Neovascularization provides tumor cells with oxygen and nourishment, allowing tumor cells to gain growth advantages to enter the rapid growth period in the presence of blood vessels from the slow growth period in the absence of blood vessels. Therefore, inhibition of tumor growth by inhibiting angiogenesis is a relatively promising and effective strategy. Among the many factors that promote angiogenesis, vascular endothelial growth factor VEGF is a very critical and important factor that promotes angiogenesis. VEGF can promote tumor cell neovascularization by binding to VEGF receptors to promote cell proliferation, migration, and increase vascular permeability. Therefore, blocking VEGF can inhibit tumor angiogenesis, thereby achieving the goal of inhibiting tumor growth and metastasis. There are many clinical biological agents that block VEGF through different strategies, such as the anti-VEGF monoclonal antibody, Avastin, soluble VEGF receptors that neutralizes VEGF, and monoclonal antibodies against VEGF receptor, which all show relatively good activity. However, tumor angiogenesis is a complex process participated by many molecules and multi signaling pathways. Blocking one pathway still cannot achieve the goal of completely inhibiting the tumor, and it is necessary to block other angiogenesis-related factors at the same time.

Tie2 is the second identified tyrosine kinase receptor specific for vascular endothelial cells, and its binding to the ligands angiopoietin-1 (ANG1) and angiopoietin-2 (ANG2) also plays an important role in angiogenesis. Both ANG1 and ANG2 bind to Tie2, among which ANG1 supports the survival of endothelial cells (ECs) and promotes the integrity and stability of blood vessels, while ANG2 has an opposite effect of causing peripheral cells to be detached from endothelial cells, leading to increased endothelial cell permeability, and allowing VEGF to exert its effect on promoting neovascularization. ANG2 and VEGF complement and coordinate with each other and act together in the process of tumor angiogenesis. Therefore, simultaneously blocking VEGF and ANG2 can more effectively inhibit angiogenesis, promote the normalization of blood vessels, and achieve the goal of inhibiting tumor growth and metastasis.

At present, four bispecific antibodies that simultaneously block VEGF and ANG2 signaling pathways have been reported in the field. Among them, Roche's crossmab Vanucizumab, which targets VEGF and ANG2, has the fastest progress and is in clinical phase 2.

Currently, ANG2-VEGF bispecific antibodies or VEGF antibodies are disclosed in patent applications WO1998045332, WO2007095338A2, WO201004058, CN102250247A, WO2011117329, etc., but there is still a need to develop new and highly effective ANG2-VEGF bispecific antibodies and methods for the treatment of tumors.

**SUMMARY OF THE INVENTION**

The present disclosure provides a bispecific antibody that specifically binds to ANG2 and VEGF.

In some embodiments, the bispecific antibody comprises an anti-VEGF antibody or antigen-binding fragment thereof that specifically binds to VEGF, and an anti-ANG2 single domain antibody that specifically binds to ANG2, wherein the anti-ANG2 single domain antibody is covalently connected to the anti-VEGF antibody directly through a peptide bond or indirectly through a linker. Preferably, the anti-VEGF antibody is a monoclonal antibody.

In some embodiments, the anti-ANG2 single domain antibody comprised in the bispecific antibody is connected to the heavy chain amino terminus, heavy chain carboxyl terminus, light chain amino terminus, or light chain carboxyl terminus of the anti-VEGF antibody or antigen-binding fragment thereof.

In some embodiments, the anti-ANG2 single domain antibody comprised in the bispecific antibody is connected to the heavy chain carboxyl terminus of the anti-VEGF antibody or antigen-binding fragment thereof.

In some embodiments, the anti-ANG2 single domain antibody of the bispecific antibody comprises CDR1 as shown in SEQ ID NO: 5 or having at most 3, at most 2 or 1 amino acid substitution mutation compared to the same, CDR2 as shown in SEQ ID NO: 6 or having at most 6, at most 5, at most 4, at most 3, at most 2 or 1 amino acid substitution mutation compared to the same, and CDR3 as shown in SEQ ID NO: 7 or having at most 3, at most 2 or 1 amino acid substitution mutation compared to the same. Exemplarily, the first amino acid D in SEQ ID NO: 5 (DFGMS) is substituted with S, and the second amino acid F is substituted with Y.

In some embodiments, the anti-ANG2 single domain antibody comprised in the bispecific antibody comprises CDR1 as shown in SEQ ID NO: 14, CDR2 as shown in SEQ ID NO: 15, and CDR3 as shown in SEQ ID NO: 7, the sequences of which are as shown below respectively:

| CDR1 | CDR2 | CDR3 |
|---|---|---|
| $X_1X_2X_3MS$ (SEQ ID NO:14) | $X_4IX_5X_6X_7GGX_8 TX_9 YADSVKG$ (SEQ ID NO:15) | DHPQGY (SEQ ID NO:7) |

wherein, $X_1$ is selected from D or S; $X_2$ is selected from F or Y; $X_3$ is selected from G or A; $X_4$ is selected from S or T; $X_5$ is selected from T or N; $X_6$ is selected from W or S; $X_7$ is selected from N, G or S; $X_8$ is selected from R or S; and $X_9$ is selected from Y or G

In some embodiments, the anti-ANG2 single domain antibody comprised in the bispecific antibody comprises CDR1, CDR2 and CDR3 as shown below respectively:

i) the CDR1 as shown in SEQ ID NO: 5, the CDR2 as shown in SEQ ID NO: 6, and the CDR3 as shown in SEQ ID NO: 7;
ii) the CDR1 as shown in SEQ ID NO: 8, the CDR2 as shown in SEQ ID NO: 9, and the CDR3 as shown in SEQ ID NO: 7;
iii) the CDR1 as shown in SEQ ID NO: 5, the CDR2 as shown in SEQ ID NO: 10, and the CDR3 as shown in SEQ ID NO: 7; or
iv) the CDR1 as shown in SEQ ID NO: 5, the CDR2 as shown in SEQ ID NO: 11, and the CDR3 as shown in SEQ ID NO: 7.

In some embodiments, the anti-ANG2 single domain antibody comprised in the bispecific antibody is a llama antibody or a humanized antibody.

In some embodiments, the anti-ANG2 single domain antibody comprised in the bispecific antibody comprises or consists of VHH as shown in SEQ ID NO: 16, preferably, comprises or consists of VHH as shown in SEQ ID NO: 3, SEQ ID NO: 4, SEQ ID NO: 12, or SEQ ID NO: 13, wherein the SEQ ID NO: 16 is as shown below:

DVQLQESGGGLVQPGGSLRLSCAASGFTF$\underline{X_{10}X_1X_2X_3}$MSWVRQAPGKGLE

WVS$\underline{X_4IX_5X_6X_7}$GG$\underline{X_8}$T$\underline{X_9}$YADSVKGRFTISRDNAKNT$\underline{X_{11}}$YLQMNSLKPEDTAIYY

CNA$\underline{DHPQGY}$WGQGTQVTVSS

wherein, $X_1$ is selected from D or S; $X_2$ is selected from F or Y; $X_3$ is selected from G or A; $X_4$ is selected from S or T; $X_5$ is selected from T or N; $X_6$ is selected from W or S; $X_7$ is selected from N, G or S; $X_8$ is selected from R or S; $X_9$ is selected from Y or G; $X_{10}$ is selected from D or N, and $X_{11}$ is selected from V or L.

In some embodiments, the sequence of the anti-ANG2 single domain antibody comprised in the bispecific antibody has at least 90%, 91%, 92%, 93%, 94%, 95%, 96%, 97%, 98% or 99% sequence identity to SEQ ID NO: 3, SEQ ID NO: 4, SEQ ID NO: 12 or SEQ ID NO: 13.

In other embodiments, the humanized antibody or antigen-binding fragment thereof comprised in the bispecific antibody comprises a heavy chain framework region derived from a human antibody or a framework region variant thereof, wherein the framework region variant has at most 10 amino acid back mutations in the heavy chain framework region of the human antibody,

preferably, the back mutation(s) is one or more selected from the group consisting of 5Q, 30N, 83K, 84P, 93N and 94A, wherein 5Q represents, according to the kabat criteria, the amino acid at position 5 of VHH is Q (Gln, glutamine), and others so forth.

In some embodiments, the anti-ANG2 single domain antibody comprised in the bispecific antibody comprises CDR1, CDR2 and CDR3 as shown below:

i) the CDR1 as shown in SEQ ID NO: 5, the CDR2 as shown in SEQ ID NO: 6, and the CDR3 as shown in SEQ ID NO: 7;
ii) the CDR1 as shown in SEQ ID NO: 8, the CDR2 as shown in SEQ ID NO: 9, and the CDR3 as shown in SEQ ID NO: 7;
iii) the CDR1 as shown in SEQ ID NO: 5, the CDR2 as shown in SEQ ID NO: 10, and the CDR3 as shown in SEQ ID NO: 7; or
iv) the CDR1 as shown in SEQ ID NO: 5, the CDR2 as shown in SEQ ID NO: 11, and the CDR3 as shown in SEQ ID NO: 7; and

the framework region of which comprises one or more back mutations selected from the group consisting of 5Q, 30N, 83K, 84P, 93N and 94A.

In some embodiments, the anti-ANG2 humanized antibody or antigen-binding fragment thereof comprised in the bispecific antibody comprises or consists of the sequence as shown in SEQ ID NO: 27, preferably, comprises or consists of the sequence as shown in SEQ ID NO: 17, 18, 19, 20, 21, 22, 23, 24, 25 or 26, wherein the sequence of SED ID NO: 27 is as shown below:

$$\text{EVQLX}_{12}\text{ESGGGLVQPGGSLRLSCAASGFTFX}_{13}\underline{X_1X_2X_3MS}\text{WVRQAPGKGLE}$$
$$\text{WVS}\underline{X_4IX_5X_6X_7\text{GGX}_8TX_9\text{YADSVKG}}\text{RFTISRDNSKNTLYLQMNSLX}_{14}X_{15}\text{EDTAVY}$$
$$\text{YC } X_{16}\ X_{17}\underline{\text{DHPQGY}}\text{WGQGTTVTVSS}$$

wherein, $X_1$ is selected from D or S; $X_2$ is selected from F or Y; $X_3$ is selected from G or A; $X_4$ is selected from S or T; $X_5$ is selected from T or N; $X_6$ is selected from W or S; $X_7$ is selected from N, G or S; $X_8$ is selected from R or S; $X_9$ is selected from Y or G; $X_{12}$ is selected from V or Q, $X_{13}$ is selected from S or N, $X_{14}$ is selected from R or K, $X_{15}$ is selected from A or P, $X_{16}$ is selected from A or N, and $X_{17}$ is selected from K or A.

In some embodiments, the sequence of the anti-ANG2 single domain antibody comprised in the bispecific antibody has at least 90%, 91%, 92%, 93%, 94%, 95%, 96%, 97%, 98% or 99% sequence identity to SEQ ID NO: 17, 18, 19, 20, 21, 22, 23, 24, 25 or 26, respectively.

In some embodiments, the anti-VEGF antibody or antigen-binding fragment thereof comprised in the bispecific antibody comprises:

HCDR1, HCDR2 and HCDR3 as shown in SEQ ID NO: 32, SEQ ID NO: 33 and SEQ ID NO: 34, respectively; and
LCDR1, LCDR2 and LCDR3 as shown in SEQ ID NO: 35, SEQ ID NO: 36 and SEQ ID NO: 37, respectively.

In some embodiments, the anti-VEGF antibody or antigen-binding fragment thereof comprised in the bispecific antibody comprises the light chain variable region as shown in SEQ ID NO: 28, and the heavy chain variable region as shown in SEQ ID NO: 30.

In some embodiments, the anti-VEGF antibody or antigen-binding fragment thereof comprised in the bispecific antibody comprises the light chain as shown in SEQ ID NO: 29, and the heavy chain as shown in SEQ ID NO: 31 or 54.

In some embodiments, the bispecific antibody comprises a first polypeptide chain selected from any one shown in SEQ ID NO: 38, 39, 40, 41, 42, 43, 44, 45, 46, 47, 55 or 56, and/or a second polypeptide chain as shown in SEQ ID NO: 29.

The present disclosure also provides a class of anti-ANG2 single domain antibodies.

In some embodiments, the anti-ANG2 single domain antibody comprises CDR1 as shown in SEQ ID NO: 5 or having at most 3, at most 2 or 1 amino acid mutation compared to the same, CDR2 as shown in SEQ ID NO: 6 or having at most 6, at most 5, at most 4, at most 3, at most 2 or 1 amino acid mutation compared to the same, and CDR3 as shown in SEQ ID NO: 7 or having at most 3, at most 2 or 1 amino acid mutation compared to the same.

In some embodiments, the anti-ANG2 single domain antibody comprises CDR1 as shown in SEQ ID NO: 14, CDR2 as shown in SEQ ID NO: 15 and CDR3 as shown in SEQ ID NO: 7, wherein the CDR sequences are as shown below:

| CDR1 | CDR2 | CDR3 |
|------|------|------|
| $X_1X_2X_3$MS (SEQ ID NO:14) | $X_4$I$X_5X_6X_7$GG$X_8$ T$X_9$ YADSVKG (SEQ ID NO:15) | DHPQGY (SEQ ID NO:7) |

wherein, $X_1$ is selected from D or S; $X_2$ is selected from F or Y; $X_3$ is selected from G or A; $X_4$ is selected from S or T; $X_5$ is selected from T or N; $X_6$ is selected from W or S; $X_7$ is selected from N, G or S; $X_8$ is selected from R or S; and $X_9$ is selected from Y or G

In some embodiments, the anti-ANG2 single domain antibody comprises CDR1, CDR2 and CDR3 as shown below respectively:

i) the CDR1 as shown in SEQ ID NO: 5, the CDR2 as shown in SEQ ID NO: 6, and the CDR3 as shown in SEQ ID NO: 7;
ii) the CDR1 as shown in SEQ ID NO: 8, the CDR2 as shown in SEQ ID NO: 9, and the CDR3 as shown in SEQ ID NO: 7;
iii) the CDR1 as shown in SEQ ID NO: 5, the CDR2 as shown in SEQ ID NO: 10, and the CDR3 as shown in SEQ ID NO: 7; or
iv) the CDR1 as shown in SEQ ID NO: 5, the CDR2 as shown in SEQ ID NO: 11, and the CDR3 as shown in SEQ ID NO: 7.

In some embodiments, the anti-ANG2 single domain antibody is selected from llama antibody or humanized antibody.

In some embodiments, the anti-ANG2 single domain antibody comprises or consists of the sequence as shown in SEQ ID NO: 16, preferably, comprises or consists of the sequence as shown in SEQ ID NO: 3, SEQ ID NO: 4, SEQ ID NO: 12 or SEQ ID NO: 13, wherein the SEQ ID NO: 16 has the sequence as shown below:

$$\text{DVQLQESGGGLVQPGGSLRLSCAASGFTF}\underline{X_{10}X_1X_2X_3\text{M}}\text{SWVRQAPGKGLE}$$
$$\text{WVS}\underline{X_4\text{I}X_5X_6X_7\text{GG}X_8\text{T}X_9}\text{YADSVKGRFTISRDNAKNT}\underline{X_{11}}\text{YLQMNSLKPEDTAIYY}$$
$$\text{CNA}\underline{\text{DHPQGY}}\text{WGQGTQVTVSS}$$

wherein, $X_1$ is selected from D or S; $X_2$ is selected from F or Y; $X_3$ is selected from G or A; $X_4$ is selected from S or T; $X_5$ is selected from T or N; $X_6$ is selected from W or S; $X_7$ is selected from N, G or S; $X_8$ is selected from R or S; $X_9$ is selected from Y or G; $X_{10}$ is selected from D or N, and $X_{11}$ is selected from V or L.

In some embodiments, the sequence of the anti-ANG2 single domain antibody has at least 90%, 91%, 92%, 93%, 94%, 95%, 96%, 97%, 98% or 99% sequence identity to SEQ ID NO: 3, SEQ ID NO: 4, SEQ ID NO: 12 or SEQ ID NO: 13, respectively.

In some embodiments, the anti-ANG2 humanized antibody comprises a heavy chain framework region derived from a human antibody or a framework region variant thereof, wherein the framework variant has at most 10 amino acid back mutations in the heavy chain framework region of the human antibody,

preferably, the back mutation is one or more selected from the group consisting of 5Q, 30N, 83K, 84P, 93N and 94A.

In some embodiments, the sequence of the anti-ANG2 humanized antibody is as shown in SEQ ID NO:22, or comprises one or more mutations selected from the group consisting of 5Q, 30N, 84P, 93N and 94A based on SEQ ID NO:22.

In some embodiments, the anti-ANG2 single domain antibody comprises CDR1, CDR2 and CDR3 as shown below:

i) the CDR1 as shown in SEQ ID NO: 5, the CDR2 as shown in SEQ ID NO: 6, and the CDR3 as shown in SEQ ID NO: 7;
ii) the CDR1 as shown in SEQ ID NO: 8, the CDR2 as shown in SEQ ID NO: 9, and the CDR3 as shown in SEQ ID NO: 7;
iii) the CDR1 as shown in SEQ ID NO: 5, the CDR2 as shown in SEQ ID NO: 10, and the CDR3 as shown in SEQ ID NO: 7; or
iv) the CDR1 as shown in SEQ ID NO: 5, the CDR2 as shown in SEQ ID NO: 11, and the CDR3 as shown in SEQ ID NO: 7; and

the framework region of which comprises one or more back mutations selected from the group consisting of 5Q, 30N, 83K, 84P, 93N and 94A.

In some embodiments, the anti-ANG2 single domain antibody or antigen-binding fragment thereof comprises or consists of the sequence as shown in SEQ ID NO: 27, preferably, comprises or consists of the sequence as shown in SEQ ID NO: 17, 18, 19, 20, 21, 22, 23, 24, 25 or 26, wherein SED ID NO: 27 has the sequence as shown below:

$$\text{EVQL}X_{12}\text{ESGGGLVQPGGSLRLSCAASGFTF}X_{13}\underline{X_1X_2X_3\text{M}}\text{SWVRQAPGKGLE}$$
$$\text{WVS}\underline{X_4\text{I}X_5X_6X_7\text{GG}X_8\text{T}X_9}\text{YADSVKGRFTISRDNSKNTLYLQMNSL}X_{14}X_{15}\text{EDTAVY}$$
$$\text{YC } X_{16} \; X_{17}\underline{\text{DHPQGY}}\text{WGQGTTVTVSS}$$

wherein, $X_1$ is selected from D or S; $X_2$ is selected from F or Y; $X_3$ is selected from G or A; $X_4$ is selected from S or T; $X_5$ is selected from T or N; $X_6$ is selected from W or S; $X_7$ is selected from N, G or S; $X_8$ is selected from R or S; $X_9$ is selected from Y or G; X12 is selected from V or Q, X13 is selected from S or N, X14 is selected from R or K, X15 is selected from A or P, X16 is selected from A or N, and X17 is selected from K or A.

In some embodiments, the sequence of the anti-ANG2 single domain antibody has at least 90%, 91%, 92%, 93%, 94%, 95%, 96%, 97%, 98% or 99% sequence identity to SEQ ID NO: 17, 18, 19, 20, 21, 22, 23, 24, 25 or 26.

The present disclosure also provides an anti-ANG2 single domain antibody, which competitively binds to human ANG2 with the aforementioned anti-ANG2 single domain antibody.

The present disclosure also provides an anti-ANG2 single domain antibody, wherein the single domain antibody comprises the same CDR1, CDR2 and CDR3 sequences as the single domain antibody shown in SEQ ID NO: 3, SEQ ID NO: 4, SEQ ID NO: 12 or SEQ ID NO: 13.

The present disclosure also provides a monoclonal antibody comprising the anti-ANG2 single domain antibody as described above.

The present disclosure also provides a bispecific antibody comprising the anti-ANG2 single domain antibody according to any one of the foregoing.

The present disclosure provides a pharmaceutical composition, which comprises a therapeutically effective amount of the bispecific antibody as described above, or the anti-ANG2 single domain antibody as described above, as well as one or more pharmaceutically acceptable carriers, diluents, buffers or excipients.

The present disclosure also provides an isolated nucleic acid molecule which encodes the bispecific antibody or the anti-ANG2 single domain antibody as described above, or the monoclonal antibody comprising the aforementioned anti-ANG2 single domain antibody.

The present disclosure also provides a vector comprising the nucleic acid molecule as described above.

The present disclosure also provides a host cell transformed with the aforementioned vector, and the host cell is selected from the group consisting of prokaryotic cell and eukaryotic cell, preferably eukaryotic cell, more preferably mammalian cell or insect cell.

The present disclosure also provides a method for producing the bispecific antibody as described above, or the anti-ANG2 single domain antibody as described above, which comprises the processes of culturing the host cell as described above in a medium to form and accumulate the bispecific antibody as described above, or the anti-ANG2 single domain antibody as described above, and recovering from the culture the bispecific antibody, or the anti-ANG2 single domain antibody or antigen-binding fragment thereof as described above.

The present disclosure provides a method for the detection or measurement of human ANG2 *in vitro,* which comprises using the anti-ANG2 single domain antibody as described above or the aforementioned bispecific antibody.

The present disclosure provides a use of the anti-ANG2 single domain antibody or the bispecific antibody as described above in preparing a reagent for the detection or measurement of human ANG2.

The present disclosure also provides a kit comprising the anti-ANG2 single domain antibody or bispecific antibody as described above.

The present disclosure also provides a method for the treatment of a disease related to VEGF-mediated and/or ANG2-mediated angiogenesis effect, which comprises administering to a subject a therapeutically effective amount of the bispecific antibody as described above, or the anti-ANG2 single domain antibody as described above, or the monoclonal antibody comprising the anti-ANG2 single domain antibody as described above, or the pharmaceutical composition as described above; preferably, the therapeutically effective amount is a unit dose of the composition containing 0.1 to 3000 mg of the bispecific antibody as described above, or the anti-ANG2 single domain antibody as described above, or the monoclonal antibody comprising the anti-ANG2 single domain antibody as described above.

The present disclosure also provides a method for the treatment of cancer or angiogenic eye disease, including administering to a subject a therapeutically effective amount of the bispecific antibody as described above, or the anti-ANG2 single domain antibody as described above, or the monoclonal antibody comprising the anti-ANG2 single variable domain antibody as described above, or the pharmaceutical composition as described above; preferably, wherein the cancer is selected from the group consisting of breast cancer, adrenal tumor, fallopian tube cancer, squamous cell carcinoma, ovarian cancer, gastric cancer, colorectal cancer, non-small cell lung cancer, cholangiocarcinoma, bladder cancer, pancreatic cancer, skin cancer and liver cancer; the angiogenic eye disease is selected from the group consisting of neovascular glaucoma, age-related macular degeneration (AMD), diabetic macular edema, corneal neovascularization, corneal graft neovascularization, corneal graft rejection, retinal/choroidal neovascularization, angulus iridocornealis neovascularization (rubeosis), ocular neovascular disease, vascular restenosis and arteriovenous malformations (AVM).

The present disclosure also provides a use of the bispecific antibody as described above, or the anti-ANG2 single domain antibody as described above, or the monoclonal antibody comprising the anti-ANG2 single domain antibody as described above, or the pharmaceutical composition as described above, in preparing a medicament for the treatment of a disease related to VEGF-mediated and/or ANG2-mediated angiogenesis effect.

The present disclosure also provides a use of the bispecific antibody as described above, or the anti-ANG2 single domain antibody as described above, or the monoclonal antibody comprising the anti-ANG2 single domain antibody as described above, or the pharmaceutical composition as described above, in preparing a medicament for the treatment of cancer or angiogenic eye disease; preferably, wherein the cancer is selected from the group consisting of breast cancer, adrenal tumor, fallopian tube cancer, squamous cell carcinoma, ovarian cancer, gastric cancer, colorectal cancer, non-small cell lung cancer, cholangiocarcinoma, bladder cancer, pancreatic cancer, skin cancer and liver cancer; the angiogenic eye disease is selected from the group consisting of neovascular glaucoma, age-related macular degeneration, diabetic macular edema, corneal neovascularization, corneal graft neovascularization, corneal graft rejection, retinal/choroidal neovascularization, angulus iridocornealis neovascularization (rubeosis), ocular neovascular disease, vascular restenosis and arteriovenous malformations (AVM).

The present disclosure also provides a method for the detection or determination of human ANG2 *in vitro,* which comprises the step of using the bispecific antibody as described above, or the anti-ANG2 single domain antibody as described above, or the monoclonal antibody comprising the anti-ANG2 single domain antibody as described above.

The present disclosure also provides the bispecific antibody as described above, or the anti-ANG2 single domain antibody as described above, or the monoclonal antibody comprising the anti-ANG2 single domain antibody as described above, for use as a medicament, and said medicament is for the treatment of a disease related to VEGF-mediated and/or ANG2-mediated angiogenesis effect, preferably for the treatment of cancer or angiogenic eye disease.

The present disclosure also provides the bispecific antibody as described above, or the anti-ANG2 single domain antibody as described above, or the monoclonal antibody comprising the anti-ANG2 single domain antibody as described above, for use as a medicament, and said medicament is for the treatment of cancer or angiogenic eye disease.

**DESCRIPTION OF THE DRAWINGS**

Figure 1 is a schematic diagram of the structure of the bispecific antibody of the present disclosure.

Figure 2A is a figure showing the binding activity of the single domain antibody to human ANG2; Figure 2B is a figure showing the binding activity of the single domain antibody to human ANG1.

Figure 3 is a figure showing that the bispecific antibody blocks the activity of ANG2 binding to Tie2 on the cell surface.

Figure 4 is a figure showing the results of the bispecific antibody inhibiting ANG2-induced phosphorylation of Tie2.

Figure 5 is a figure showing the activity of the bispecific antibody inhibiting VEGF-induced phosphorylation of VEGFR.

Figure 6 is a figure showing the activity of the bispecific antibody inhibiting VEGF-induced proliferation of HUVEC.

Figure 7 is a figure showing the effect of the bispecific antibody on inhibiting the growth of subcutaneously transplanted tumor of human colon cancer cells.

Figure 8A is a figure showing the effect of the antibody on inhibiting the growth of subcutaneously transplanted tumor of non-small cell lung cancer; Figure 8B is a figure showing the results of liver metastasis of non-small cell lung cancer in mice.

Figure 9A is a figure showing the pharmacological effect of the antibody in inhibiting the human A431 mouse transplanted tumor model; Figure 9B is a figure showing the pharmacological effect of the antibody in inhibiting the human PC-3 mouse transplanted tumor model.

Figure 10A is a figure showing the improvement rate of the bispecific antibody in the fluorescence leakage area in the eyes of rhesus monkeys; Figure 10B is a figure showing the effect of the bispecific antibody on the number of level four fluorescent spots in the eyes of rhesus monkeys.

Figure 11 is a figure showing the VEGF concentration in aqueous humor in the rhesus monkeys 28 days after ocular administration.

**DETAILED DESCRIPTION OF THE DISCLOSURE**

To make it easier to understand the present disclosure, certain technical and scientific terms are specifically defined below. Unless clearly defined otherwise herein, all other technical and scientific terms used herein have the meanings commonly understood by those of ordinary skill in the art to which the present disclosure belongs.

The three-letter codes and one-letter codes of amino acids used in the present disclosure are as described in J. biol. chem, 243, p3558 (1968).

The term "ANG-2" refers to angiopoietin-2 (ANG-2) (or abbreviated as ANGPT2 or ANG2), which is documented in, for example, Maisonpierre, P.C. et al., Science 277 (1997) 55-60 and Cheung, A.H. et al., Genomics 48 (1998) 389-91. Angiopoietin-1 and -2 are found to be ligands of Tie (i.e., a tyrosine kinase family selectively expressed in the endothelium of blood vessels), Yancopoulos, G.D. et al., Nature 407 (2000) 242-48. Currently, four members have been identified as belonging to angiopoietin family. Angiopoietin-3 and -4 (ANG-3 and ANG-4) can represent the counterparts of the extensive regions of the same gene loci in mice and humans. Kim, I. et al., FEBS Let 443 (1999) 353-56; Kim, I. et al., J Biol Chem 274 (1999) 26523-28. ANG1 and ANG2 were initially identified in tissue culture experiments as an agonist and an antagonist respectively (for ANG1, see Davis, S. et al., Cell 87 (1996) 1161-69; and for ANG2, see Maisonpierre, P.C. et al., Science 277 (1997) 55-60). All known angiopoietins mainly bind to Tie2, and both ANG1 and 2 bind to Tie2 with an affinity of 3 nM (Kd), Maisonpierre, P.C. et al., Science 277 (1997) 55-60.

The term "VEGF" refers to human vascular endothelial growth factor (VEGF/VEGF-A), which is documented in, for example, Leung, D.W. et al., Science 246 (1989) 1306-9; Keck, P.J. et al., Science 246 (1989) 1309-12, and Connolly, D.T. et al., J. Biol. Chem. 264 (1989) 20017-24. VEGF is involved in regulating normal and abnormal angiogenesis and neovascularization associated with tumors and intraocular disorders (Ferrara, N., Endocr. Rev. 18 (1997) 4-25; Berkman, R.A., J. Clin. Invest. 91 (1993) 153-159; Brown, L.F. et al., Human Pathol. 26 (1995) 86-91; Brown, L.F. et al., Cancer Res. 53 (1993) 4727-4735; Mattern, J. et al., Brit. J. Cancer. 73 (1996) 931-934; and Dvorak, H.F. et al., Am. J. Pathol. 146 (1995) 1029-1039). VEGF is a homodimeric glycoprotein that can promote mitogenesis of endothelial cells.

A "bispecific antibody" is an antibody with binding activity for two different antigens (or different epitopes of the same antigen). The antibodies of the present disclosure are specific to two different antigens, namely VEGF as the first antigen and ANG2 as the second antigen.

The term "binding site" or "antigen binding site" refers to the region in the antibody molecule where the ligand actually binds. The term "antigen binding site" comprises antibody heavy chain variable domain (VH) and antibody light chain variable domain (VL), or comprises either antibody heavy chain variable domain or light chain variable domain.

A "single domain antibody" is an antibody fragment consisting of single domain Fv unit. Like whole antibodies, it can selectively and specifically bind to an antigen. The molecular weight of a single domain antibody is only 12-15 kDa, which is much smaller than a common antibody consisting of two heavy chains and two light chains (150-160 kDa), and even smaller than a Fab fragment (about 50 kDa, one light chain and half of a heavy chain) or a single chain variable fragment (about 25 kDa, two variable domains, one from the light chain and the other from the heavy chain). The single domain antibodies in the present disclosure include, but are not limited to, heavy chain antibodies (antibody naturally devoid of light chains, such as VHH), single domain antibodies derived from conventional 4-chain antibodies, engineered antibodies, and single domain antibodies different from those derived from antibodies. The single domain antibody can be any single domain antibody present in the art or to be discovered in the future. The single domain antibody can be derived from any species, including but not limited to mouse, human, camel, yamma, llama, guanaco, goat, rabbit, cattle, shark, and the like.

The antigen binding site of a "single domain antibody" is present on and formed by a single immunoglobulin domain. This distinguishes the "single domain antibody" from "conventional" immunoglobulins or fragments thereof (such as Fab, scFv, etc.) (in which two immunoglobulin domains, especially two variable domains, interact to form the antigen binding site). Generally, in conventional immunoglobulins, the heavy chain variable domain (VH) and the light chain variable domain (VL) interact to form the antigen binding site. In this case, the complementarity determining regions (CDRs) of both VH and VL will contribute to the antigen binding site, that is, a total of 6 CDRs will participate in the formation of the antigen binding site. In contrast, the binding site of a single domain antibody is formed by a single VH or VL domain. Therefore, the antigen binding site of an immunoglobulin single variable domain is formed by no more than three CDRs.

"VHH domain", also known as VHH or VHH antibody fragment, was originally described as the antigen-binding immunoglobulin (variable) domain of a "heavy chain antibody" (i.e., an "antibody devoid of light chains") (Hamers-Casterman C, Atarhouch T, Muyldermans S, Robinson G, Hamers C, Songa EB, Bendahman N, Hamers R.: "Naturally occurring antibodies devoid of light chains"; Nature 363 (1993) 446-448). The term "VHH domain" distinguishes these variable domains from the heavy chain variable domains present in conventional 4-chain antibodies (which are referred to herein as "VH domain" or "VH") and from the light chain variable domains present in conventional 4-chain antibodies (which are referred to herein as "VL domain" or "VL"). The VHH domain can specifically bind to an epitope in the case that no other antigen-binding domain is present (in contrast, for the VH or VL domain in conventional 4-chain antibodies, the epitope is recognized by the VL domain together with the VH domain). The VHH domain is a small, stable and highly efficient antigen recognition unit formed by a single immunoglobulin domain.

In the context of the present disclosure, the terms VHH domain, VHH, VHH antibody fragment, VHH antibody, as well as "nanobody" and "single domain antibody" are used interchangeably and refer to an immunoglobulin single variable domain with FR1-CDR1-FR2-CDR2-FR3-CDR3-FR4 structure and specifically bind to an epitope without any other immunoglobulin variable domain.

The term "antibody (Ab)" comprises any antigen-binding molecule or molecular complex that comprises at least one complementarity determining region (CDR) that specifically binds to or interacts with a specific antigen (for example ANG2).

The term "antibody" comprises: immunoglobulin molecules comprising four polypeptide chains, two heavy (H) chains and two light (L) chains, interconnected by disulfide bonds, and multimers thereof (for example IgM). Each heavy chain comprises a heavy chain variable region (abbreviated as HCVR or VH herein) and a heavy chain constant region (CH). This heavy chain constant region comprises three regions (domains), CHI, CH2 and CH3. Each light chain comprises a light chain variable region (abbreviated as LCVR or VL herein) and a light chain constant region (CL). The VH and VL regions can be further subdivided into hypervariable regions, called complementarity determining regions (CDRs), interspersed with more conservative regions, called framework regions (FRs, also called framework regions). Each of VH and VL consists of three CDRs and four FRs, arranged from the amino terminus to the carboxyl terminus according to the following order: FR1, CDR1, FR2, CDR2, FR3, CDR3, FR4. In different examples of the present disclosure, the FRs of the anti-ANG2 antibody (or antigen-binding fragment thereof) can be the same as the human germline sequence, or can be naturally or artificially modified. The antibodies can be antibodies of different subclasses, for example, IgG (for example, IgG1, IgG2, IgG3 or IgG4 subclasses), IgA1, IgA2, IgD, IgE or IgM antibodies.

The term "antibody" also includes antigen-binding fragments of complete antibody molecules. The terms "antigen-binding moiety", "antigen-binding domain", "antigen-binding fragment", etc. of an antibody, as used herein, include any naturally occurring, enzymatically produced, synthetic or genetically engineered peptides or glycoproteins that specifically binds to an antigen to form complexes. The antigen-binding fragments of antibodies can be derived from, for example, whole antibody molecules by using any suitable standard techniques, for example proteolytic digestion or recombinant genetic engineering techniques involving manipulation and expression of DNAs encoding antibody variable regions and (as needed) constant regions. Such DNAs are known and/or can be easily obtained from, for example, commercially available sources, DNA databases (including, for example, phage-antibody databases), or can be synthesized. Such DNAs can be sequenced and manipulated chemically or by using molecular biotechnology, for example arranging one or more variable and/or constant regions into a suitable configuration, or introducing codons, generating cysteine residues, and modifying, adding or deleting amino acids, etc.

Non-limiting examples of antigen-binding fragment include: (i) Fab fragment; (ii) F(ab')2 fragment; (iii) Fd fragment; (iv) Fv fragment; (v) single-chain Fv (scFv) molecule; (vi) dAb fragment; and (vii) the smallest recognition unit consisting of amino acid residues mimicking the antibody hypervariable region (for example isolated complementarity determining regions (CDR), for example CDR3 peptide) or restrictive FR3-CDR3-FR4 peptide. Other engineered molecules, for example region-specific antibody, single domain antibody, region-deleted antibody, chimeric antibody, CDR-grafted antibody, diabody, triabody, tetrabody, minibody, nanobody (e.g. monovalent nanobody, bivalent nanobody, etc.), small modular immunopharmaceutical (SMIP) and shark variable IgNAR region, are also encompassed in the term "antigen-binding fragment" as used herein.

The antigen-binding fragment will typically comprise at least one variable region. The variable region can be a region of any size or amino acid composition and will generally comprise CDRs adjacent to or within the framework of one or more framework sequences.

In certain examples, in any configuration of the variable region and the constant region of the antigen-binding fragment, the variable region and the constant region can be directly connected to each other or can be connected through an intact or partial hinge or linker region. The hinge region can consist of at least 2 (for example 5, 10, 15, 20, 40, 60 or more) amino acids, so that it produces flexible and semi-flexible connection between adjacent variable and/or constant regions in a single polypeptide molecule. A "murine antibody" in the present disclosure is a mouse or rat-derived monoclonal antibody prepared according to the knowledge and skills in the art. During preparation, the test subject is injected with antigen, and then hybridomas expressing antibodies with the desired sequence or functional properties are isolated. When the injected test subject is a mouse, the antibody produced is a mouse-derived antibody, and when the injected test subject is a rat, the antibody produced is a rat-derived antibody.

A "chimeric antibody" is an antibody formed by fusing the antibody variable region of the first species (such as mouse) with the antibody constant region of the second species (such as human), which can alleviate the immune response induced by antibody of the first species. Establishing a chimeric antibody requires first establishing a hybridoma secreting specific monoclonal antibodies of the first species, then cloning the variable region gene from the hybridoma cells, and then cloning the antibody constant region gene of the second species as necessary, linking the mouse variable region gene with the constant region gene of the second species to form a chimeric gene and inserting into an expression vector, and finally expressing the chimeric antibody molecule in a eukaryotic system or a prokaryotic system. The term "humanized antibody", including CDR-grafted antibody, refers to the antibody produced by grafting CDR sequences of an antibody derived from animals, for example murine, into the framework regions of a human antibody variable region. The humanized antibody can overcome the heterogeneous reaction induced by the chimeric antibody due to carrying a large amount of heterogeneous protein components. Such framework sequences can be obtained from public DNA databases or published references that include germline antibody gene sequences. For example, the germline DNA sequences of the human heavy chain and light chain variable region genes can be found in the "VBase" human germline sequence database (available on the Internet http://www.vbase2.org/), as well as in Kabat, E. A., et al., 1991, Sequences of Proteins of Immunological Interest, 5th edition. In order to avoid the decrease in activity along with the decrease in immunogenicity, the human antibody variable region framework sequence can be subjected to minimal reverse mutations or back mutations to maintain activity. The humanized antibody of the present disclosure also includes a humanized antibody in which the CDRs have been subjected to affinity maturation by further phage display.

Due to the contact residues of the antigen, CDR grafting may result in reduced affinity of the produced antibody or antigen-binding fragment thereof to the antigen due to the framework residues in contact with the antigen. Such interactions can be the result of hypermutation of somatic cells. Therefore, it may still be necessary to graft such donor framework amino acids to the framework of the humanized antibody. The amino acid residues involved in antigen binding and from non-human antibodies or antigen-binding fragments thereof can be identified by examining the sequence and structure of the animal monoclonal antibody variable region. Residues in the CDR donor framework that differ from the germline can be considered related. If the closest germline cannot be determined, the sequence can be compared with the consensus sequence of a subclass or animal antibody sequence with a high percentage of similarity. Rare framework residues are thought to be the result of hypermutation of somatic cells and thus play an important role in binding.

In an embodiment of the present disclosure, the antibody or antigen-binding fragment thereof may further comprise the light chain constant region of human or murine $\kappa$, $\lambda$ chain or variant thereof, or further comprise the heavy chain constant region of human or murine IgG1, IgG2, IgG3, IgG4 or variant thereof.

The "conventional variant" of the human antibody heavy chain constant region and the human antibody light chain constant region refers to the variant of heavy chain constant region or light chain constant region derived from human that has been disclosed in the prior art and does not change the structure and function of the antibody variable region. Exemplary variants include IgG1, IgG2, IgG3 or IgG4 heavy chain constant region variants with site-directed modifications and amino acid substitutions in the heavy chain constant region. Specific substitutions are such as YTE, L234A and/or L235A, or S228P mutation, or mutations to obtain a knob-into-hole structure (so that the antibody heavy chain has a combination of knob-Fc and hole-Fc) known in the art. These mutations have been confirmed to make the antibody have new properties, but do not change the function of the antibody variable region.

"Human antibody" and "human-derived antibody" can be used interchangeably, and can be an antibody derived from human or an antibody obtained from a transgenic organism which is "engineered" to produce specific human antibodies in response to antigen stimulation and can be produced by any method known in the art. In some technologies, the elements of human heavy chain and light chain gene loci are introduced into cell lines of organisms derived from embryonic stem cell lines, in which the endogenous heavy chain and light chain genetic loci are target disrupted. Transgenic organisms can synthesize human antibodies specific to human antigens, and the organisms can be used to produce human antibody-secreting hybridomas. A human antibody can also be an antibody in which the heavy chain and light chain are encoded by one or more nucleotide sequences derived from human DNA origins. A fully human antibody can also be constructed by gene or chromosome transfection methods and phage display technology, or constructed by B cells activated *in vitro,* all of which are known in the art.

"Monoclonal antibody" refers to an antibody obtained from a population of substantially homogeneous antibodies, that is, except for possible variant antibodies (for example, containing naturally-occurring mutations or mutations generated during the manufacture of monoclonal antibody preparations, these variants are usually present in a small amount), the population consisting of individual antibodies recognize and/or bind to the same epitope. Unlike polyclonal antibody preparations that usually comprise different antibodies against different determinants (epitopes), each monoclonal antibody of a monoclonal antibody preparation (preparation) is directed against a single determinant on an antigen. Therefore, the modifier "monoclonal" indicates the characteristics of the antibody as obtained from a substantially homogeneous antibody population, and should not be interpreted as requiring any specific method to manufacture the antibody. For example, monoclonal antibodies used according to the present disclosure can be prepared by various techniques, including but not limited to hybridoma methods, recombinant DNA methods, phage display methods, as well as methods that utilizes transgenic animals containing the complete or partial human immunoglobulin gene loci. Such methods and other exemplary methods for preparing monoclonal antibodies are described herein. The monoclonal antibody of the present disclosure is a full-length antibody.

The terms "full-length antibody", "intact antibody", "complete antibody" and "whole antibody" are used interchangeably herein and refer to an antibody in a substantially intact form, as distinguished from the antigen-binding fragments defined below. The terms specifically refer to an antibody in which the heavy chain comprises VH region, CH1 region, hinge region and Fc region in sequence from the amino terminus to the carboxyl terminus, and the light chain comprises VL region and CL region in sequence from the amino terminus to the carboxyl terminus.

In addition, although the two domains VL and VH of the Fv fragment are encoded by separate genes, recombination methods can be used to connect them by synthetic linkers, so that they can be produced as a single protein chain in which the VL and VH regions pair to form a monovalent molecule (referred to as single-chain Fv (scFv); see, for example, Bird et al. (1988) Science 242: 423-426; and Huston et al. (1988) Proc. Natl. Acad. Sci USA 85: 5879-5883). Such single chain antibodies are also intended to be included in the term "antigen-binding fragment" of antibody. Such antibody fragments are obtained by using conventional techniques known to those skilled in the art, and the fragments are screened for functionality in the same manner as for intact antibodies. The antigen binding moiety can be produced by recombinant DNA technology or by enzymatic or chemical fragmentation of the intact immunoglobulin.

The antigen-binding fragment can also be incorporated into a single-chain molecule comprising a pair of tandem Fv fragments (VH-CH1-VH-CH1), which together with a complementary light chain polypeptide forms a pair of antigen-binding regions (Zapata et al., 1995, Protein Eng. 8(10): 1057-1062; and U.S. Patent No. 5,641,870).

Fab, an antibody fragment with a molecular weight of about 50,000 Da, is obtained by treating IgG antibody molecules with the protease papain (cleaves the amino acid residue at position 224 of the H chain) and has antigen-binding activity, in which about half of the H chain of the N-terminal side and the entire L chain are joined together by disulfide bonds.

F(ab')2, an antibody fragment with a molecular weight of about 100,000 Da, is obtained by digesting the lower part of the two disulfide bonds in the hinge region of IgG with pepsin. It has antigen-binding activity, and comprises two Fab regions connected at the hinge position.

Fab', an antibody fragment with antigen-binding activity and a molecular weight of about 50,000 Da, is obtained by cleaving the disulfide bond in the hinge region of the aforementioned F(ab')2. Fab' can be produced by using reducing agents, for example dithiothreitol, to treat the F(ab')2 that specifically recognizes and binds to an antigen.

In addition, the Fab' can be produced by inserting the DNA encoding the Fab' fragment of the antibody into a prokaryotic expression vector or a eukaryotic expression vector and introducing the vector into a prokaryotic organism or eukaryotic organism to express the Fab'.

The term "single-chain antibody", "single-chain Fv" or "scFv" refers to molecules comprising an antibody heavy chain variable domain (or region; VH) and an antibody light chain variable domain (or region; VL) connected by a linker. Such scFv molecules can have the general structure: $NH_2$-VL-linker-VH-COOH or $NH_2$-VH-linker-VL-COOH. Suitable linkers of prior art consist of the repetitive GGGGS amino acid sequences or variants thereof, for example using 1 to 4 (including 1, 2, 3 or 4) repetitive variants (Holliger et al. (1993), Proc. Natl. Acad. Sci. USA 90: 6444-6448). Other linkers that can be used in the present disclosure are described in Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol. Immunother. 50:51-59.

"Linker" refers to a polypeptide sequence used to connect polypeptides (such as protein domains), usually with a certain degree of flexibility. The use of linkers will not lead to loss of the original structures and functions of the protein domains.

Diabody refers to an antibody fragment of dimerized scFv, and is an antibody fragment with bivalent antigen binding activity. In the bivalent antigen binding activity, the two antigens can be the same or different.

dsFv is obtained by connecting polypeptides in which one amino acid residue in each of VH and VL is substituted with a cysteine residue via a disulfide bond between cysteine residues. The amino acid residues substituted with cysteine residues can be selected according to a known method (Protein Engineering. 7:697 (1994)) based on the three-dimensional structure prediction of the antibody.

The antigen-binding fragment in some examples of the present disclosure can be produced by the following steps: obtaining the cDNAs encoding the VH and/or VL and other required domains of the monoclonal antibody of the present disclosure that specifically recognizes and binds to an antigen, constructing the DNAs encoding the antigen-binding fragment, inserting the DNAs into a prokaryotic expression vector or a eukaryotic expression vector, and then introducing the expression vector into a prokaryote or eukaryote to express the antigen-binding fragment.

A "Fc region" can be native Fc region sequence or Fc region variant. Although the boundaries of the Fc region of an immunoglobulin heavy chain may vary, the Fc region of human IgG heavy chain is usually defined as extending from the amino acid residue at position Cys226 or from Pro230 to its carboxyl terminus. The numbering of residues in the Fc region is according to the EU index numbering as described in Kabat et al., Sequences of Proteins of Immunological Interest, 5th Edition, Public Health Service, National Institutes of Health, Bethesda, Md., 1991. The Fc region of an immunoglobulin usually has two constant region domains, CH2 and CH3. The "knob-Fc" of the present disclosure refers to a point mutation, T366W, comprised in the Fc region of the antibody to form a knob-like spatial structure. Correspondingly, "hole-Fc" refers to the point mutations, T366S, L368A, and Y407V, comprised in the Fc region of the antibody to form a hole-like spatial structure. Knob-Fc and hole-Fc are more likely to form heterodimers due to steric hindrance. To further promote the formation of heterodimers, point mutations S354C and Y349C can be introduced into knob-Fc and hole-Fc, respectively, to further promote the formation of heterodimers through disulfide bonds. At the same time, in order to eliminate or weaken the ADCC effect caused by antibody Fc, substitution mutations 234A and 235A can also be introduced into Fc. For example, the preferred knob-Fc and hole-Fc of the present disclosure are as shown in SEQ ID NO: 69 and 70, respectively. In a bispecific antibody, knob-Fc or hole-Fc can be used as either the Fc region of the first polypeptide chain or the Fc region of the second polypeptide chain. In one bispecific antibody, the Fc region of the first polypeptide chain and the Fc region of the second polypeptide chain cannot be knob-Fc or hole-Fc at the same time.

The term "amino acid difference" or "amino acid mutation" means that compared with the original protein or polypeptide, the protein or polypeptide variant has amino acid changes or mutations, including the insertion, deletion or substitution of one or more amino acids on the basis of the original protein or polypeptide.

The "variable region" of an antibody refers to the antibody light chain variable region (VL) or the antibody heavy chain variable region (VH), alone or in combination. As known in the art, the variable regions of the heavy chain and the light chain each consists of 4 framework regions (FRs) connected by 3 complementarity determining regions (CDRs) (also called hypervariable regions). The CDRs in each chain are held tightly together by FRs, and contribute to the formation of the antigen binding site of the antibody together with the CDRs from the other chain. At least two techniques for determining CDR are available: (1) a method based on cross-species sequence variability (i.e., Kabat et al., Sequences of Proteins of Immunological Interest, (5th edition, 1991, National Institutes of Health, Bethesda MD)); and (2) a method based on the crystallographic study of antigen-antibody complexes (Al-Lazikani et al., J. Molec. Biol. 273:927-948 (1997)). As used herein, CDRs can refer to those determined by either method or a combination of the two methods.

The term "antibody framework" or "FR region" refers to a portion of the variable domain VL or VH, which serves as a scaffold for the antigen binding loop (CDR) of the variable domain. Essentially, it is a variable domain without CDR.

The term "complementarity determining region" and "CDR" refer to a hypervariable region in the variable domain of an antibody that mainly contributes to antigen binding. Generally, three CDRs (HCDR1, HCDR2, HCDR3) are present in each heavy chain variable region, and three CDRs (LCDR1, LCDR2, LCDR3) are present in each light chain variable region. Any one of a variety of well-known schemes can be used to determine the amino acid sequence boundaries of a CDR, including the "Kabat" numbering rules (see Kabat et al. (1991), "Sequences of Proteins of Immunological Interest", 5th edition, Public Health Service, National Institutes of Health, Bethesda, MD), "Chothia" numbering rules (Al-Lazikani et al., (1997) JMB 273:927-948) and ImMunoGenTics (IMGT) numbering rules (Lefranc M. P., Immunologist, 7, 132-136 (1999); Lefranc, M. P. et al., Dev. Comp. Immunol., 27, 55-77 (2003)), etc. For example, for the classical format, following the Kabat rule, the CDR amino acid residue numbers in the heavy chain variable domain (VH) are 31-35 (HCDR1), 50-65 (HCDR2) and 95-102 (HCDR3); the CDR amino acid residue numbers in the light chain variable domain (VL) are 24-34 (LCDR1), 50-56 (LCDR2) and 89-97 (LCDR3). Following the Chothia rule, the CDR amino acid numbers in VH are 26-32 (HCDR1), 52-56 (HCDR2) and 95-102 (HCDR3); and the amino acid residue numbers in VL are 26-32 (LCDR1), 50- 52 (LCDR2) and 91-96 (LCDR3). By combining the CDR definitions of both Kabat and Chothia, the CDRs consist of amino acid residues 26-35 (HCDR1), 50-65 (HCDR2) and 95-102 (HCDR3) in human VH and amino acid residues 24-34 (LCDR1), 50-56 (LCDR2) and 89-97 (LCDR3) in human VL. Following IMGT rules, the CDR amino acid residue numbers in VH are roughly 26-35 (CDR1), 51-57 (CDR2) and 93-102 (CDR3), and the CDR amino acid residue numbers in VL are roughly 27-32 (CDR1), 50-52 (CDR2) and 89-97 (CDR3). Following IMGT rules, the CDR regions of an antibody can be determined by using the program IMGT/DomainGap Align.

"Any CDR variant thereof" in "HCDR1, HCDR2 and HCDR3 regions or any CDR variant thereof" refers to a variant obtained by subjecting any one, or two, or three of the HCDR1, HCDR2 and HCDR3 regions to amino acid mutations.

"Antibody constant region domain" refers to the domain derived from the constant regions of the light chain and heavy chain of an antibody, including CL and CHI, CH2, CH3 and CH4 domains derived from different types of antibodies.

The "epitope" or "antigenic determinant" refers to a site on an antigen where an immunoglobulin or an antibody specifically binds. Epitopes usually include at least 3, 4, 5, 6, 7, 8, 9, 10, 11, 12, 13, 14 or 15 consecutive or non-consecutive amino acids in a unique spatial conformation. See, for example, Epitope Mapping Protocols in Methods in Molecular Biology, Vol. 66, G E. Morris, Ed. (1996).

The terms "specifically bind", "selectively bind", "bind selectively" and "bind specifically" refer to the binding of an antibody to an epitope on a predetermined antigen.

When the term "competition" is used in the context of antigen binding proteins that compete for the same epitope (for example neutralizing antigen binding protein or neutralizing antibody), it means the competition between antigen binding proteins, which is determined by an assay in which the antigen-binding protein to be tested (for example an antibody or antigen-binding fragment thereof) prevents or inhibits (for example reduces) the specific binding of a reference antigen-binding protein (for example a ligand or a reference antibody) to a common antigen. Numerous types of competitive binding assays can be used to determine whether one antigen-binding protein competes with another, these assays are for example: solid phase direct or indirect radioimmunoassay (RIA), solid phase direct or indirect enzyme immunoassay (EIA), sandwich competition assay (see for example Stahli et al., 1983, Methods in Enzymology 9:242-253); solid phase direct biotin-avidin EIA (see for example Kirkland et al., 1986, J. Immunol. 137:3614-3619), solid phase direct labeling assay, solid phase direct labeling sandwich assay (see for example Harlow and Lane, 1988, Antibodies, A Laboratory Manual, Cold Spring Harbor Press); solid phase direct labeling RIA with I-125 labels (see for example Morel et al., 1988, Molec. Immunol. 25: 7-15); solid-phase direct biotin-avidin EIA (see for example Cheung, et al., 1990, Virology 176: 546-552); and directly labeling RIA (Moldenhauer et al., 1990, Scand. J. Immunol. 32:77-82). Generally, the assay involves using purified antigens (the antigens are on a solid surface or on a cell surface) that can bind to unlabeled test antigen-binding proteins and to labeled reference antigen binding proteins. Competitive inhibition is measured by measuring the amount of labels bound to the solid surface or cells in the presence of the antigen-binding protein to be tested. Usually, the antigen binding protein to be tested is present in excess. The antigen binding proteins identified by competition assays (competitive antigen binding proteins) include: antigen binding proteins that bind to the same epitope as the reference antigen binding protein; and antigen binding proteins that binds to adjacent epitopes that are sufficiently close to the binding epitope of the reference antigen binding protein, and the two epitopes sterically hinder each other from binding. Additional details on the methods used to determine competitive binding are provided in the examples herein. Usually when the competitive antigen binding protein is present in excess, it will inhibit (for example reduce) at least 40-45%, 45-50%, 50-55%, 55-60%, 60-65%, 65-70%, 70%-75% or 75% or more of the specific binding of the reference antigen binding protein to the common antigen. In some cases, the binding is inhibited by at least 80-85%, 85-90%, 90-95%, 95-97%, or 97% or more.

The term "affinity" refers to the strength of the interaction between an antibody and an antigen at a single epitope. Within each antigenic site, the variable region of the antibody "arm" interacts with the antigen at multiple amino acid sites through weak non-covalent forces; the greater the interaction, the stronger the affinity. As used herein, the term "high affinity" of an antibody or antigen-binding fragment thereof (e.g. Fab fragment) generally refers to an antibody or antigen-binding fragment with a $K_D$ of $1E^{-9}$ M or less (e.g., a $K_D$ of $1E^{-10}$ M or less, a $K_D$ of $1E^{-11}$ M or less, a $K_D$ of $1E^{-12}$ M or less, a $K_D$ of $1E^{-13}$ M or less, a $K_D$ of $1E^{-14}$M or less, etc.).

The term "KD" or "$K_D$" refers to the dissociation equilibrium constant of a specific antibody-antigen interaction.

Generally, an antibody binds to an antigen with a dissociation equilibrium constant (KD) of less than about $1E^{-8}M$, for example, less than about $1E^{-9}M$, $1E^{-10}M$ or $1E^{-11}$or less, for example, as measured in a BIACORE instrument using surface plasmon resonance (SPR) technology. The smaller the KD value, the greater the affinity is.

The term "nucleic acid molecule" refers to DNA molecules and RNA molecules. The nucleic acid molecule can be single-stranded or double-stranded, but is preferably a double-stranded DNA or mRNA. When a nucleic acid is placed in a functional relationship with another nucleic acid sequence, the nucleic acid is "operably linked." For example, if a promoter or enhancer affects the transcription of a coding sequence, then the promoter or enhancer is operably linked to the coding sequence.

The term "vector" means a construct capable of delivering one or more target genes or sequences and preferably expressing the same in a host cell. Examples of vectors include, but are not limited to, viral vector, naked DNA or RNA expression vector, plasmid, cosmid or phage vector, DNA or RNA expression vector associated with cationic flocculant, DNA or RNA expression vector encapsulated in liposome, and certain eukaryotic cell such as producer cell.

The methods for producing and purifying antibodies and antigen-binding fragments are well known in the prior art, such as Antibody Experiment Technical Guide, Cold Spring Harbor, Chapters 5-8 and 15. For example, mice can be immunized with an antigen or fragment thereof, and the obtained antibody can be renatured and purified, and amino acid sequencing can be performed by using conventional methods. Antigen-binding fragments can also be prepared by using conventional methods. The antibody or antigen-binding fragment according to the present disclosure is genetically engineered to add one or more human FR regions to the non-human CDR regions. The human FR germline sequences can be obtained from the website http://www.imgt.org/ by comparing the IMGT human antibody variable region germline gene database and MOE software, or be obtained from The Immunoglobulin Facts Book, 2001ISBN012441351.

The term "host cell" refers to a cell into which an expression vector has been introduced. Host cells can include bacteria, microorganisms, plant or animal cells. Bacteria that can be easily transformed include members of the *enterobacteriaceae*, for example *Escherichia coli* or *Salmonella* strains; *Bacillaceae*, for example *Bacillus subtilis; Pneumococcus*; *Streptococcus* and *Haemophilus influenzae*. Suitable microorganisms include *Saccharomyces cerevisiae* and *Pichia pastoris*. Suitable animal host cell lines include CHO (Chinese Hamster Ovary Cell Line), HEK293 cells (non-limiting examples such as HEK293E cells) and NS0 cells.

The engineered antibody or antigen-binding fragment can be prepared and purified by conventional methods. For example, the cDNA sequences encoding the heavy chain and light chain can be cloned and recombined into a GS expression vector. The recombinant immunoglobulin expression vectors can stably transfect CHO cells. As an alternative prior art, mammalian expression systems can lead to glycosylation of antibodies, especially at highly conserved N-terminal sites of the Fc region. Stable clones are obtained by expressing antibodies that specifically bind to antigens. Positive clones are expanded in serum-free medium of bioreactors to produce antibodies. The medium into which the antibodies are secreted can be purified by conventional techniques. For example, use Protein A or Protein G Sepharose FF column containing adjusted buffer for purification. Non-specifically bound components are washed away. Then the bound antibodies are eluted by the pH gradient method, and the antibody fragments are detected by SDS-PAGE and collected. The antibodies can be filtered and concentrated by conventional methods. Soluble mixtures and polymers can also be removed by conventional methods, for example molecular sieves and ion exchange. The resulting product needs to be frozen immediately, such as at -70°C, or lyophilized.

"Administering", "administration", "giving" and "treating", when applied to animals, humans, experimental subjects, cells, tissues, organs or biological fluids, refer to providing the exogenous medicament, therapeutic agent, diagnostic agent, composition or manual operation (for example "euthanasia" in the example) to the animals, humans, subjects, cells, tissues, organs or biological fluids. "Giving" and "treating" can refer to for example treatment, pharmacokinetics, diagnosis, research and experimental methods. The treatment of cells includes contacting reagents with cells, and contacting reagents with fluids, in which the fluids are in contact with the cells. "Giving" and "treating" also mean treating for example cells by reagents, diagnosis, binding compositions or by another kind of cells *in vitro* and *ex vivo*. "Treating" when applied to human, veterinary or research subjects, refers to therapeutic treatment, preventing or preventive measures, research and diagnostic applications.

"Treatment" means giving an internal or external therapeutic agent, for example a composition comprising any one of the compounds of the present disclosure, to a subject who has (or is suspected of having, or is susceptible to) one or more disease symptoms on which the therapeutic agent is known to have therapeutic effect. Generally, the therapeutic agent is administered in an amount effective to alleviate one or more disease symptoms in the treated patient or population to induce the regression of such symptoms or inhibit the development of such symptoms to any clinically measured extent. The amount of therapeutic agent that is effective to alleviate any specific disease symptom (also referred to as a "therapeutically effective amount") can vary according to a variety of factors, for example the subject's disease state, age and body weight, and the ability of the medicament to produce the desired therapeutic effect in the subject. Whether the disease symptoms have been alleviated can be evaluated through any clinical testing methods commonly used by doctors or other health care professionals to evaluate the severity or progression of the symptoms. Although the embodiments of the present disclosure (for example treatment methods or products) may be ineffective in alleviating each target disease symptom, but as determined according to any statistical test methods known in the art such as Student t-test, chi-square test, Mann and Whitney's U test, Kruskal-Wallis test (H test), Jonckheere-Terpstra test and Wilcoxon test, they should reduce the target disease symptom in a statistically significant number of subjects.

"Amino acid conservative modification" or "amino acid conservative substitution" refers to the substitution of amino acids in a protein or polypeptide with other amino acids with similar characteristics (for example charge, side chain size, hydrophobicity/hydrophilicity, main chain conformation and rigidity, etc.), thereby allowing frequent changes without changing the biological activity or other required characteristics (for example antigen affinity and/or specificity) of the protein or polypeptide. Those skilled in the art know that, generally, a single amino acid substitution in a non-essential region of a polypeptide does not substantially change the biological activity (see, for example, Watson et al., (1987) Molecular Biology of the Gene, The Benjamin/Cummings Pub. Co., Page 224, (4th edition)). In addition, the substitution of amino acids with similar structure or function is unlikely to disrupt the biological activity. Exemplary amino acid conservative substitutions are shown in the table below:

| Original residue | Conservative substitution |
|---|---|
| Ala(A) | Gly; Ser |
| Arg(R) | Lys; His |
| Asn(N) | Gln; His; Asp |
| Asp(D) | Glu; Asn |
| Cys(C) | Ser; Ala; Val |
| Gln(Q) | Asn; Glu |
| Glu(E) | Asp; Gln |
| Gly(G) | Ala |
| His(H) | Asn; Gln |
| Ile(I) | Leu; Val |
| Leu(L) | Ile; Val |
| Lys(K) | Arg; His |
| Met(M) | Leu; Ile; Tyr |
| Phe(F) | Tyr; Met; Leu |
| Pro(P) | Ala |
| Ser(S) | Thr |
| Thr(T) | Ser |
| Trp(W) | Tyr; Phe |
| Tyr(Y) | Trp; Phe |
| Val(V) | Ile; Leu |

"Effective amount" and "effective dose" refer to the amount of a medicament, compound or pharmaceutical composition necessary to obtain any one or more beneficial or desired therapeutic results. For preventive use, the beneficial or desired results include elimination or reduction of risk, reduction of severity or delay of the disease onset, including the biochemistry, histology and/or behavioral symptoms of the disease, complications thereof and intermediate pathological phenotypes that appear during the development of the disease. For therapeutic applications, the beneficial or desired results include clinical results, for example reducing the incidence of various target antigen-related disorders of the present disclosure or improving one or more symptoms of the disorder, reducing the dose of other agents required to treat the disorder, enhancing the therapeutic effect of another agent, and/or delaying the progression of target antigen-related disorder of the present disclosure of the subject.

"Exogenous" refers to substances produced outside organisms, cells or human bodies according to circumstances. "Endogenous" refers to substances produced inside cells, organisms or human bodies according to circumstances.

"Homology" and "identity" can be interchanged herein and refer to the sequence similarity between two polynucleotide sequences or between two polypeptides. When the positions in the two sequences compared are occupied by the same base or amino acid monomer subunit, for example if each position of two DNA molecules is occupied by adenine, then the molecules are homologous at that position. The homology percentage between two sequences is a function of the number of matching or homologous positions shared by the two sequences divided by the number of positions compared $\times$ 100. For example, in the optimal sequence alignment, if there are 6 matches or homology out of 10 positions in the two sequences, then the two sequences are 60% homologous; if there are 95 matches or homology out of 100 positions in the two sequences, then the two sequences are 95% homologous. Generally, when two sequences are aligned, comparison is made to give the maximum percentage homology. For example, the comparison can be performed by the BLAST algorithm, in which the parameters of the algorithm are selected to give the maximum match between each sequence over the entire length of each reference sequence.

The following references relate to the BLAST algorithm that is often used for sequence analysis: BLAST ALGORITHMS: Altschul, S.F. et al., (1990) J. Mol. Biol. 215:403-410; Gish, W. et al., (1993) Nature Genet. 3:266-272; Madden, T.L. et al., (1996) Meth. Enzymol. 266:131-141; Altschul, S.F. et al., (1997) Nucleic Acids Res. 25:3389-3402; Zhang, J. et al., (1997) Genome Res. 7:649-656. Other conventional BLAST algorithms such as provided by NCBI BLAST are also well known to those skilled in the art.

"Isolated" refers to separation from of its original state, and being in this state means that the designated molecule is substantially free of other biomolecules, for example nucleic acids, proteins, lipids, carbohydrates or other materials, for example cell debris and growth medium. Generally, the term "isolated" is not intended to mean the complete absence of these materials or the absence of water, buffer or salt, unless they are present in an amount that significantly interferes with the experimental or therapeutic use of the compound as described herein.

"Optional" or "optionally" means that the event or circumstance described later can but does not have to occur, and this description includes occasions where the event or circumstance occurs or does not occur.

"Pharmaceutical composition" means a mixture containing one or more of the compounds described in the present disclosure, or a physiologically/pharmaceutically acceptable salt or a prodrug thereof, and other chemical compositions, for example physiological/pharmaceutically acceptable carriers and excipients. The purpose of the pharmaceutical composition is to promote the administration to organisms, which facilitates the absorption of the active ingredient and thereby exerts biological activity.

The term "pharmaceutically acceptable carrier" refers to any inactive substance suitable for use in a formulation for the delivery of antibodies or antigen-binding fragments. The carrier can be an anti-adhesive agent, binder, coating, disintegrant, filler or diluent, preservative (such as antioxidant, antibacterial or antifungal agent), sweetener, absorption delaying agent, wetting agent, emulsifier, buffer, etc. Examples of suitable pharmaceutically acceptable carriers include water, ethanol, polyol (for example glycerol, propanediol, polyethylene glycol, etc.), dextrose, vegetable oil (for example olive oil), saline, buffer, buffered saline, and isotonic agent, for example sugar, polyol, sorbitol and sodium chloride.

In addition, another aspect of the present disclosure relates to methods for immunodetection or determination of target antigens, reagents for immunodetection or determination of target antigens, methods for immunodetection or determination of cells expressing target antigens and diagnostic agents which comprise the monoclonal antibody or antibody fragment of the present disclosure specifically recognizing and binding to a target antigen as an active ingredient, for diagnosing diseases related to target antigen-positive cells.

In the present disclosure, the method used for detecting or measuring the amount of the target antigen can be any known method. For example, it includes immunodetection or measurement methods.

The immunodetection or measurement methods are methods of detecting or measuring the amount of antibody or antigen using labeled antigens or antibodies. Examples of immunodetection or measurement methods include radioimmunoassay (RIA), enzyme immunoassay (EIA or ELISA), fluorescence immunoassay (FIA), luminescence immunoassay, western blotting, physicochemical methods, etc.

The aforementioned diseases related to target antigen-positive cells can be diagnosed by detecting or measuring cells expressing the target antigen using the monoclonal antibody or antibody fragment of the present disclosure.

In order to detect cells expressing the polypeptide, known immunodetection methods can be used, preferably using immunoprecipitation, fluorescent cell staining, immunohistochemical staining, etc. In addition, fluorescent antibody staining method utilizing the FMAT8100HTS system (Applied Biosystem) can be used.

In the present disclosure, there is no particular limitation on the live sample used for detection or measurement of the target antigen, as long as it has the possibility of containing cells expressing the target antigen, for example histocyte, blood, plasma, serum, pancreatic juice, urine, feces, tissue fluid or culture fluid.

According to the required diagnostic method, the diagnostic agent containing the monoclonal antibody or antibody fragment thereof of the present disclosure can also contain reagents for performing antigen-antibody reaction or reagents for detecting the reaction. The reagents used to perform the antigen-antibody reaction include buffer, salt, etc. The reagents used for detection include reagents commonly used in immunodetection or measurement methods, for example a labeled secondary antibody that recognizes the monoclonal antibody, antibody fragment thereof or conjugate thereof, and a substrate corresponding to the label, etc.

The terms "cancer" and "cancerous" refer to or describe the physiological condition in mammals that is generally characterized by unregulated cell growth/ proliferation. Examples of cancers that can be treated with the bispecific binding molecules of the present disclosure include, but are not limited to, carcinoma, lymphoma, blastoma, sarcoma and leukemia. More specific examples of these cancers that is suggested to be treated with VEGF antagonists in US2008/0014196 include squamous cell carcinoma, small cell lung cancer, non-small cell lung cancer, lung adenocarcinoma, lung squamous cell carcinoma, peritoneal cancer, hepatoma, gastrointestinal cancer, pancreatic cancer, glioblastoma, cervical cancer, ovarian cancer, bladder cancer, hepatoma, breast cancer, colon cancer, colorectal cancer, endometrial or uterine cancer, salivary gland cancer, kidney cancer, prostate cancer, vulvar cancer, thyroid cancer, hepatic carcinoma, gastric cancer, melanoma, skin cancer and various types of head and neck cancers. Abnormal regulation of angiogenesis can lead to many conditions that can be treated by the compositions and methods of the present disclosure. These conditions include both non-neoplastic symptoms and neoplastic symptoms. Neoplastic conditions include but are not limited to the aforementioned conditions.

Non-neoplastic conditions include but are not limited to those treated with VEGF antagonists as described in US2008/0014196: undesired or abnormal hypertrophy, arthritis, rheumatoid arthritis (RA), psoriasis, psoriatic plaque, sarcoidosis, atherosclerosis, atherosclerotic plaque, diabetic and other proliferative retinopathy (including retinopathy of prematurity, retrolental fibroplasia, neovascular glaucoma, age-related macular degeneration, diabetic macular edema, corneal neovascularization, corneal graft neovascularization, corneal graft rejection, retinal/choroid neovascularization, angulus iridocornealis neovascularization (rubeosis), ocular neovascular disease, vascular restenosis, arteriovenous malformations (AVM), meningioma, hemangioma, angiofibroma, thyroid hyperplasia (including Grave's disease)), corneal and other tissue transplantation, chronic inflammation, pneumonia, acute lung injury/ARDS, sepsis, primary pulmonary hypertension, malignant pulmonary effusion, cerebral edema (e.g. related with acute stroke/closed head injury/trauma), synovia inflammation, pannus formation in rheumatoid arthritis (RA), myositis ossificans, hypertrophic bone formation, osteoarthritis (OA), refractory ascites, polycystic ovarian disease, endometriosis, 3rd spacing of fluid disease (pancreatitis, compartment syndrome, burns and bowel disease), uterine fibroids, premature birth, chronic inflammation such as IBD (Crohn's disease and ulcerative colitis), renal allograft rejection, inflammatory bowel disease, nephrotic syndrome, undesired or abnormal tissue growth (non-cancerous), hemophilic joint, hypertrophic scar, hair growth inhibition, Osier-Weber syndrome, pyogenic granuloma retrolental fibroplasias, scleroderma, trachoma, vascular adhesion, synovitis, dermatitis, preeclampsia, ascites, pericardial effusion (e.g. pericardial effusion related to pericarditis) and pleural effusion. The present disclosure is further described below in combination with the examples, but these examples do not limit the scope of the present disclosure. The experimental methods that do not specify specific conditions in the examples of the present disclosure usually follow conventional conditions, such as Antibodies: A Laboratory Manual and Molecular Cloning Manual, Cold Spring Harbor; or according to the conditions recommended by the raw material or commodity manufacturer. The reagents without specific sources are the conventional reagents purchased on the market.

## Example 1. Expression of ANG2 and ANG2 receptor Tie2

The sequences encoding the extracellular region of human ANG2 and human ANG2 receptor Tie2 with human IgG1-Fc tag were inserted into phr vectors to construct an expression plasmid, which was then transfected into HEK293. The specific transfection steps were as follows: one day before transfection, HEK293E cells were seeded in freestyle expression medium (containing 1% FBS, Gibco, 12338-026) at $1 \times 10^6$/ml and placed on a 37°C constant temperature shaker (120 rpm) for continuous culture for 24 hours. After 24 hours, the transfection plasmid and the transfection reagent PEI were sterilized with 0.22 $\mu$m filters, then the transfection plasmid was adjusted to 100 $\mu$g/100 ml cells, the mass ratio of PEI (1 mg/ml) and plasmid was 2:1. 10 ml of Opti-MEM and 200 $\mu$g plasmid were taken and mixed well, and let stand for 5 min; another 10 ml of Opti-MEM and 400 $\mu$g PEI were taken and mixed well, and let stand for 5 min. The plasmid and PEI were mixed well and let stand for 15 min. The mixture of plasmid and PEI was slowly added to 200 ml HEK293E cells, and placed on a shaker at 8% $CO_2$, 120 rpm and 37°C for culturing. On day 3 of transfection, the culture was supplemented with 10% volume of supplementary medium (20 mM glucose + 2 mM L-glutamic acid). Until day 6 of transfection, samples were taken and centrifuged at 4500 rpm for 10 min to collect the cell supernatant. The supernatant with recombinant ANG2 or Tie2 receptor protein was purified as described in Example 2. The purified protein could be used in the following examples or test examples.

Among them, the amino acid sequence of human ANG2 is as shown in SEQ ID NO: 1, and the amino acid sequence of Tie2 extracellular region Fc fusion protein is shown in SEQ ID NO: 2.

The relevant sequences are as follows:

    (1) The amino acid sequence of human ANG2 with human Fc tag

YNNFRKSMDSIGKKQYQVQHGSCSYTFLLPEMDNCRSSSSPYVSNAVQRDAPL

EYDDSVQRLQVLENIMENNTQWLMKLENYIQDNMKKEMVEIQQNAVQNQTAV

MIEIGTNLLNQTAEQTRKLTDVEAQVLNQTTRLELQLLEHSLSTNKLEKQILDQT

SEINKLQDKNSFLEKKVLAMEDKHIIQLQSIKEEKDQLQVLVSKQNSIIEELEKKI

VTATVNNSVLQKQQHDLMETVNNLLTMMSTSNSAKDPTVAKEEQISFRDCAEV

FKSGHTTNGIYTLTFPNSTEEIKAYCDMEAGGGGWTIIQRREDGSVDFQRTWKE

YKVGFGNPSGEYWLGNEFVSQLTNQQRYVLKIHLKDWEGNEAYSLYEHFYLSS

EELNYRIHLKGLTGTAGKISSISQPGNDFSTKDGDNDKCICKCSQMLTGGWWFD

ACGPSNLNGMYYPQRQNTNKFNGIKWYYWKGSGYSLKATTMMIRPADFDIEG

RMD*EPKSSDKTHTCPPCPAPELLGGPSVFLFPPKPKDTLMISRTPEVTCVVVDVSHE*

*DPEVKFNWYVDGVEVHNAKTKPREEQYNSTYRVVSVLTVLHQDWLNGKEYKCKVS*

*NKALPAPIEKTISKAKGQPREPQVYTLPPSRDELTKNQVSLTCLVKGFYPSDIAVEWE*

*SNGQPENNYKTTPPVLDSDGSFFLYSKLTVDKSRWQQGNVFSCSVMHEALHNHYTQ*

*KSLSLSPGKHHHHHH*

<div align="right">SEQ ID NO：1</div>

Note: the underlined part represents the full-length sequence of the ANG2 protein, the dotted line represents the linker, and the italicized part represents the human IgGlFc tag.
(2) The amino acid sequence of Tie2 with human Fc tag

AMDLILINSLPLVSDAETSLTCIASGWRPHEPITIGRDFEALMNQHQDPLEVTQD

VTREWAKKVVWKREKASKINGAYFCEGRVRGEAIRIRTMKMRQQASFLPATLT

MTVDKGDNVNISFKKVLIKEEDAVIYKNGSFIHSVPRHEVPDILEVHLPHAQPQD

AGVYSARYIGGNLFTSAFTRLIVRRCEAQKWGPECNHLCTACMNNGVCHEDTG

ECICPPGFMGRTCEKACELHTFGRTCKERCSGQEGCKSYVFCLPDPYGCSCATG

WKGLQCNEACHPGFYGPDCKLRCSCNNGEMCDRFQGCLCSPGWQGLQCERE

GIPRMTPKIVDLPDHIEVNSGKFNPICKASGWPLPTNEEMTLVKPDGTVLHPKDF

NHTDHFSVAIFTIHRILPPDSGVWVCSVNTVAGMVEKPFNISVKVLPKPLNAPNV

IDTGHNFAVINISSEPYFGDGPIKSKKLLYKPVNHYEAWQHIQVTNEIVTLNYLEP

RTEYELCVQLVRRGEGGEGHPGPVRRFTTASIGLPPPRGLNLLPKSQTTLNLTWQ

PIFPSSEDDFYVEVERRSVQKSDQQNIKVPGNLTSVLLNNLHPREQYVVRARVN

TKAQGEWSEDLTAWTLSDILPPQPENIKISNITHSSAVISWTILDGYSISSITIRYKV

QGKNEDQHVDVKIKNATITQYQLKGLEPETAYQVDIFAENNIGSSNPAFSHELVT

LPESQAPADLGGGK*EPKSCDKTHTCPPCPAPELLGGPSVFLFPPKPKDTLMISRTP*

*EVTCVVVDVSHEDPEVKFNWYVDGVEVHNAKTKPREEQYNSTYRVVSVLTVLHQDW*

*LNGKEYKCKVSNKALPAPIEKTISKAKGQPREPQVYTLPPSRDELTKNQVSLTCLVKG*

*FYPSDIAVEWESNGQPENNYKTTPPVLDSDGSFFLYSKLTVDKSRWQQGNVFSCSVM*

*HEALHNHYTQKSLSLSP*

<div align="right">SEQ ID NO：2</div>

Note: the underlined part represents the extracellular region of Tie2, and the italicized part represents the human IgG1 Fc tag.

**Example 2. Purification of Fc-tagged recombinant protein or antibody by Protein A affinity chromatography**

The antibody or ANG2 and Tie2 supernatant samples expressed by the cells were centrifuged at high speed to remove impurities and purified by a Protein A column. The column was washed with PBS until the A280 reading dropped to baseline. The target protein was eluted with 100 M acetate buffer pH 3.5, and neutralized with 1 M Tris-HCl pH 8.0. The eluted sample was appropriately concentrated and further purified by using PBS-balanced gel chromatography Superdex200 (GE). After electrophoresis, peptide map and LC-MS, the obtained protein was identified as correct and aliquoted for later use.

**Example 3. Construction and identification of the cell line expressing recombinant ANG2 receptor Tie2**

A CHO-K1/Tie2 cell line expressing Tie2 was constructed in the present disclosure for the screening of functional antibodies.
The human Tie2 full-length gene was cloned into the mammalian cell expression vector pBABE. HEK293T cells (ATCC, CRL-3216) were co-transfected with three plasmids pVSV-G, pGag-pol and pBABE-Tie2 to package the virus. 48 hours after transfection, the viruses were collected to infect CHOK1 cells (ATCC, CRL-9618).
72 hours after infection, 10 μg/ml puromycin were used for selection under pressure. After the colonies were expanded and grown, the cells were digested, and the expression level was detected with FACS. The positive rate was about 40%, and then the monoclonal cells were sorted to obtain a single clone 1B11 expressing Tie2.

**Example 4. Preparation and screening of anti-human ANG2 single domain antibodies**

In the present disclosure, an immune library was obtained by immunizing llama, and then the immune library was enriched and screened for llama-derived monoclonal antibodies against human ANG2. The obtained antibodies can specifically bind to ANG2 and have cross-reactivity with cyno and mouse Ang2, can block the binding of ANG2 to its receptor and can inhibit ANG2-mediated phosphorylation of Tie2.
Specifically, the llamas were immunized with human Ang2-Fc protein mixed with Freund's adjuvant, with 250 μg of protein used for each immunization, once every three weeks. After a total of 4 immunizations, 200 ml of blood was collected to isolate PBMC, and total RNA was extracted from the PBMC, which was then reverse transcribed into cDNA, and the cDNA was used as a template to amplify antibody gene sequences. The antibody gene was linked into a phagemid vector by restriction enzyme digestion and ligation, and was electro-transformed into TG1 competent cells. After two rounds of enrichment with 5 nM and 2 nM biotin-hAng2-His (sinobiological, 10691-H07H), the enriched library was screened by, ELISA binding to human ANG2 (see Test Example 1), blocking the binding of ANG2 and Tie2 (see Test Example 2, Test Example 3), and cross-binding with murine ANG2 (see Test Example 1). A variety of single domain antibodies with excellent activity were screened out, for example nano14 and nano15, the specific sequences of which are shown in SEQ ID NO: 3 and SEQ ID NO: 4 respectively.

Llama-derived single domain antibody nano14

DVQLQESGGGLVQPGGSLRLSCAASGFTFDDFGMSWVRQAPGKGLEWVSSIT

WNGGSTYYADSVKGRFTISRDNAKNTVYLQMNSLKPEDTAIYYCNADHPQGY

WGQGTQVTVSS

SEQ ID NO: 3

Llama-derived single domain antibody nano15

DVQLQESGGGLVQPGGSLRLSCAASGFTFNSYAMSWVRQAPGKGLEWVSTINS
GGGRTGYADSVKGRFTISRDNAKNTLYLQMNSLKPEDTAIYYCNADHPQGYWG
QGTQVTVSS

SEQ ID NO: 4

The CDR sequences of llama-derived single domain antibodies in the present disclosure were determined and annotated by the Kabat criteria, and the specific sequences are described in Table 1.

Table 1. CDR region sequences of the llama-derived antibodies

| Antibody | CDR1 | CDR2 | CDR3 |
|---|---|---|---|
| nano14 | DFGMS (SEQ ID NO:5) | SITWNGGSTYYADSVKG (SEQ ID NO:6) | DHPQGY (SEQ ID NO:7) |
| nano15 | SYAMS (SEQ ID NO:8) | TINSGGGRTGYADSVKG (SEQ ID NO:9) | DHPQGY (SEQ ID NO:7) |

**Example 5. CDR mutation and humanization of anti-human ANG2 single domain antibodies**

1. Design of CDR mutations

The CDR2 of the single domain antibody nano14was mutated to obtain the single domain antibodies nano14.1 and nano14.2 comprising the new CDR2 sequence, as shown below:

Table 2. CDR sequences of the single domain antibodies obtained after mutation (determined by Kabat criteria)

| Antibody | CDR1 | CDR2 | CDR3 |
|---|---|---|---|
| nano14.1 | DFGMS (SEQ ID NO:5) | SITWGGGSTYYADSVKG (SEQ ID NO:10) | DHPQGY (SEQ ID NO:7) |
| nano14.2 | DFGMS (SEQ ID NO:5) | SITWSGGSTYYADSVKG (SEQ ID NO:11) | DHPQGY (SEQ ID NO:7) |

The full-length sequences of nano14.1 and nano14.2 obtained after mutation are as shown below:

Llama nano14.1:

DVQLQESGGGLVQPGGSLRLSCAASGFTFD DFGMSWVRQAPGKGLEWVS
SITWGGGSTYYADSVKGRFTISRDNAKNTVYLQMNSLKPEDTAIYYCNADHPQ
GYWGQGTQVTVSS

(SEQ ID NO: 12)

Llama nano14.2:

DVQLQESGGGLVQPGGSLRLSCAASGFTFD DFGMSWVRQAPGKGLEWVS
SITWSGGSTYYADSVKGRFTISRDNAKNTVYLQMNSLKPEDTAIYYCNADHPQ
GYWGQGTQVTVSS

(SEQ ID NO: 13)

It can be seen from the above that the CDR sequences of the anti-ANG2 single domain antibodies in the present disclosure are highly similar, and the general formula sequence of which is as shown below:

Table 3. The CDR general formula of llama single domain antibody

| CDR1 | CDR2 | CDR3 |
|---|---|---|
| $X_1X_2X_3$MS (SEQ ID NO:14) | $X_4$I$X_5X_6X_7$GG$X_8$T$X_9$YADSVKG (SEQ ID NO:15) | DHPQGY (SEQ ID NO:7) |

The general formula of the obtained llama anti-ANG2 single domain antibody is:

DVQLQESGGGLVQPGGSLRLSCAASGFTF$\underline{X_{10}X_1X_2X_3}$MSWVRQAPGKGLE
WVS$\underline{X_4IX_5X_6X_7}$GG$\underline{X_8}$T$\underline{X_9}$YADSVKGRFTISRDNAKNT$\underline{X_{11}}$YLQMNSLKPEDTAIYY
CNA$\underline{DHPQGY}$WGQGTQVTVSS

SEQ ID NO: 16

wherein, $X_1$ is selected from D or S; $X_2$ is selected from F or Y; $X_3$ is selected from G or A; $X_4$ is selected from S or T; $X_5$ is selected from T or N; $X_6$ is selected from W or S; $X_7$ is selected from N, G or S; $X_8$ is selected from R or S; $X_9$ is selected from Y or G; $X_{10}$ is selected from D or N, and $X_{11}$ is selected from V or L.

2. Humanization of the llama-derived anti-human ANG2 single domain antibodies

In order to reduce the immunogenicity of llama-derived antibodies, the screened antibodies with excellent *in vivo* and *in vitro* activities were humanized in the present disclosure.

The humanization of the llama-derived anti-human ANG2 single domain antibodies was performed according to the methods published in many documents in the art. Briefly speaking, as the camel-derived single domain antibodies have only one heavy chain variable region, their CDRs were grafted to the human heavy chain template with the highest homology, and the FR region was subjected to back mutations at the same time. "Grafted" represents grafting the CDR region of the llama-derived antibody to the FR of the template sequence, and the positions of the back mutations were determined according to the Kabat criteria.

2.1 Selection of the human FR region of nano14and back mutations of key amino acids

IGHV3-23*04 was selected as the template for the VH of nano14, and IGHJ6*01 was selected as the template for the J region (FR4). CDRs of nano14 were grafted to the human template, and the embedded residues and the residues with direct interaction with the CDR regions were found by using MOE software and were subjected to back mutation to design humanized antibodies with different heavy chain variable regions, as shown in Table 4.

Table 4. Design of back mutations of nano14

| Antibody | Back mutations |
|---|---|
| hu14.A | Grafted + A93N+K94A |
| hu14.1A | Grafted + A93N+K94A |
| hu14.2A | Grafted + A93N+K94A |
| hu14.1B | Grafted + A93N+K94A +R83K+A84P |
| hu14.2B | Grafted + A93N+K94A +R83K+A84P |
| Note: for example, A93N represents that according to the Kabat criteria, A at position 93 is mutated back to N. Grafted represents that the llama antibody CDRs were implanted into the human germline FR region sequences. | |

The antibody sequences obtained after humanization of the llama anti-ANG2 nano14 antibody were as shown below, wherein the determination of the amino acid residues in the CDR regions was determined and annotated by the Kabat criteria.

> hul4.A (SEQ ID NO: 17)

EVQLVESGGGLVQPGGSLRLSCAASGFTFS<u>DFGMS</u>WVRQAPGKGLEWVS<u>SITW</u>
<u>NGGSTYYADSVK</u>GRFTISRDNSKNTLYLQMNSLRAEDTAVYYCNA<u>DHPQGY</u>W
GQGTTVTVSS

> hu14.1A (SEQ ID NO: 18)

EVQLVESGGGLVQPGGSLRLSCAASGFTFS<u>DFGMS</u>WVRQAPGKGLEWVS<u>SITW</u>
<u>GGGSTYYADSVK</u>GRFTISRDNSKNTLYLQMNSLRAEDTAVYYCNA<u>DHPQGY</u>W
GQGTTVTVSS

>hu14.2A (SEQ ID NO: )9)

EVQLVESGGGLVQPGGSLRLSCAASGFTFS<u>DFGMS</u>WVRQAPGKGLEWVS<u>SITW</u>
<u>SGGSTYYADSVK</u>GRFTISRDNSKNTLYLQMNSLRAEDTAVYYCNA<u>DHPQGY</u>WG
QGTTVTVSS

>hu14.1B (SEQ ID NO: 20)

EVQLVESGGGLVQPGGSLRLSCAASGFTFS<u>DFGMS</u>WVRQAPGKGLEWVS<u>SITW</u>
<u>GGGSTYYADSVK</u>GRFTISRDNSKNTLYLQMNSLKPEDTAVYYCNA<u>DHPQGY</u>W
GQGTTVTVSS

> hu14.2B (SEQ ID NO: 21)

EVQLVESGGGLVQPGGSLRLSCAASGFTFS<u>DFGMS</u>WVRQAPGKGLEWVS<u>SITW</u>
<u>SGGSTYYADSVK</u>GRFTISRDNSKNTLYLQMNSLKPEDTAVYYCNA<u>DHPQGY</u>WG
QGTTVTVSS

The aforementioned VHH was fused to the C-terminus of the ranibizumab heavy chain variable region + IgG1 heavy chain constant region as shown in SEQ ID NO: 31, to form the heavy chain of the bispecific antibody.

2.2 Selection and back mutations of the human FR region of nano15

IGHV3-23*04 was selected as the template for the VH of nano15, and IGHJ6*01 was selected as the template for the J region (FR4). CDRs of nano15 were grafted to the human template, and the embedded residues and the residues with direct interaction with the CDR region were found by using MOE software and were subjected to back mutation to design humanized antibodies with different heavy chain variable regions, as shown in Table 5.

Table 5. Template selection and design of back mutations of nano15

| Antibody | Back mutations |
|---|---|
| hu15.A | Grafted |
| hu15.B | Grafted + A93N, K94A |
| hu15.C | Grafted +V5Q, A93N, K94A |
| hu15.D | Grafted +V5Q, S30N, A93N, K94A |
| hu15.E | Grafted + A84P, A93N, K94A |
| Note: for example, A93N represents that according to the Kabat criteria, A at position 93 was mutated back to N. Grafted represents that the llama antibody CDRs were implanted into the human germline FR region sequence. | |

The specific sequences of the variable region of the humanized nano15 antibodies are as follows:

> hu15.A (SEQ ID NO: 22)

EVQLVESGGGLVQPGGSLRLSCAASGFTFSSYAMSWVRQAPGKGLEWVSTINSGGGRTGYADSVKGRFTISRDNSKNTLYLQMNSLRAEDTAVYYCAKDHPQGYWGQGTTVTVSS

> hu15.B (SEQ ID NO: 23)

EVQLVESGGGLVQPGGSLRLSCAASGFTFSSYAMSWVRQAPGKGLEWVSTINSGGGRTGYADSVKGRFTISRDNSKNTLYLQMNSLRAEDTAVYYCNADHPQGYWGQGTTVTVSS

> hu15.C (SEQ ID NO: 24)

EVQLQESGGGLVQPGGSLRLSCAASGFTFSSYAMSWVRQAPGKGLEWVSTINSGGGRTGYADSVKGRFTISRDNSKNTLYLQMNSLRAEDTAVYYCNADHPQGYWGQGTTVTVSS

>hu15.D (SEQ ID NO: 25)

EVQLQESGGGLVQPGGSLRLSCAASGFTFNSYAMSWVRQAPGKGLEWVSTINSGGGRTGYADSVKGRFTISRDNSKNTLYLQMNSLRAEDTAVYYCNADHPQGYWGQGTTVTVSS

> hu15.E (SEQ ID NO: 26)

EVQLVESGGGGLVQPGGSLRLSCAASGFTFS$\underline{SYAMS}$WVRQAPGKGLEWV$\underline{STINS}$
$\underline{GGGRTGYADSVKG}$RFTISRDNSKNTLYLQMNSLRPEDTAVYYCNA$\underline{DHPQGY}$W
GQGTTVTVSS

The full-length general formula sequence of the single domain antibodies obtained after humanization is as follows:

EVQL$X_{12}$ESGGGGLVQPGGSLRLSCAASGFTF$X_{13}$$\underline{X_1 X_2 X_3 MS}$WVRQAPGKGLE

WVS$\underline{X_4 I X_5 X_6 X_7 GGX_8 TX_9 YADSVKG}$RFTISRDNSKNTLYLQMNSL$X_{14}X_{15}$EDTAVY

YC $X_{16}$ $X_{17}$$\underline{DHPQGY}$WGQGTTVTVSS

（SEQ ID NO：27）

wherein, $X_1$ is selected from D or S; $X_2$ is selected from F or Y; $X_3$ is selected from G or A; $X_4$ is selected from S or T; $X_5$ is selected from T or N; $X_6$ is selected from W or S; $X_7$ is selected from N, G or S; $X_8$ is selected from R or S; $X_9$ is selected from Y or G; $X_{12}$ is selected from V or Q, $X_{13}$ is selected from S or N, $X_{14}$ is selected from R or K, $X_{15}$ is selected from A or P, $X_{16}$ is selected from A or N, and $X_{17}$ is selected from K or A.

**Example 6. Preparation and identification of anti-VEGF/ANG2 bispecific antibodies**

The anti-VEGF antibody present in the bispecific antibody of the present disclosure can be any currently available antibody against VEGF, such as Avastin, RAZUMAB (Axxiom Inc), GNR-011 (Affitech A/S), R-TPR-024 (Reliance Life Sciences Grou), ramucirumab (ImClone Systems), etc. An exemplary antibody is Genentech's Fab antibody ranibizumab (Lucentis), the light chain variable region sequence of which is as shown in SEQ ID NO: 28 (see WO1998045332 or CAS Registry Number: 347396-82-1). The heavy chain of the anti-VEGF antibody is a complete IgG1 heavy chain formed by combining the heavy chain variable region of ranibizumab (as shown in SEQ ID NO: 30, see WO1998045332) with human IgG1 constant region, and the terminal K was mutated to G. The specific sequence is as shown in SEQ ID NO: 31. The relevant sequences are as follows:
(1) The light chain variable region of ranibizumab

DIQLTQSPSSLSASVGDRVTITC$\underline{SASQDISNYLN}$WYQQKPGKAPKVLIY$\underline{FTSSLHS}$GVPS
RFSGSGSGTDFTLTISSLQPEDFATYYC$\underline{QQYSTVPWT}$FGQGTKVEIK

SEQ ID NO: 28

(2) The light chain of ranibizumab

DIQLTQSPSSLSASVGDRVTITC$\underline{SASQDISNYLN}$WYQQKPGKAPKVLIY$\underline{FTSSLHS}$GVPS
RFSGSGSGTDFTLTISSLQPEDFATYYC$\underline{QQYSTVPWT}$FGQGTKVEIK$\underline{RTVAAPSVFIFP}$
$\underline{PSDEQLKSGTASVVCLLNNFYPREAKVQWKVDNALQSGNSQESVTEQDSKDST}$
$\underline{YSLSSTLTLSKADYEKHKVYACEVTHQGLSSPVTKSFNRGEC}$

SEQ ID NO: 29

(3) The heavy chain variable region of ranibizumab

*EVQLVESGGGLVQPGGSLRLSCAASGYDFT*HYGMN*WVRQAPGKGLEWVG*WINTY TGEPTYAADFKR*RFTFSLDTSKSTAYLQMNSLRAEDTAVYYCAK*YPYYYGTSHWY FDV*WGQGTLVTVSS*

SEQ ID NO: 30

(4) The heavy chain variable region of ranibizumab + IgG1 constant region (heavy chain)

*EVQLVESGGGLVQPGGSLRLSCAASGYDFT*HYGMN*WVRQAPGKGLEWVG*WINTY TGEPTYAADFKR*RFTFSLDTSKSTAYLQMNSLRAEDTAVYYCAK*YPYYYGTSHWY FDV*WGQGTLVTVSS*ASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV

DKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGK EYKCKVSNKALPAPIEKTISKAKGQPREPQVYTLPPSRDELTKNQVSLTCLVKG

FYPSDIAVEWESNGQPENNYKTTPPVLDSDGSFFLYSKLTVDKSRWQQGNVFS CSVMHEALHNHYTQKSLSLSP

SEQ ID NO: 31

(5) The heavy chain variable region of ranibizumab + IgG1 constant region variant (heavy chain)

*EVQLVESGGGLVQPGGSLRLSCAASGYDFT*HYGMN*WVRQAPGKGLEWVG*WINTY TGEPTYAADFKR*RFTFSLDTSKSTAYLQMNSLRAEDTAVYYCAK*YPYYYGTSHWYF DV*WGQGTLVTVSS*ASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHTCPPCPAPEAAGGPSVFLFPPKPKDTLMASRTPEVTCVVVDVSH EDPEVKFNWYVDGVEVHNAKTKPREEQYNSTYRVVSVLTVLAQDWLNGKEY KCKVSNKALPAPIEKTISKAKGQPREPQVYTLPPSRDELTKNQVSLTCLVKGFYP SDIAVEWESNGQPENNYKTTPPVLDSDGSFFLYSKLTVDKSRWQQGNVFSCSVM HEALHNAYTQKSLSLSP

SEQ ID NO: 54

Note: The order is FR1-CDR1-FR2-CDR2-FR3-CDR3-FR4. In the sequence, italicized represents the FR sequences, underlined represents the CDR sequences, and dotted line represents the constant region sequences.

Table 6. Sequences of CDR regions of ranibizumab

| HCDR1 | HYGMN (SEQ ID NO:32) | LCDR1 | SASQDISNYLN (SEQ ID NO:35) |
|---|---|---|---|
| HCDR2 | WINTYTGEPTYAADFKR (SEQ ID NO:33) | LCDR2 | FTSSLHS (SEQ ID NO:36) |
| HCDR3 | YPYYYGTSHWYFDV (SEQ ID NO:34) | LCDR3 | QQYSTVPWTF (SEQ ID NO:37) |

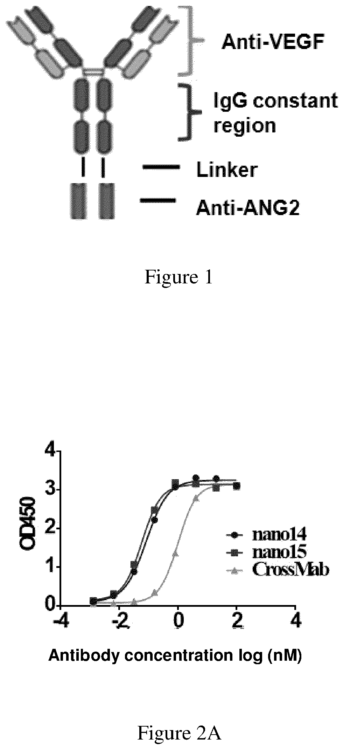

The N-terminal amino acid of the anti-ANG2 single domain antibody of the present disclosure was connected directly (through peptide bonds) or indirectly through a linker (such as GG) to the C-terminal amino acid of the anti-VEGF antibody heavy chain by using homologous recombination technology, and conventionally expressed through the 293 expression system to obtain the bispecific antibodies. The schematic structure of the bispecific antibodies is shown in Figure 1.

Table 7. Schematic structure of ANG2/VEGF bispecific antibodies

| Name | Schematic structure Ab*-linker- ANG2 |
|---|---|
| Bispecific antibody 1 | Ab*-linker- hu14.A |
| Bispecific antibody 2 | Ab*-linker- hu14.B |
| Bispecific antibody 3 | Ab*-linker- hu14.C |
| Bispecific antibody 4 | Ab*-linker- hu14.D |
| Bispecific antibody 5 | Ab*-linker- hu14.E |
| Bispecific antibody 6 | Ab*-linker- hu15.A |
| Bispecific antibody 7 | Ab*-linker- hu15.B |
| Bispecific antibody 8 | Ab*-linker- hu15.C |
| Bispecific antibody 9 | Ab*-linker- hu15.D |
| Bispecific antibody 10 | Ab*-linker- hu15.E |
| *Note: Ab can be any kind of anti-VEGF antibody. | |

The anti-ANG2 single domain antibody can be connected to the amino terminus or carboxyl terminus of the heavy chain of the anti-VEGF antibody, or to the amino terminus of the light chain of the anti-VEGR antibody. It has been verified that the bispecific antibodies obtained by connecting the anti-ANG2 single domain antibody to the carboxyl terminus of the anti-VEGF antibody heavy chain have better stability.

Exemplarily, different anti-ANG2 single domain antibodies were connected at amino terminus to the carboxyl terminus of the heavy chain of ranibizumab through a linker, such as $(G)_n$ (n>=1) linker, to form the following bispecific antibodies:

Table 8. Sequences of the bispecific antibodies

| Name | The first chain (including the heavy chain portion) | The second chain (including the light chain portion) |
|---|---|---|
| hu14.A-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSP*GG GEVQLVESGGGLVQPGGSLRLSCAASGFTFSD FGMSWVRQAPGKGLEWVSSITWNGGSTYYADS VKGRFTISRDNSKNTLYLQMNSLRAEDTAVYYC NADHPQGYWGQGTTVTVSS* (SEQ ID NO: 38) | *DIQLTQSPSSLSAS VGDRVTITCSASQ DISNYLNWYQQK PGKAPKVLIYFTSS LHSGVPSRFSGSG SGTDFTLTISSLQP EDFATYYCQQYST VPWTFGQGTKVEI KRTVAAPSVFIFPP SDEQLKSGTASV VCLLNNFYPREA KVQWKVDNALQ SGNSQESVTEQDS KDSTYSLSSTLTL SKADYEKHKVYA CEVTHQGLSSPVT KSFNRGEC* (SEQ ID NO: 29) |

(continued)

| Name | The first chain (including the heavy chain portion) | The second chain (including the light chain portion) |
|---|---|---|
| hu14.1A-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG *GEVQLVESGGGLVQPGGSLRLSCAASGFTFSD FGMSWVRQAPGKGLEWVSSITWGGGSTYYADS VKGRFTISRDNSKNTLYLQMNSLRAEDTAVYYC NADHPQGYWGQGTTVTVSS* (SEQ ID NO: 39) | |
| hu14.2A-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG *GEVQLVESGGGLVQPGGSLRLSCAASGFTFSD FGMSWVRQAPGKGLEWVSSITWGGGSTYYADS VKGRFTISRDNSKNTLYLQMNSLRAEDTAVYYC NADHPQGYWGQGTTVTVSS* (SEQ ID NO: 40) | |

(continued)

| Name | The first chain (including the heavy chain portion) | The second chain (including the light chain portion) |
|------|------------------------------------------------------|-------------------------------------------------------|
| hu14.1B-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG GEVQLVESGGGLVQPGGSLRLSCAASGFTFSD FGMSWVRQAPGKGLEWVSSITWGGGSTYYADS VKGRFTISRDNSKNTLYLQMNSLKPEDTAVYYC NADHPQGYWGQGTTVTVSS (SEQ ID NO: 41) | |
| hu14.2B-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG GEVQLVESGGGLVQPGGSLRLSCAASGFTFSD FGMSWVRQAPGKGLEWVSSITWSGGSTYYADS VKGRFTISRDNSKNTLYLQMNSLKPEDTAVYYC NADHPQGYWGQGTTVTVSS (SEQ ID NO: 42) | |

(continued)

| Name | The first chain (including the heavy chain portion) | The second chain (including the light chain portion) |
|---|---|---|
| hu15.A-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG *GEVQLVESGGGLVQPGGSLRLSCAASGFTFSSY AMSWVRQAPGKGLEWVSTINSGGGRTGYADSV KGRFTISRDNSKNTLYLQMNSLRAEDTAVYYCA KDHPQGYWGQGTTVTVSS* (SEQ ID NO: 43) | |
| hu15.B-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG *GEVQLVESGGGLVQPGGSLRLSCAASGFTFSSY AMSWVRQAPGKGLEWVSTINSGGGRTGYADSV KGRFTISRDNSKNTLYLQMNSLRAEDTAVYYCN ADHPQGYWGQGTTVTVSS* (SEQ ID NO: 44) | |

(continued)

| Name | The first chain (including the heavy chain portion) | The second chain (including the light chain portion) |
|---|---|---|
| hu15.C-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG *GEVQLQESGGGLVQPGGSLRLSCAASGFTFSSY AMSWVRQAPGKGLEWVSTINSGGGRTGYADSV KGRFTISRDNSKNTLYLQMNSLRAEDTAVYYCN ADHPQGYWGQGTTVTVSS* (SEQ ID NO: 45) | |
| hu15.D-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG *GEVQLQESGGGLVQPGGSLRLSCAASGFTFNS YAMSWVRQAPGKGLEWVSTINSGGGRTGYADS VKGRFTISRDNSKNTLYLQMNSLRAEDTAVYYC NADHPQGYWGQGTTVTVSS* (SEQ ID NO:46) | |

(continued)

| Name | The first chain (including the heavy chain portion) | The second chain (including the light chain portion) |
|---|---|---|
| hu15.E-V | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG CLVKDYFPEPVTVSWNSGALTSGVHTFPAV LQSSGLYSLSSVVTVPSSSLGTQTYICNVNH KPSNTKVDKKVEPKSCDKTHTCPPCPAPELL GGPSVFLFPPKPKDTLMISRTPEVTCVVVDV SHEDPEVKFNWYVDGVEVHNAKTKPREEQ YNSTYRVVSVLTVLHQDWLNGKEYKCKVS NKALPAPIEKTISKAKGQPREPQVYTLPPSRD ELTKNQVSLTCLVKGFYPSDIAVEWESNGQP ENNYKTTPPVLDSDGSFFLYSKLTVDKSRW QQGNVFSCSVMHEALHNHYTQKSLSLSPGG G*EVQLVESGGGLVQPGGSLRLSCAASGFTFSSY AMSWVRQAPGKGLEWVSTINSGGGRTGYADSV KGRFTISRDNSKNTLYLQMNSLRPEDTAVYYCN ADHPQGYWGQGTTVTVSS* (SEQ ID NO: 47) | |
| hu15.E-V1 | EVQLVESGGGLVQPGGSLRLSCAASGYDFTH YGMNWVRQAPGKGLEWVGWINTYTGEPTY AADFKRRFTFSLDTSKSTAYLQMNSLRAEDT AVYYCAKYPYYYGTSHWYFDVWGQGTLVT VSSASTKGPSVFPLAPSSKSTSGGTAALGCLV KDYFPEPVTVSWNSGALTSGVHTFPAVLQSS GLYSLSSVVTVPSSSLGTQTYICNVNHKPSNT KVDKKVEPKSCDKTHTCPPCPAPEAAGGPSV FLFPPKPKDTLMASRTPEVTCVVVDVSHEDP EVKFNWYVDGVEVHNAKTKPREEQYNSTY RVVSVLTVLAQDWLNGKEYKCKVSNKALPA PIEKTISKAKGQPREPQVYTLPPSRDELTKNQ VSLTCLVKGFYPSDIAVEWESNGQPENNYKT TPPVLDSDGSFFLYSKLTVDKSRWQQGNVFS CSVMHEALHNAYTQKSLSLSPGGG*EVQLVES GGGLVQPGGSLRLSCAASGFTFSSYAMSWVRQ APGKGLEWVSTINSGGGRTGYADSVKGRFTISR DNSKNTLYLQMNSLRPEDTAVYYCNADHPQGY WGQGTTVTVSS* (SEQ ID NO: 55) | |

(continued)

| Name | The first chain (including the heavy chain portion) | The second chain (including the light chain portion) |
|---|---|---|
| hu14.2B-V1 | EVQLVESGGGLVQPGGSLRLSCAASGYDFT HYGMNWVRQAPGKGLEWVGWINTYTGEPT YAADFKRRFTFSLDTSKSTAYLQMNSLRAE DTAVYYCAKYPYYYGTSHWYFDVWGQGT LVTVSSASTKGPSVFPLAPSSKSTSGGTAALG | |
| | CLVKDYFPEPVTVSWNSGALTSGVHTFPAVL QSSGLYSLSSVVTVPSSSLGTQTYICNVNHKP SNTKVDKKVEPKSCDKTHTCPPCPAPEAAG GPSVFLFPPKPKDTLMASRTPEVTCVVVDVS HEDPEVKFNWYVDGVEVHNAKTKPREEQY NSTYRVVSVLTVLAQDWLNGKEYKCKVSN KALPAPIEKTISKAKGQPREPQVYTLPPSRDE LTKNQVSLTCLVKGFYPSDIAVEWESNGQPE NNYKTTPPVLDSDGSFFLYSKLTVDKSRWQQ GNVFSCSVMHEALHNAYTQKSLSLSPGGG*E VQLVESGGGLVQPGGSLRLSCAASGFTFSDFG MSWVRQAPGKGLEWVSSITWSGGSTYYADSVK GRFTISRDNSKNTLYLQMNSLKPEDTAVYYCNA DHPQGYWGQGTTVTVSS* (SEQ ID NO: 56) | |

Note: the order of the first chain is ranibizumab FR1-CDR1-FR2-CDR2-FR3-CDR3-FR4-CH-linker-single domain antibody FR1-CDR1-FR2-CDR2-FR3-CDR3-FR4. In the sequence, the normal format part represents the FR sequences of the variable region of ranibizumab, the wavy underline represents the CDR sequences of ranibizumab, the dotted line represents the IgG1 heavy chain constant region sequences, the double underline represents the linkers, the italic part represents the single domain antibody sequences and the underline represents the CDR sequences.

According to the purification method in Example 2, bispecific antibody molecules with a purity of >98% can be obtained by purification using protein A affinity chromatography.

At the same time, Roche's VEGF/ANG2 bispecific antibody crossmab (Vanucizumab) was used as a positive control, the sequences of which are as shown in SEQ ID NO: 48 and SEQ ID NO: 49, SEQ ID NO: 50 and SEQ ID NO: 51 (refer to WHO Drug Information, Vol. 29, No. 1, 2015). In addition, Avastin was used as a positive control, the heavy chain sequence of which is as shown in SEQ ID NO: 52, and the light chain sequence is as shown in SEQ ID NO: 53 (refer to CAS Registry Number: 216974-75-3).

>crossmab anti-ANG2 heavy chain

QVQLVQSGAEVKKPGASVKVSCKASGYTFTGYYMHWVRQAPGQGLEWMGWI
NPNSGGTNYAQKFQGRVTMTRDTSISTAYMELSRLRSDDTAVYYCARSPNPYYY
DSSGYYYPGAFDIWGQGTMVTVSSASVAAPSVFIFPPSDEQLKSGTASVVCLLN
NFYPREAKVQWKVDNALQSGNSQESVTEQDSKDSTYSLSSTLTLSKADYEKHK
VYACEVTHQGLSSPVTKSFNRGECDKTHTCPPCPAPELLGGPSVFLFPPKPKDTL
MISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVCTLPPSRDE

LTKNQVSLSCAVKGFYPSDIAVEWESNGQPENNYKTTPPVLDSDGSFFLVSKLTV
DKSRWQQGNVFSCSVMHEALHNHYTQKSLSLSPGK

SEQ ID NO: 48

>crossmab anti-ANG2 light chain

QPGLTQPPSVSVAPGQTARITCGGNNIGSKSVHWYQQKPGQAPVLVVYDDSDRP
SGIPERFSGSNSGNTATLTISRVEAGDEADYYCQVWDSSSDHYVFGTGTKVTVLS
SASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSC

SEQ ID NO: 49

>crossmab anti-VEGF heavy chain

EVQLVESGGGLVQPGGSLRLSCAASGYTFTNYGMNWVRQAPGKGLEWVGWIN
TYTGEPTYAADFKRRFTFSLDTSKSTAYLQMNSLRAEDTAVYYCAKYPHYYGSS
HWYFDVWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPCRDELTKNQVSLWCLV
KGFYPSDIAVEWESNGQPENNYKTTPPVLDSDGSFFLYSKLTVDKSRWQQGNVF
SCSVMHEALHNHYTQKSLSLSPGK

SEQ ID NO: 50

>crossmab anti-VEGF light chain

DIQMTQSPSSLSASVGDRVTITCSASQDISNYLNWYQQKPGKAPKVLIYFTSSLH
SGVPSRFSGSGSGTDFTLTISSLQPEDFATYYCQQYSTVPWTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 51

Note: underlined represents variable region sequences, and the rest represent constant region sequences.

>Avastin heavy chain

EVQLVESGGGLVQPGGSLRLSCAASGYTFTNYGMNWVRQAPGKGLEWVGW
INTYTGEPTYAADFKRRFTFSLDTSKSTAYLQMNSLRAEDTAVYYCAKYPHYYG
SSHWYFDVWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDW
LNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGVF

SCSVMHEALHNHYTQKSLSLSPGK

SEQ ID NO: 52

>Avastin light chain

DIQMTQSPSSLSASVGDRVTITCSASQDISNYLNWYQQKPGKAPKVLIYFTSSLH
SGVPSRFSGSGSGTDFTLTISSLQPEDFATYYCQQYSTVPWTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 53

>RG7716 (prepared by referring to VEGFang2-0016 in CN105143262A):

Anti-VEGF-heavy chain (Knob)

EVQLVESGGGLVQPGGSLRLSCAASGYDFTHYGMNWVRQAPGKGLEWVGWI
NTYTGEPTYAADFKRRFTFSLDTSKSTAYLQMNSLRAEDTAVYYCAKYPYYYG
TSHWYFDVWGQGTLVTVSSASTKGPSVFPLAPSSKSTSGGTAALGCLVKDYFPE

PVTVSWNSGALTSGVHTFPAVLQSSGLYSLSSVVTVPSSSLGTQTYICNVNHKPS
NTKVDKKVEPKSCDKTHTCPPCPAPEAAGGPSVFLFPPKPKDTLMASRTPEVTC
VVVDVSHEDPEVKFNWYVDGVEVHNAKTKPREEQYNSTYRVVSVLTVLAQD
WLNGKEYKCKVSNKALGAPIEKTISKAKGQPREPQVYTLPPCRDELTKNQVSL
WCLVKGFYPSDIAVEWESNGQPENNYKTTPPVLDSDGSFFLYSKLTVDKSRWQQ
GNVFSCSVMHEALHNAYTQKSLSLSPGK

SEQ ID NO: 57

Anti-Ang2 heavy chain (Hole)

QVQLVQSGAEVKKPGASVKVSCKASGYTFTGYYMHWVRQAPGQGLEWMGWI
NPNSGGTNYAQKFQGRVTMTRDTSISTAYMELSRLRSDDTAVYYCARSPNPYYY
DSSGYYYPGAFDIWGQGTMVTVSSASVAAPSVFIFPPSDEQLKSGTASVVCLLN
NFYPREAKVQWKVDNALQSGNSQESVTEQDSKDSTYSLSSTLTLSKADYEKHK
VYACEVTHQGLSSPVTKSFNRGECDKTHTCPPCPAPEAAGGPSVFLFPPKPKDTL
MASRTPEVTCVVVDVSHEDPEVKFNWYVDGVEVHNAKTKPREEQYNSTYRVV
SVLTVLAQDWLNGKEYKCKVSNKALGAPIEKTISKAKGQPREPQVCTLPPSRDE
LTKNQVSLSCAVKGFYPSDIAVEWESNGQPENNYKTTPPVLDSDGSFFLVSKLTV
DKSRWQQGNVFSCSVMHEALHNAYTQKSLSLSPGK

SEQ ID NO: 58

Anti-VEGF light chain

DIQLTQSPSSLSASVGDRVTITCSASQDISNYLNWYQQKPGKAPKVLIYFTSSLHS
GVPSRFSGSGSGTDFTLTISSLQPEDFATYYCQQYSTVPWTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 29

Anti-Ang2 light chain

SYVLTQPPSVSVAPGQTARITCGGNNIGSKSVHWYQQKPGQAPVLVVYDDSDRP
SGIPERFSGSNSGNTATLTISRVEAGDEADYYCQVWDSSSDHWVFGGGTKLTVL
SSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSC

SEQ ID NO: 59

In addition, the negative control (NC) used in the present disclosure refers to an IgG form monoclonal antibody against HIV.

**Biological evaluation of *in vitro* activity Test Example 1. ELISA determination of the affinity of ANG2 antibodies to ANG2 and the same family protein ANG1**

(1) Human ANG2-His binding ELISA

The plate was coated with streptavidin (abcam, ab123480) at a concentration of 1 ng/$\mu$l, 100 $\mu$l per well overnight at 4°C, and then the supernatant was removed. 250 $\mu$l 5% skimmed milk powder was added for blocking at 37°C for 1 h, and the plate was washed with a washing machine for 3 times. 0.5 ng/$\mu$l biotin-hAng2-His (sinobiological, 10691-H07H) was added and incubated at 37°C for 1 h. The plate was washed with a washing machine for 3 times, and 100 $\mu$l 1:1 diluted phage supernatant was added and incubated at 37°C for 1 h. The plate was washed with a washing machine for 3 times, and 100 $\mu$l 1:10000 diluted anti-M13-HRP (GE, 27-9421-01) was added to each well and incubated at 37°C for 1 h. The plate was washed with a washing machine for 3 times, and 100 $\mu$l TMB was added to each well for color development. After 5-10 min, 100 $\mu$l 1 M $H_2SO_4$ was added to each well to stop the color development, and the OD450 value was measured with a microplate reader. The results are shown in Figure 2A.

(2) ANG1 binding ELISA

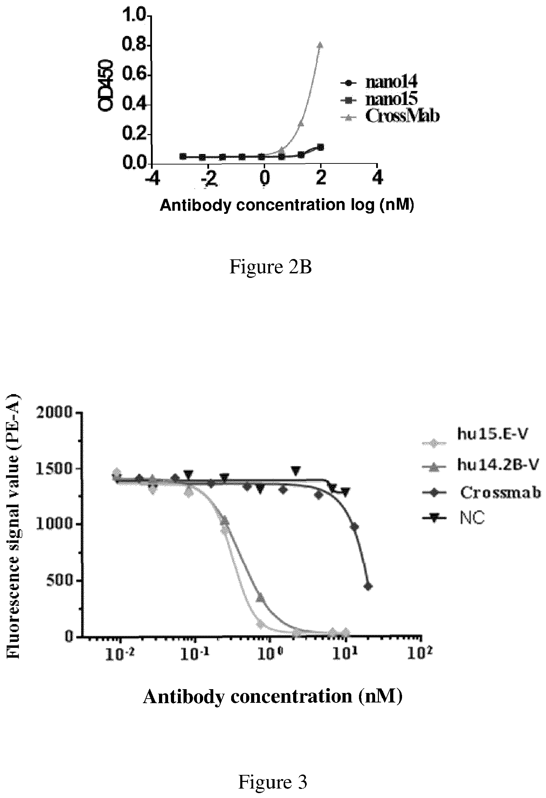

The plate was coated with human ANG1 (RD,923-AN) at a concentration of 1 ng/$\mu$l, 100 $\mu$l per well overnight at 4°C. The supernatant was removed and 250 $\mu$l 5% skimmed milk powder was added for blocking at 37°C for 1 h. The plate was washed with a washing machine for 3 times, and 100 $\mu$l 1:1 diluted phage supernatant was added and incubated at 37°C for 1 h. The plate was washed with a washing machine for 3 times, and 100 $\mu$l 1:10000 diluted M13-HRP was added to each well and incubated at 37°C for 1 h. The plate was washed with a washing machine for 3 times, and 100 μl TMB was added to each well for color development. After 5-10 min, 100 μl 1 M $H_2SO_4$ was added to each well to stop the color development, and the OD450 value was measured with a microplate reader. The results are shown in Figure 2B.

The results show that nano14 and nano15 show very strong binding ability to human ANG2 but do not bind to human ANG1, thus having good selectivity.


**Test Example 2. Biacore measurement of the affinity of VEGF/ANG2 bispecific antibodies with different species of VEGF/ANG2**

The affinity of the humanized VEGF/ANG2 bispecific antibodies to be tested with human, cyno and mouse VEGF and ANG2 was measured by using Biacore T200 (GE) instrument.

The antibodies were affinity captured by using a Protein A biosensor chip, and then the antigens, i.e., human VEGF (R&D, 293-VE), cyno VEGF (sinobiological, 11066), mouse VEGF (sinobiological, 51059), human ANG2 (sinobiological, 10691-H08H) cyno ANG2 (sinobiological, 90026-C07H) and mouse ANG2 (sinobiological, 50298-M07H) flowed through on the surface of the chip. The reaction signals were real-time detected by using Biacore T200 instrument to obtain the binding and dissociation curves. After the dissociation of each experimental cycle was completed, the biosensor chip was washed and regenerated with 10 mM Glycine-HCl regeneration buffer (pH 1.5). The data was fit with a (1:1) Langmuir model by using BIA evaluation version 4.1, GE software to obtain the affinity value, as shown in Table 9.

Table 9. Affinity of the bispecific antibodies with different species of ANG2

| Antibody | Affinity KD (M) | | | | | | |
|---|---|---|---|---|---|---|---|
| | Human ANG2 | Cyno ANG2 | Mouse ANG2 | Human ANG1 | Human VEGF | Cyno VEGF | Mouse VEGF |
| Crossmab | 5.5E-10 | 9E-9 | 7.5E-10 | With binding | 1E-13 | 1E-11 | No binding |
| hu15.E-V | 4E-10 | 7E-9 | 5.1E-10 | No binding | 1.4E-14 | 5E-12 | No binding |
| hu14.2B-V | 1.15E-0 9 | 5.53E-0 8 | 2.64E-0 7 | No binding | 1.48E-1 1 | 4.36E-1 2 | No binding |

The results show that hu15.E-V and hu14.2B-V have relatively high affinity with all species of VEGF (except for mouse VEGF) and ANG2 tested.


**Test Example 3. ELISA-based experiment of the antibodies blocking the binding of ANG2 to Tie2 receptor**

ANG2 binds to the ANG2 receptor Tie2 on the surface of vascular endothelial cells, triggering phosphorylation of tyrosine kinases in Tie2 cells, which then transduces signals to detach peripheral cells from vascular endothelial cells, leaving blood vessels in an unstable and easy to proliferate state. Therefore, blocking the binding of ANG2 to Tie2 by antibodies can make blood vessels more stable and inhibit neovascularization. The identification results of this experiment show that the bispecific antibodies can block the binding of ANG2 to the extracellular domain of the recombinantly expressed Tie2 protein.

Specific methods: the ELISA plate was coated with Tie2-Fc (SEQ ID NO: 2, 3 μg/ml dissolved in PBS, 100 μl/well) overnight at 4°C and the coating solution was removed, 5% skimmed milk blocking solution diluted with PBS was added at 200 μl/well and incubated in a 37°C incubator for 2 h for blocking. After the blocking was completed, the blocking solution was discarded and the plate was washed with PBST buffer (PBS containing 0.05% tween-20, pH 7.4) for 5 times. Then added were 50 μl of huANG2-Fc (SEQ ID NO: 1, bio-huANG2-Fc, final concentration 0.15 μg/ml) labeled with the biotin labeling kit (Dojindo Laboratories, LK03) diluted with 1% BSA and 50 μl of the antibody to be tested (initial concentration 10 μg/ml, 3-fold serial dilution). The solution was mixed well and then incubated at 37°C for 15 min, added to the ELISA plate and incubated at 37°C for 1 h. After the incubation was completed, the reaction solution in the ELISA plate was discarded and the plate was washed 5 times with PBST. Then 1:4000 diluted streptavidin-peroxidase polymer (Sigma, S2438-250UG) was added at 100 μl/well and incubated at 37°C for 1 h. After washing the plate 5 times with PBST, 100 μl/well TMB chromogenic substrate (KPL, 52-00-03) was added and incubated at room temperature for 3-10min, and 1 M $H_2SO_4$ was added at 100 μl/well to stop the reaction. The absorption value was read by using a NOVOStar microplate reader at 450nm and the IC50 value of the antibody blocking the binding of ANG2 to Tie2 was calculated. The results show that both ANG2 single domain antibodies and the bispecific antibodies can strongly inhibit the binding of ANG2 to Tie2 (see Table 10 and Table 11).

Table 10. Results of the single domain antibodies inhibiting the binding of ANG2 to Tie2

| Antibody | nano14 | nano15 | Crossmab |
|---|---|---|---|
| IC50(nM) | 0.03927 | 0.02810 | 2.583 |

Table 11. Results of the bispecific antibodies inhibiting the binding of ANG2 to Tie2

| Antibody | hu15.E-V | hu14.2B-V | Crossmab | NC |
|---|---|---|---|---|
| IC50(nM) | 0.192 | 0.2702 | 24.82 | ~ |

**Test Example 4. FACS-based experiment of the bispecific antibodies blocking the binding of ANG2 to Tie2**

In order to identify that the selected bispecific antibodies can block the Tie2 receptor on the cell surface, a CHOK1 recombinant cell line highly expressing Tie2 was constructed. This experiment identified that the bispecific antibodies can block the binding of ANG2 to the recombinant Tie2 on the surface of the CHOK1 cell line.

The specific methods were as follows:

During the cell experiment, PBS containing 2% FBS was used as the experiment buffer. After digesting and resuspending the stably transfected line CHO-Tie2#1B11 overexpressing Tie2, the cells were washed once with 2% FBS/PBS, resuspended, the density was adjusted to $20\times10^6$ cell/ml, and the cells were plated at 50 $\mu$l/well into a 96-well round bottom culture plate, i.e., at $1.0\times10^5$ cell/well. Bio-huANG2-Fc, the antibodies and the negative control were respectively diluted with 2% FBS/PBS and mixed well in equal volume. After incubating at 37°C for 15 min, 50 $\mu$l of the antigen-antibody mixture was added to the cell suspension. The final concentration of bio-huANG2-Fc in the mixed system was 0.20 $\mu$g/ml, the initial concentration of the tested antibody was 10 nM with 3-fold gradient dilution. The mixture was incubated at 4°C for 1 h. The cells were washed twice with 200 $\mu$l/well PBS. 1:1000 diluted PE-streptavidin (BD Pharmingen, Cat# 554061) was added at 100 $\mu$l/well and incubated at 4°C for 40 min. The cells were washed twice with 200 $\mu$l/well PBS. 2% FBS/PBS was added at 100 $\mu$l/well to resuspend the cells, which were then detected on a flow cytometer, and the data of 10,000 cells per well were analyzed. The IC50 value of the bispecific antibodies blocking the binding of ANG2 to Tie2 was calculated according to the fluorescence signal value. The results are as shown in Figure 3.

The results show that the antibodies hu15.E-V and hu14.2B-V show stronger ability to block the binding of ANG2 to Tie2 on the cell surface than the positive antibody, respectively.

**Test Example 5. The bispecific antibodies inhibiting ANG2-induced phosphorylation of Tie2**

ANG2 binds to the ANG2 receptor Tie2 present on the surface of vascular endothelial cells, triggering phosphorylation of tyrosine kinases in Tie2 cells, which then transduces signals to detach peripheral cells from vascular endothelial cells, resulting in blood vessels in an unstable and easy to proliferate state. This experiment was for identifying that the bispecific antibodies can inhibit ANG2-induced phosphorylation of Tie2.

The specific experimental methods were as follows:

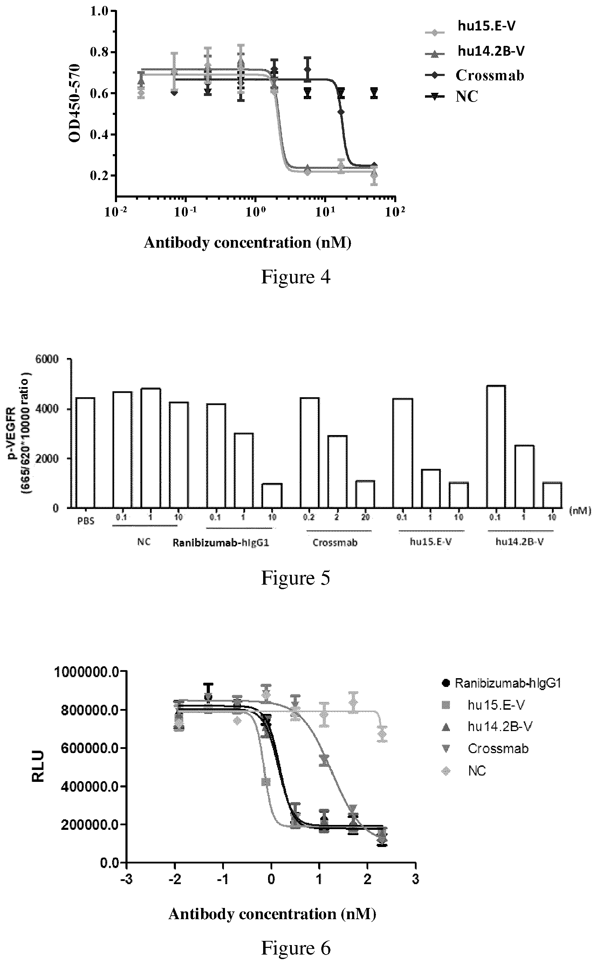

After digesting and resuspending the stably transfected line CHO-Tie2#1B11-1 overexpressing huTie2, the density was adjusted to $2.5\times10^5$ cell/ml with complete medium, and the cells were plated at 100 $\mu$l/well into a 96-well culture plate, i.e., at $2.5\times10^4$ cell/well. The medium was changed after 4-5 h (DME/F-12, HyClone, Cat#SH30023.01 + 0.1% BSA + 20 $\mu$g/ml puromycin, Gibco, Cat# A1113803) and the cells were starved overnight. The plate was coated with 4.0 $\mu$g/ml anti-huTie2 capture (R&D Systems, Cat# DYC2720E) at 100 $\mu$l/well at room temperature overnight. The coating solution was removed, and the plate was blocked with 1% BSA + 0.05% NaN$_3$ blocking solution at 250 $\mu$l/well at room temperature for 2 h. 25 $\mu$l of huAng2-Fc (final concentration 2.5 $\mu$g/ml) and 25 $\mu$l of the antibody to be tested with 3-fold serial dilution (maximum concentration 50.0 nM) were mixed in equal volumes and incubated at 37°C for 15 min. Then Na$_3$VO$_4$ (1.0 mM, Sigma, Cat# S6508) was added and mixed well. The cells were cultured overnight, 50 $\mu$l of the culture supernatant was discarded, 50 $\mu$l of the prepared antigen-antibody mixture was added and incubated at 37°C for 10 min. The cells were washed twice with 200 $\mu$l/well washing solution (PBS + 2.0 mM Na$_3$VO4), and 90 $\mu$l lysis buffer (($1\times$ lysis buffer + 10 $\mu$g/ml Leupeptin hemisulfate (Tocris, Cat# 1167) + 10.0 $\mu$g/ml APROTININ, Sigma, Cat# SRE0050)) was added to lyse the cells on ice for 10-15 min. The cell lysate was collected by centrifuging at 4000 g for 5 min, added into the blocked ELISA plate and incubated at room temperature for 2 h. The plate was washed for 5 times with PBST. 1:1000 diluted secondary antibody anti-PY-HRP (R&D Systems, Cat# DYC2720E) was added and incubated for 1-2 h at room temperature. The plate was washed for 5 times with PBST. Color development was done by TMB for 15-30 min, and stopped by 1 M $H_2SO_4$. The OD450 was read by using a Versa Max microplate reader and the IC50 was calculated. The results (see Figure 4) show that the antibodies hu15.E-V and hu14.2B-V show a very strong ability to inhibit ANG2-mediated phosphorylation of Tie2.

**Test Example 6. The bispecific antibodies inhibiting VEGF-induced phosphorylation of VEGFR**

VEGF binds to VEGFR present on vascular endothelial cells to phosphorylate the kinases in the VEGFR cell and promote the proliferation of the endothelial cells to form new blood vessels, thus promoting the growth and metastasis of tumor cells. This experiment was for identifying that the bispecific antibodies can inhibit VEGF-induced phosphorylation of VEGFR.

Specifically: after digestion of HUVEC cells (PromoCell/MT-Bio, C-12205), the cell density was adjusted to $1.5 \times 10^5$ cells per 500 $\mu$l with complete medium, and the cells were added into a 24-well plate at 500 $\mu$l per well. After culturing in a 37°C incubator overnight, the medium was discarded. The cells were washed once with 500 $\mu$l of ice-cold DPBS (Gibco, 14190-250), and 200 $\mu$l of minimal medium containing 0.1% BSA was added to each well for starvation culture for 30 min. The antibodies to be tested were diluted to 10 nM, 1 nM and 0.1 nM with minimal medium (20 nM, 2 nM and 0.2 nM for crossmab). VEGF (R&D system, Cat#293-VE) was diluted to 400 ng/ml with minimal medium. Diluted VEGF and antibody of equal volume were taken and mixed well, and 200 $\mu$l of mixture was added to the corresponding well of the culture plate and incubated at 37°C for 5 min. 4$\times$ Lysis Buffer #1 (cisbio, 63ADK041PEG) was diluted to 1$\times$ with dd $H_2O$. The blocking solution was diluted 100 times with 1$\times$ lysis buffer to prepare the lysis solution. The cell culture plate was taken out and the medium in it was discarded. 500 $\mu$l ice-cold PBS was added, shaken slightly and discarded. 50 $\mu$l of the prepared lysis buffer was immediately added, and the plate was placed on a shaker and incubated at room temperature for 30 min. The supernatant was collected by centrifuging at 2400 g for 10 min. Phospho-VEGFR2 (Tyr1175) kit (cisbio, 63ADK041PEG) was used to detect p-VEGFR in the supernatant. The detection method was as follows: 10 $\mu$l phospho-VEGFR2 (Tyr1175) d2 antibody was taken and added with 200 $\mu$l detection buffer to prepare a working solution. 10 $\mu$l phospho-VEGFR2 (Tyr1175) Cryptate antibody was taken and added with 200 $\mu$l detection buffer to prepare a working solution. The d2 antibody working solution and the Cryptate antibody working solution were mixed in equal volumes. 16 $\mu$l cell lysate, and 4 $\mu$l mixture of d2 antibody and Cryptate antibody were added into a HTRF 96-well microtiter plate, the plate was sealed with a sealing membrane, centrifuged for 1 min in a centrifuge, and incubated at room temperature for 4-24 h in the dark. The fluorescence values excited at 340 nm wavelength and emitted at 665 nm and 620 nm wavelengths were read by using a PHERAstar multi-function microplate reader. Data processing: ratio = signal 665 nm/signal 620 nm$\times$10000, a histogram was plotted by using Graphpad Prism 5. The results are as shown in Figure 5.

The results show that hu15.E-V and hu14.2B-V can significantly inhibit the increase of phosphorylated VEGFR level in HUVEC cells caused by VEGF.

**Test Example 7. The bispecific antibodies inhibiting VEGF-induced proliferation of HUVEC**

VEGF binds to the VEGFR present on HUVEC to phosphorylate the kinases in VEGFR cells and promote the proliferation of HUVEC. This experiment was used to identify that the bispecific antibodies can prevent VEGF-induced proliferation of HUVEC.

The specific methods were as follows:

HUVEC was seeded into T75 cell flasks at a density of $5 \times 10^4$ cell/ml, and the cells grew to the logarithmic phase in about 2-3 days. The HUVEC cells in the logarithmic growth phase were digested with 0.08% trypsin for about 1-2 min at room temperature, and the digestion was terminated by adding 10% FBS. The digested HUVEC was collected, centrifuged at 800 rpm/min for 5 min and washed for three times with PBS to remove the cytokines in the medium which would stimulate the proliferation of HUVEC (800 rpm/min, centrifuged for 5 min). The HUVEC cells were resuspended in 6% FBS medium. After cell counting, the cells were seeded in a white 96-well cell culture plate at 4000 cell/50 $\mu$l/well, and cultured in an incubator for 2 h. The VEGF was adjusted to an initial concentration of 300 ng/ml, and was added at 120 $\mu$l/well to a sterile 96-well plate. The antibodies to be tested were gradient diluted at 4-fold gradient dilution from the initial concentration of 600 nM, and then were added to the above 96-well plate in equal volume and incubated at room temperature for 30 min. After incubation, 100 $\mu$l/well of the antibody and antigen mixture was added to the adherent HUVEC cells and cultured in an incubator for 5 days. After the culture was finished, CellTiter-Glo® (G7573, PROMEGA) was added at 50 $\mu$l/well, incubated at room temperature for 10min in the dark, and detected by using the Luminescence program of a Cytation5 cell imager. The results are as shown in Figure 6. The results show that hu15.E-V and hu14.2B-V can significantly inhibit the proliferation of HUVEC caused by VEGF.

**Biological evaluation of *in vivo* activity**

**Test Example *8. In vivo* efficacy of the bispecific antibodies in nude mice transplanted with COLO205 tumor**

This experiment evaluated the efficacy of ANG2/VEGF bispecific antibodies and the control antibody Avastin after intraperitoneal injection in nude mice transplanted with human colon cancer cell COLO205.

Balb/c nude mice, SPF, 16-18 g, ♀, were purchased from Beijing Vital River Laboratory Animal Technology Co., Ltd. Colo205 cells (ATCC® CCL-222™) were inoculated into 100 Balb/c nude mice subcutaneously on the right ribs at $3×10^6$ cell/mouse/100 μl. When the tumor volume reached about 120 mm$^3$ in the tumor-bearing mice, the mice were randomly divided into 4 groups: i.e., the vehicle (PBS) group, Avastin 3 mpk group, hu15.E-V 3.5 mpk group and hu14.2B-V 3.5 mpk group, each with 8 animals. The day of grouping was defined as Day 0 of the experiment. Intraperitoneal injection of each antibody was started on the day of grouping, continued twice a week, for a total of 8 administrations. Tumor volume and animal weight were monitored twice a week and the data was recorded. When the tumor volume exceeded 1500 mm$^3$ or most tumors were ruptured or the body weight was reduced by 20%, the tumor-bearing animals were euthanized as the experimental endpoint. All data were plotted and statistically analyzed by using Excel and GraphPad Prism 5 software. Calculation formula of tumor volume (V) is: V=1/2×a×b$^2$, wherein a and b represent length and width respectively.

Relative tumor proliferation rate T/C (%)=(T-T0)/(C-C0)×100, wherein T and C represent the tumor volume of the treatment group and the PBS control group at the end of the experiment; T0 and C0 represent the tumor volume at the beginning of the experiment.

Tumor growth inhibition rate (TGI) (%) = 1- T/C (%).

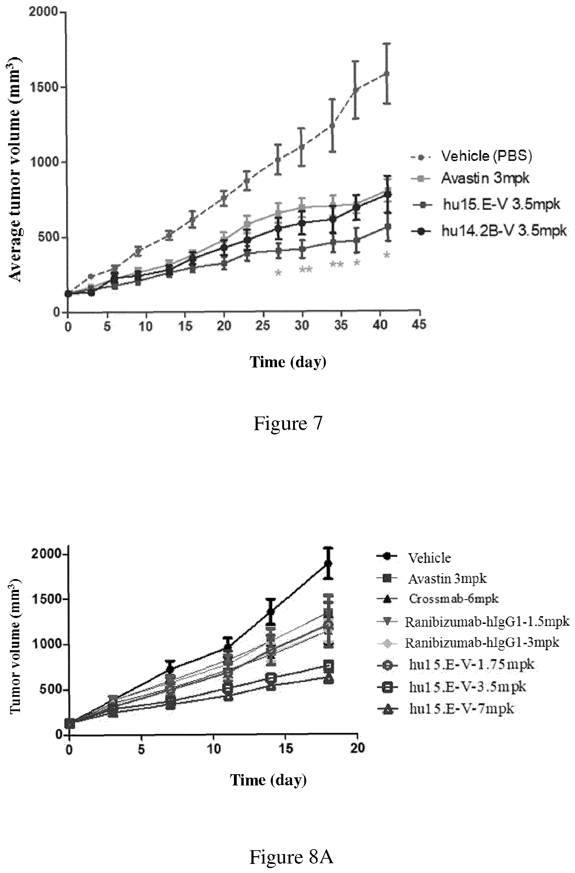

The results are as shown in Table 12 and Figure 7.

Table 12. Tumor growth inhibition rate (TGI%) of each group of antibodies

| Group | Day | | | | | |
|---|---|---|---|---|---|---|
| | 6 | 9 | 16 | 20 | 23 | 27 |
| Vehicle (PBS) | - | - | - | - | - | - |
| Avastin-3mpk | 41.00 | 50.52 | 48.19 | 44.30 | 38.90 | 40.27 |
| hu14.2B-V-3.5mpk | 36.75 | 58.41 | 52.91 | 52.28 | 52.82 | 51.90 |
| hu15.E- V-3.5mpk | 69.39 | 70.72 | 65.49 | 69.01 | 64.93 | 68.59 |

The experimental results of the candidate bispecific antibodies hu15.E-V and hu14.2B-V of the present disclosure show that: compared with the vehicle group, all antibody in this experiment, including VEGF monoclonal antibody Avastin and each tested antibody can inhibit the growth of the subcutaneous Colo205 transplanted tumors in Balb/c nude mice, and they all show very significant differences compared with the vehicle group on Day 27, when the administration was terminated (Table 12 and Figure 7). In terms of the tumor growth inhibition rate, hu15.E-V always exhibits a higher tumor growth inhibition rate than VEGF monoclonal antibody Avastin-hIgG1 during the entire administration process, and displays a statistical difference in tumor volume from the Avastin group after 6 administrations, until the end of administration (p=0.0081 at the end of administration).

**Test Example 9. The efficacy of the bispecific antibodies in BALB/c nude mice model subcutaneously transplanted with the highly metastatic non-small cell lung cancer H460-Luc cell line**

The effect of the ANG2/VEGF bispecific antibodies and the control antibody Avastin, ranibizumab-hIgG1 and crossmab after intraperitoneal injection on inhibiting the growth and metastasis of human non-small cell lung cancer H460 transplanted tumor was evaluated in this experiment.

BALB/c nude mice, female, 4-5 weeks, 18-20 g, were purchased from Shanghai Lingchang Bio-tech Co., Ltd. Human non-small cell lung cancer H460-Luc (stably transfected with luciferase gene) was cultured in RPMI 1640 medium supplemented with 10% FBS in a 37°C incubator containing 5% CO$_2$. The cells were cultured continuously for 5 generations and inoculated subcutaneously in mice. The mice were anesthetized with 3-4% isoflurane before inoculation. About $1×10^6$ H460 cells were resuspended in a suspension with serum-free medium and Matrigel (medium: Matrigel=50%:50%), and inoculated into 100 mice by subcutaneous injection at an inoculation volume of 200 μL. When the tumors grew to an average of about 100-150 mm$^3$, 72 mice with appropriate tumor sizes were randomly divided into 8 groups according to the tumor size and body weight, with 8 mice in each group. The grouping and administration date was defined as Day 0. The grouping and administration regimen are as shown in Table 13.

Table 13. Grouping, administration regimen and tumor growth inhibition rate

| Group | Substance tested | Dose (mg/kg) | Administration regimen | TGI (%) |
|---|---|---|---|---|
| 1 | PBS | 3 | i.p. BIW*3 weeks | 3 |
| 2 | Avastin | 3 | i.p. BIW*3 weeks | 31 |
| 3 | Ranibizumab-hIgG1 | 1.5 | i.p. BIW*3 weeks | 31 |
| 4 | Ranibizumab-hIgG1 | 3 | i.p. BIW*3 weeks | 43 |
| 5 | Crossmab | 6 | i.p. BIW*3 weeks | 43 |
| 6 | hu15.E-V | 1.75 | i.p. BIW*3 weeks | 39 |
| 7 | hu15.E-V | 3.5 | i.p. BIW*3 weeks | 64 |
| 8 | hu15.E-V | 7 | i.p. BIW*3 weeks | 72 |

After grouping, the tumor volume was measured twice a week for 3 consecutive weeks. The calculation method of the tumor volume (V) is as follows:

$$V = (length \times width^2)/2.$$

The calculation method of the relative tumor volume (RTV) of each mouse is:

RTV = Vt/V0, wherein Vt is the volume measured daily and V0 is the volume at the beginning of treatment.

At the end of the experiment, all tumor-bearing animals were photographed, all tumors were taken out, weighed and photographed.

Statistical Analysis

The results were presented as mean $\pm$ S.E.M. The comparison between the two groups was tested by Dunnett's multiple comparison test. The difference was considered statistically significant if $p < 0.05$.

Detection method for liver and lung metastasis:

As the H460 cell line contained luciferase label, after the mice were euthanized, the livers of each group of mice were dissected, the liver metastatic lesions were collected by fluorescence imaging, and the degree of metastasis was calculated according to the intensity of the fluorescence signal.

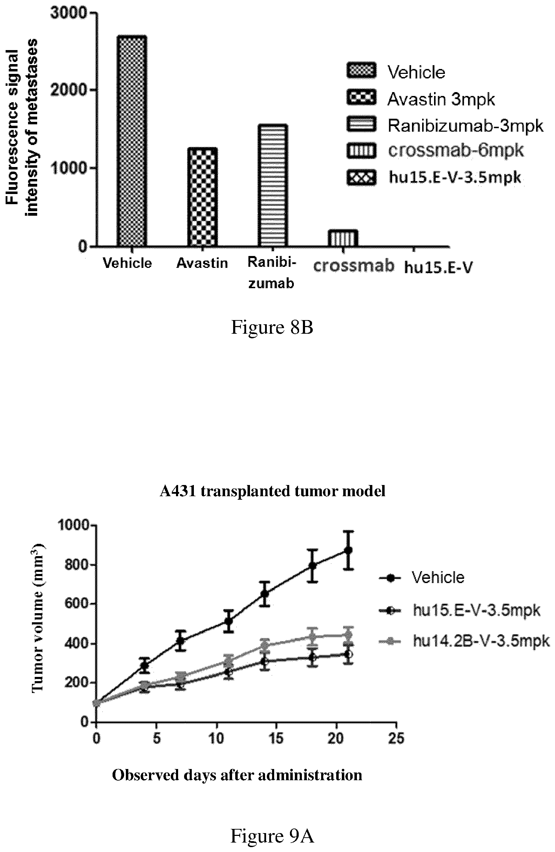

The test results are as shown in Table 13 and Figure 8A and Figure 8B.

Figure 8A shows that the candidate molecule hu15.E-V of the present disclosure can significantly inhibit the growth of H460 tumors. Also, hu15.E-V shows a dose-dependent effect. The 3.5 mpk hu15.E-V has stronger effect of inhibiting tumor growth compared with the same molar of 3 mpk Avastin and ranibizumab-hIgG1, and there are statistical differences in the tumor growth inhibition rate. Figure 8B shows that more severe liver and lung metastasis are seen in both Avastin and ranibizumab-hIgG1 with the same molar, while the bispecific antibody hu15.E-V can significantly inhibit liver and lung metastasis of tumors, with no metastases detected in any mice of the group.

**Test Example 10. The efficacy of the bispecific antibodies on mice model subcutaneously transplanted with human skin cancer and prostate cancer cell**

A431 cells at $2 \times 10^6$ cell/mouse/100 $\mu$l or PC-3 cells at $5 \times 10^6$ were inoculated subcutaneously on the right ribs of Balb/c nude mice. When the tumor volume reached about 100 mm$^3$ in the tumor-bearing mice, the mice were randomly divided into 3 groups respectively: vehicle (PBS), hu15.E-V 3.5 mpk and hu14.2B-V 3.5 mpk, with 8 animals in each group. The day of grouping was defined as Day 0. Each antibody was started to be injected intraperitoneally on the day of grouping, twice a week, for a total of 6 administrations. Tumor volume and animal weight were monitored twice a week and the data was recorded. When the tumor volume exceeded 1000mm$^3$ or most tumors were ruptured or the body weight was reduced by 20%, the tumor-bearing animals were euthanized as the experimental endpoint. All data were plotted and statistically analyzed by using Excel and GraphPad Prism 5 software. Calculation formula of tumor volume (V) is: $V = 1/2 \times a \times b^2$, wherein a and b represent length and width respectively.

Relative tumor proliferation rate T/C (%)=(T-T0)/(C-C0)×100, wherein T and C represent the tumor volume of the treatment group and the control group at the end of the experiment; T0 and C0 represent the tumor volume at the beginning of the experiment.

$$\text{Tumor growth inhibition rate (TGI) (\%)} = 1\text{- T/C (\%)}.$$

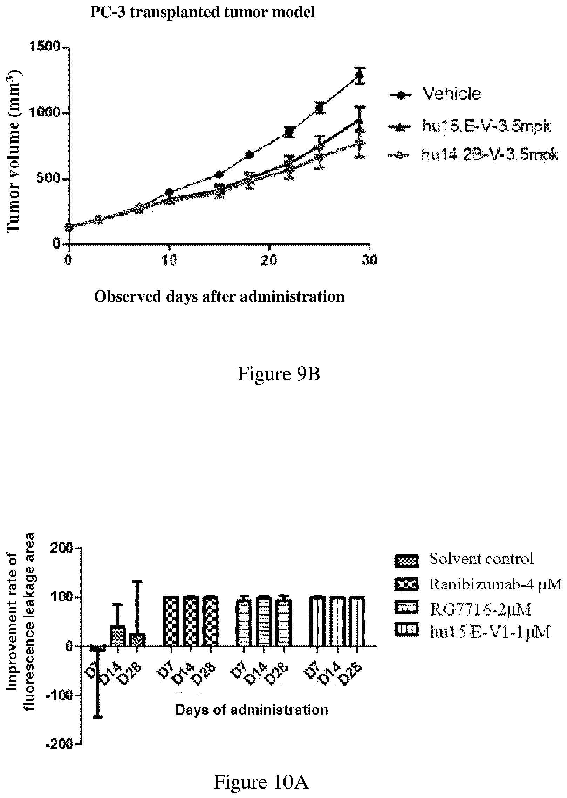

The grouping and administration regimen are as shown in Table 14. The tumor growth curve is as shown in Figure 9A and Figure 9B.

Table 14. Grouping, administration regimen and tumor growth inhibition rate

| Group | Substance tested | Dose (mg/kg) | Administration regimen | TGI (%)-A431 | TGI (%)-PC-3 |
|---|---|---|---|---|---|
| 1 | PBS | 3 | i.p. BIW*3 weeks | 0 | 0 |
| 2 | hu15.E-V | 3.5 | i.p. BIW*3 weeks | 68 | 46 |
| 3 | hu14.2B-V | 3.5 | i.p. BIW*3 weeks | 51 | 41 |

The results are shown in Table 14 and Figure 9A and Figure 9B, which show that both bispecific antibodies hu15.E-V and hu14.2B-V in the present disclosure can significantly inhibit the growth of A431 tumors and PC-3 tumors.

**Test Example 11. Test of the inhibitory function of the bispecific antibodies on laser-induced choroidal neovascularization in rhesus monkeys**

Through the effect of ocular intravitreal injection administration on the laser-induced choroidal neovascularization leakage and growth in rhesus monkeys, this test example was to verify that the bispecific antibodies in this application can be used for the treatment of diseases such as age-related macular degeneration (AMD) by intravitreal injection. The specific methods were as follows:

An animal model similar to human choroidal neovascularization was established by laser photocoagulation around the macula fovea of the fundus of rhesus monkeys, which induced choroidal neovascularization in the fundus. Fluorescein fundus angiography was performed before photocoagulation and 20 days after photocoagulation to determine the status of modeling. The successful models of 16 rhesus monkeys (Sichuan Greenhouse Biotech Co., Ltd., production license number: SCXK (Sichuan) 2014-013, laboratory animal quality certificate number: No: 0022202) were selected and randomly divided into solvent control group, ranibizumab (96 μg, 4 μM) group, RG7716 (292 μg, 2 μM) group and hu15.E-V1 (170 μg, 1 μM) group, a total of 4 groups with 4 monkeys in each group.

21 days after photocoagulation, the ranibizumab group, RG7716 292 group and hu15.E-V1 group, based on 96 μg, 292 μg and 170 μg/eye respectively, were given 50 μL of ranibizumab at a concentration of 1.92 mg/mL, RG7716 at 5.84 mg/mL and hu15.E-V1 at 3.4 mg/mL by intravitreal injection into both eyes. The solvent control group was given an equal volume of solvent. For animals in each group, the inhibition of choroidal neovascularization by the antibodies was observed by intraocular pressure examination, fundus color photography, fluorescein fundus angiography and optical coherence tomography (OCT) on Day 7, 14, and 28 after administration. On Day 28 after administration, 100-200 μL of aqueous humor was collected, 100 μL of which was used for the determination of VEGF in aqueous humor. After euthanasia on Day 29 after administration, 3 eyeballs from each group were collected for HE staining histological examination. The results are as follows:

**AMD Modeling**

20 days after laser modeling, the 16 monkeys included in the experiment show 9 laser spots around the macula in both eyes by color photography of the fundus. It can be seen that laser spots with high fluorescence are present around the macula in the fundus in all animals, with obvious fluorescein leakage that exceeds the edge of the fluorescent spot. 20 days after laser modeling (before administration), the number of level 4 fluorescent spots in the vehicle control group, ranibizumab group, RG7716 group and hu15.E-V1 group are 46, 42, 40 and 40, respectively. The above changes are similar to clinical choroidal neovascularization (CNV) changes, suggesting modeling is successful.

**Fluorography examination**

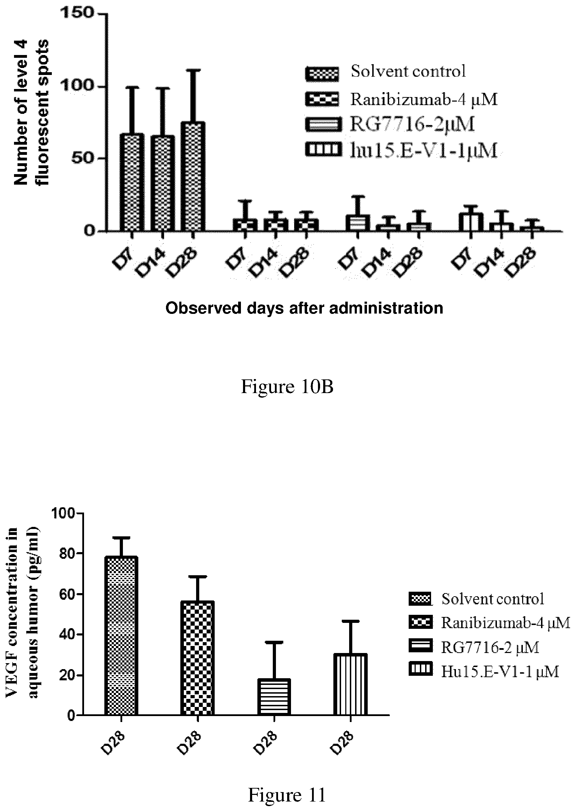

The area of the fluorescent spots in ranibizumab group, RG7716 group and hu15.E-V1 group decreased to a certain extent on Day 7, 14 and 28 after the administration. The improvement rate of the fluorescence leakage area and the decreased amount of the fluorescein leakage area in each group are all superior to that of the solvent control group, and the number of level 4 fluorescent spots in each group is significantly lower than that of the solvent control group. In the hu15.E-V1 group with 1 $\mu$M of dose, the ranibizumab group with 4 $\mu$M of dose and the RG7716 group with 2 $\mu$M of dose, the improvement rates of the fluorescence leakage area are equivalent at each time point. The results are shown in Figure 10A and Figure 10B.

**Aqueous humor VEGF**

The VEGF expression in aqueous humor of the ranibizumab group, RG7716 292 group and hu15.E-V1 170 group is significantly lower than that of the solvent control group on Day 28 after administration. The VEGF expression in aqueous humor of both RG7716 group and hu15.E-V1 group is significantly lower than that of ranibizumab group. The results are shown in Figure 11.

In summary, hu15.E-V1 at a dose of 170$\mu$g/eye has a significant inhibitory effect on CNV in monkeys under the conditions of this experiment, i.e., the laser CNV rhesus monkey models administered with hu15.E-V1 at dose of 170 $\mu$g/eye via single intravitreal injection into both eyes, and examined by the retinal fluorescent angiography, optical coherence tomography and aqueous humor VEGF and ocular histopathological examination.

**Claims**

1. A bispecific antibody that specifically binds to VEGF and ANG2, comprising an anti-VEGF antibody or antigen-binding fragment thereof that specifically binds to VEGF, and an anti-ANG2 single domain antibody that specifically binds to ANG2, wherein the anti-ANG2 single domain antibody is covalently connected to the anti-VEGF antibody or antigen-binding fragment thereof directly through a peptide bond or indirectly through a linker.

2. The bispecific antibody that specifically binds to VEGF and ANG2 according to claim 1, wherein the anti-ANG2 single domain antibody is connected to the carboxyl terminus of the heavy chain of the anti-VEGF antibody or antigen-binding fragment thereof directly through a peptide bond or indirectly through a linker, preferably, the linker is $(G)_n$, wherein n>=1.

3. The bispecific antibody that specifically binds to VEGF and ANG2 according to claim 1 or 2, wherein the anti-ANG2 single domain antibody comprises CDR1 as shown in SEQ ID NO: 5 or having at most 3 amino acid substitution mutations compared to the same, CDR2 as shown in SEQ ID NO: 6 or having at most 6 amino acid substitution mutations compared to the same, and CDR3 region as shown in SEQ ID NO: 7 or having at most 3 amino acid substitution mutations compared to the same.

4. The bispecific antibody that specifically binds to VEGF and ANG2 according to any one of claims 1 to 3, wherein the anti-ANG2 single domain antibody comprises CDR1 as shown in SEQ ID NO: 14, CDR2 as shown in SEQ ID NO: 15, and CDR3 as shown in SEQ ID NO: 7.

5. The bispecific antibody that specifically binds to VEGF and ANG2 according to any one of claims 1 to 4, wherein the anti-ANG2 single domain antibody comprises CDR1, CDR2 and CDR3 as shown in any one of the following:

   i) CDR1 as shown in SEQ ID NO: 5, CDR2 as shown in SEQ ID NO: 6, and CDR3 as shown in SEQ ID NO: 7;
   ii) CDR1 as shown in SEQ ID NO: 8, CDR2 as shown in SEQ ID NO: 9, and CDR3 as shown in SEQ ID NO: 7;
   iii) CDR1 as shown in SEQ ID NO: 5, CDR2 as shown in SEQ ID NO: 10, and CDR3 as shown in SEQ ID NO: 7; or
   iv) CDR1 as shown in SEQ ID NO: 5, CDR2 as shown in SEQ ID NO: 11, and CDR3 as shown in SEQ ID NO: 7.

6. The bispecific antibody that specifically binds to VEGF and ANG2 according to any one of claims 1 to 5, wherein the anti-ANG2 single domain antibody is a llama antibody or a humanized antibody.

7. The bispecific antibody that specifically binds to VEGF and ANG2 according to claim 6, wherein the humanized antibody comprises a heavy chain framework region derived from a human antibody or a framework region variant thereof, wherein the framework region variant has at most 10 amino acid back mutations in the heavy chain framework region of the human antibody;
   preferably, the back mutation(s) is one or more selected from the group consisting of 5Q, 30N, 83K, 84P, 93N and 94A.

8. The bispecific antibody that specifically binds to VEGF and ANG2 according to claim 6, wherein the anti-ANG2 single domain antibody comprises a sequence as shown in any one of the following:

   v) the sequence as shown in SEQ ID NO: 16, preferably, the sequence as shown in SEQ ID NO: 3, SEQ ID NO: 4, SEQ ID NO: 12, or SEQ ID NO: 13, or
   vi) the sequence as shown in SEQ ID NO: 27, preferably, the sequence as shown in SEQ ID NO: 17, 18, 19, 20, 21, 22, 23, 24, 25 or 26.

9. The bispecific antibody that specifically binds to VEGF and ANG2 according to any one of claims 1 to 8, wherein the anti-VEGF antibody or antigen binding fragment thereof comprises:
   HCDR1, HCDR2 and HCDR3 as shown in SEQ ID NO: 32, SEQ ID NO: 33 and SEQ ID NO: 34, respectively; and
   LCDR1, LCDR2 and LCDR3 as shown in SEQ ID NO: 35, SEQ ID NO: 36 and SEQ ID NO: 37, respectively.

10. The bispecific antibody that specifically binds to VEGF and ANG2 according to claim 9, wherein the anti-VEGF antibody or antigen binding fragment thereof comprises a light chain variable region as shown in SEQ ID NO: 28, and a heavy chain variable region as shown in SEQ ID NO: 30.

11. The bispecific antibody that specifically binds to VEGF and ANG2 according to claim 10, wherein the anti-VEGF antibody or antigen binding fragment thereof comprises a light chain as shown in SEQ ID NO: 29 and a heavy chain as shown in SEQ ID NO: 31 or 54.

12. The bispecific antibody that specifically binds to VEGF and ANG2 according to any one of claims 1 to 11, comprising a first polypeptide chain selected from any one of the group consisting of SEQ ID NO: 38, 39, 40, 41, 42, 43, 44, 45, 46, 47, 55 or 56, and/or a second polypeptide chain as shown in SEQ ID NO:29.

13. An anti-ANG2 single domain antibody, comprising CDR1 as shown in SEQ ID NO: 5 or having at most 3 amino acid substitution mutations compared to the same, CDR2 as shown in SEQ ID NO: 6 or having at most 6 amino acid substitution mutations compared to the same, and CDR3 region as shown in SEQ ID NO: 7 or having at most 3 amino acid substitution mutations compared to the same.

14. The anti-ANG2 single domain antibody according to claim 13, wherein the anti-ANG2 single domain antibody comprises CDR1 as shown in SEQ ID NO: 14, CDR2 as shown in SEQ ID NO: 15, and CDR3 as shown in SEQ ID NO: 7.

15. The anti-ANG2 single domain antibody according to claim 14, wherein the anti-ANG2 single domain antibody comprises CDR1, CDR2 and CDR3 as shown below:

    i) CDR1 as shown in SEQ ID NO: 5, CDR2 as shown in SEQ ID NO: 6, and CDR3 as shown in SEQ ID NO: 7;
    ii) CDR1 as shown in SEQ ID NO: 8, CDR2 as shown in SEQ ID NO: 9, and CDR3 as shown in SEQ ID NO: 7;
    iii) CDR1 as shown in SEQ ID NO: 5, CDR2 as shown in SEQ ID NO: 10, and CDR3 as shown in SEQ ID NO: 7; or
    iv) CDR1 as shown in SEQ ID NO: 5, CDR2 as shown in SEQ ID NO: 11, and CDR3 as shown in SEQ ID NO: 7.

16. The anti-ANG2 single domain antibody according to claim 15, which is selected from llama antibody and humanized antibody.

17. The anti-ANG2 single domain antibody according to claim 16, wherein the humanized antibody comprises a heavy chain framework region derived from a human antibody or a framework region variant thereof, wherein the framework region variant has at most 10 amino acid back mutations in the heavy chain framework region of the human antibody; preferably, the back mutation(s) is one or more selected from the group consisting of 5Q, 30N, 83K, 84P, 93N and 94A.

18. The anti-ANG2 single domain antibody according to claim 16, wherein the anti-ANG2 single domain antibody comprises a sequence as shown below:

    v) the sequence as shown in SEQ ID NO: 16, preferably, comprising the sequence as shown in SEQ ID NO: 3, SEQ ID NO: 4, SEQ ID NO: 12, or SEQ ID NO: 13, or
    vi) the sequence as shown in SEQ ID NO: 27, preferably, comprising the sequence as shown in SEQ ID NO: 17, 18, 19, 20, 21, 22, 23, 24, 25 or 26.

19. An anti-ANG2 single domain antibody, which competitively binds to human ANG2 with the anti-ANG2 single domain antibody or antigen-binding fragment thereof according to any one of claims 13 to 18.

20. An anti-ANG2 single domain antibody, wherein the single domain antibody comprises the same CDR1, CDR2 and CDR3 region sequence as the single domain antibody shown in SEQ ID NO: 3, SEQ ID NO: 4, SEQ ID NO: 12 or SEQ ID NO: 13.

21. A monoclonal antibody comprising the anti-ANG2 single domain antibody according to any one of claims 13 to 20.

22. A bispecific antibody comprising the anti-ANG2 single domain antibody according to any one of claims 13 to 20.

23. A pharmaceutical composition comprising a therapeutically effective amount of the bispecific antibody that specifically binds to VEGF and ANG2 according to any one of claims 1 to 12, or the anti-ANG2 single domain antibody according to any one of claims 13 to 20, or the monoclonal antibody according to claim 21, or the bispecific antibody according to claim 22, as well as one or more pharmaceutically acceptable carriers, diluents, buffers or excipients.

24. A nucleic acid molecule encoding the bispecific antibody that specifically binds to VEGF and ANG2 according to any one of claims 1 to 12, or the anti-ANG2 single domain antibody according to any one of claims 13 to 20, or the monoclonal antibody according to claim 21, or the bispecific antibody according to claim 22.

25. A host cell transformed with the nucleic acid molecule according to claim 24, wherein the host cell is selected from the group consisting of prokaryotic cell and eukaryotic cell.

26. A method for the detection or determination of human ANG2 *in vitro,* including using the bispecific antibody that specifically binds to VEGF and ANG2 according to any one of claims 1 to 12, or the anti-ANG2 single domain antibody according to any one of claims 13 to 20, or the monoclonal antibody according to claim 21, or the bispecific antibody according to claim 22.

27. A kit comprising the bispecific antibody that specifically binds to VEGF and ANG2 according to any one of claims 1 to 12, or the anti-ANG2 single domain antibody according to any one of claims 13 to 20, or the monoclonal antibody according to claim 21, or the bispecific antibody according to claim 22.

28. A method for the treatment of a disease related with VEGF-mediated and/or ANG2-mediated angiogenesis, including administering to a subject a therapeutically effective amount of the bispecific antibody that specifically binds to VEGF and ANG2 according to any one of claims 1 to 12, or the anti-ANG2 single domain antibody according to any one of claims 13 to 20, or the monoclonal antibody according to claim 21, or the bispecific antibody according to claim 22, or the pharmaceutical composition according to claim 23.

29. A method for the treatment of cancer or angiogenic eye disease, including administering to a subject a therapeutically effective amount of the bispecific antibody that specifically binds to VEGF and ANG2 according to any one of claims 1 to 12, or the anti-ANG2 single domain antibody according to any one of claims 13 to 20, or the monoclonal antibody according to claim 21, or the bispecific antibody according to claim 22, or the pharmaceutical composition according to claim 23; preferably, wherein the cancer is selected from the group consisting of breast cancer, adrenal tumor, fallopian tube cancer, squamous cell carcinoma, ovarian cancer, gastric cancer, colorectal cancer, non-small cell lung cancer, cholangiocarcinoma, bladder cancer, pancreatic cancer, skin cancer and liver cancer; the angiogenic eye disease is selected from the group consisting of neovascular glaucoma, age-related macular degeneration (AMD), diabetic macular edema, corneal neovascularization, corneal graft neovascularization, corneal graft rejection, retinal/choroidal neovascularization, angulus iridocornealis neovascularization (rubeosis), ocular neovascular disease, vascular restenosis and arteriovenous malformations (AVM).

Figure 1

Figure 2A

Figure 2B

Figure 3

Figure 4

Figure 5

Figure 6

Figure 7

Figure 8A

Figure 8B

**A431 transplanted tumor model**

Observed days after administration

Figure 9A

**PC-3 transplanted tumor model**

Figure 9B

Figure 10A

Figure 10B

Figure 11

## INTERNATIONAL SEARCH REPORT

| International application No. |
|---|
| **PCT/CN2020/079690** |

### A.    CLASSIFICATION OF SUBJECT MATTER

C07K 16/46(2006.01)i;  C07K 16/22(2006.01)i;  C07K 16/28(2006.01)i;  C12N 15/13(2006.01)i;  C12N 5/10(2006.01)i;  A61K 39/395(2006.01)i;  A61P 35/00(2006.01)i;  A61P 27/02(2006.01)i

According to International Patent Classification (IPC) or to both national classification and IPC

### B.    FIELDS SEARCHED

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, CNTXT, CNKI and keywords: VEGF, 血管内皮生长因子, ANG2, 血管生成素, 单域抗体, VHH, 双特异性, etc.; DWPI, SIPOABS, WOTXT, EPTXT, USTXT, ISI_Web of Scienceand keywords: VEGF, vascular endothelial growth factor, ANG2, angiopoietin-2, single domain antibody, VHH, bispecific, etc.; Genbank; EMBL; 中国专利生物序列检索系统和检索的序列: SEQ ID NO: 5-16, China Patent Biological Sequence Search System and searched sequences: SEQ ID NO: 5-16

### C.    DOCUMENTS CONSIDERED TO BE RELEVANT

| Category* | Citation of document, with indication, where appropriate, of the relevant passages | Relevant to claim No. |
|---|---|---|
| X | CN 107849126 A (ALLERGAN, INC.) 27 March 2018 (2018-03-27) <br> claims 1, 19 and 22, SEQ ID NO: 9, and description, paragraphs 0051-0053 | 1, 2, 6, 7, 9-11, 23-27 |
| A | CN 107849126 A (ALLERGAN, INC.) 27 March 2018 (2018-03-27) <br> claims 1, 19 and 22, SEQ ID NO: 9, and description, paragraphs 0051-0053 | 3-5, 8, 12, 13-18, 20-22 |
| Y | CN 109476762 A (NANJING LEGEND BIOTECH CO., LTD.) 15 March 2019 (2019-03-15) <br> claims 1, 10, 37 and 38 | 1, 2, 6, 7, 9-11, 23-27 |
| Y | CN 102250247 A (CHANGZHOU ADAM BIOTECH INC.) 23 November 2011 (2011-11-23) <br> abstract, and claim 1 | 1, 2, 6, 7, 9-11, 23-27 |
| Y | CN 104428315 A (ROCHE GLYCART AG) 18 March 2015 (2015-03-18) <br> claim 2 | 1, 2, 6, 7, 9-11, 23-27 |
| Y | CN 104321344 A (BOEHRINGER INGELHEIM INTERNATIONAL GMBH) 28 January 2015 (2015-01-28) <br> claims 1-7 | 1, 2, 6, 7, 9-11, 23-27 |

☑ Further documents are listed in the continuation of Box C.        ☑ See patent family annex.

| | | |
|---|---|---|
| * | Special categories of cited documents: | "T"  later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
|---|---|
| **05 June 2020** | **23 June 2020** |

| Name and mailing address of the ISA/CN | Authorized officer |
|---|---|
| **China National Intellectual Property Administration** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2020/079690** |

### C. DOCUMENTS CONSIDERED TO BE RELEVANT

| Category* | Citation of document, with indication, where appropriate, of the relevant passages | Relevant to claim No. |
|---|---|---|
| Y | CN 102753577 A (HOFFMANN-LA ROCHE INC.) 24 October 2012 (2012-10-24)<br>the claims | 1, 2, 6, 7, 9-11, 23-27 |
| Y | CN 102906114 A (HOFFMANN-LA ROCHE INC.) 30 January 2013 (2013-01-30)<br>the claims | 1, 2, 6, 7, 9-11, 23-27 |
| Y | WO 2018037000 A1 (MEDIMMUNE LTD.) 01 March 2018 (2018-03-01)<br>the claims | 1, 2, 6, 7, 9-11, 23-27 |

Form PCT/ISA/210 (second sheet) (January 2015)

**INTERNATIONAL SEARCH REPORT**

International application No.

**PCT/CN2020/079690**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **28-29**
   because they relate to subject matter not required to be searched by this Authority, namely:

   [1]  PCT Rule 39.1(iv) - a method for the treatment of a human or animal body by surgry or therapy.

2. ☑ Claims Nos.: **19**
   because they relate to parts of the international application that do not comply with the prescribed requirements to such an extent that no meaningful international search can be carried out, specifically:

   [1]  Claim 19 attempts to define the anti-ANG2 single domain antibody by using the achieved effect (i.e. competitive binding activity with a reference antibody), without any limitation on the structural features necessary to achieve the effect. Claim 19 fails to clearly define the claimed invention and does not comply with PCT Article 6. The degree of such non-compliance renders a meaningful international search impossible.

3. ☐ Claims Nos.:
   because they are dependent claims and are not drafted in accordance with the second and third sentences of Rule 6.4(a).

Form PCT/ISA/210 (continuation of first sheet) (January 2015)

### INTERNATIONAL SEARCH REPORT
#### Information on patent family members

International application No.

**PCT/CN2020/079690**

| Patent document cited in search report | | | Publication date (day/month/year) | Patent family member(s) | | | Publication date (day/month/year) |
|---|---|---|---|---|---|---|---|
| CN | 107849126 | A | 27 March 2018 | AU | 2016298398 | A1 | 08 February 2018 |
| | | | | WO | 2017020001 | A2 | 02 February 2017 |
| | | | | RU | 2018103960 | A3 | 16 January 2020 |
| | | | | US | 2017107279 | A1 | 20 April 2017 |
| | | | | CA | 2992660 | A1 | 02 February 2017 |
| | | | | JP | 2018527327 | A | 20 September 2018 |
| | | | | BR | 112018001762 | A2 | 18 September 2018 |
| | | | | US | 2019248882 | A1 | 15 August 2019 |
| | | | | US | 10266589 | B2 | 23 April 2019 |
| | | | | RU | 2018103960 | A | 28 August 2019 |
| | | | | EP | 3328886 | A2 | 06 June 2018 |
| | | | | WO | 2017020001 | A3 | 16 March 2017 |
| CN | 109476762 | A | 15 March 2019 | JP | 2019524693 | A | 05 September 2019 |
| | | | | MX | 2019000730 | A | 20 June 2019 |
| | | | | KR | 20190040483 | A | 18 April 2019 |
| | | | | WO | 2018014260 | A1 | 25 January 2018 |
| | | | | US | 2019202935 | A1 | 04 July 2019 |
| | | | | IL | 264211 | D0 | 28 February 2019 |
| | | | | BR | 112019000970 | A2 | 30 April 2019 |
| | | | | AU | 2017300349 | A1 | 14 February 2019 |
| | | | | CA | 3030933 | A1 | 25 January 2018 |
| | | | | SG | 11201811640S | A | 27 February 2019 |
| | | | | EP | 3487887 | A1 | 29 May 2019 |
| | | | | EA | 201990331 | A1 | 28 June 2019 |
| | | | | WO | 2018014855 | A1 | 25 January 2018 |
| | | | | EP | 3487887 | A4 | 25 March 2020 |
| CN | 102250247 | A | 23 November 2011 | CN | 102250247 | B | 19 June 2013 |
| CN | 104428315 | A | 18 March 2015 | HR | P20181595 | T1 | 14 December 2018 |
| | | | | SI | 2872534 | T1 | 30 November 2018 |
| | | | | TW | I506036 | B | 01 November 2015 |
| | | | | AR | 092027 | A1 | 18 March 2015 |
| | | | | MX | 2014015518 | A | 23 June 2015 |
| | | | | SG | 11201408538P | A | 27 February 2015 |
| | | | | EP | 3495387 | A1 | 12 June 2019 |
| | | | | CL | 2015000061 | A1 | 08 May 2015 |
| | | | | CR | 20140542 | A | 12 February 2015 |
| | | | | AU | 2013288641 | A1 | 11 December 2014 |
| | | | | DK | 2872534 | T3 | 22 October 2018 |
| | | | | PH | 12014502713 | B1 | 02 February 2015 |
| | | | | EP | 2872534 | B1 | 08 August 2018 |
| | | | | LT | 2872534 | T | 25 October 2018 |
| | | | | JP | 2019062905 | A | 25 April 2019 |
| | | | | PL | 2872534 | T3 | 31 December 2018 |
| | | | | CA | 2874554 | C | 03 December 2019 |
| | | | | HK | 1206365 | A1 | 08 January 2016 |
| | | | | JP | 2015527064 | A | 17 September 2015 |
| | | | | US | 2014017244 | A1 | 16 January 2014 |
| | | | | MA | 37794 | A1 | 31 March 2016 |
| | | | | EA | 032192 | B1 | 30 April 2019 |
| | | | | TW | 201406782 | A | 16 February 2014 |

Form PCT/ISA/210 (patent family annex) (January 2015)

# EP 3 943 511 A1

<table>
<tr><td colspan="2" style="text-align:center"><b>INTERNATIONAL SEARCH REPORT</b><br><b>Information on patent family members</b></td><td colspan="2">International application No.<br><br><b>PCT/CN2020/079690</b></td></tr>
</table>

| Patent document cited in search report | | | Publication date (day/month/year) | Patent family member(s) | | Publication date (day/month/year) |
|---|---|---|---|---|---|---|
| | | | | JP 6462750 B2 | | 30 January 2019 |
| | | | | EA 201500132 A1 | | 31 August 2015 |
| | | | | JP 6154900 B2 | | 28 June 2017 |
| | | | | IN 11157DEN2014 A | | 02 October 2015 |
| | | | | PT 2872534 T | | 26 October 2018 |
| | | | | CA 2874554 A1 | | 16 January 2014 |
| | | | | CN 104428315 B | | 29 September 2017 |
| | | | | US 2017260265 A1 | | 14 September 2017 |
| | | | | AU 2013288641 B2 | | 06 July 2017 |
| | | | | PH 12014502713 A1 | | 02 February 2015 |
| | | | | IL 236292 A | | 26 February 2015 |
| | | | | KR 20150023688 A | | 05 March 2015 |
| | | | | RS 57704 B1 | | 31 December 2018 |
| | | | | PE 20150361 A1 | | 14 March 2015 |
| | | | | US 9695233 B2 | | 04 July 2017 |
| | | | | PE 03612015 A1 | | 14 March 2015 |
| | | | | CO 7151542 A2 | | 29 December 2014 |
| | | | | ES 2690312 T3 | | 20 November 2018 |
| | | | | MX 361337 B | | 04 December 2018 |
| | | | | KR 101774121 B1 | | 01 September 2017 |
| | | | | ZA 201408969 B | | 24 April 2019 |
| | | | | CL 2017002146 A1 | | 13 April 2018 |
| | | | | EP 2872534 A1 | | 20 May 2015 |
| | | | | JP 6640319 B2 | | 05 February 2020 |
| | | | | NZ 702201 A | | 26 January 2018 |
| | | | | MA 37794 B1 | | 31 July 2017 |
| CN 104321344 A | | | 28 January 2015 | HR P20190817 T1 | | 28 June 2019 |
| | | | | US 2019135907 A1 | | 09 May 2019 |
| | | | | PH 12014502179 A1 | | 10 December 2014 |
| | | | | HU E044263 T2 | | 28 October 2019 |
| | | | | MX 350248 B | | 31 August 2017 |
| | | | | US 2017107281 A1 | | 20 April 2017 |
| | | | | IN 6904DEN2014 A | | 15 May 2015 |
| | | | | JP 5970734 B2 | | 17 August 2016 |
| | | | | PL 2831111 T3 | | 30 September 2019 |
| | | | | WO 2013144266 A1 | | 03 October 2013 |
| | | | | BR 112014023415 A2 | | 11 July 2017 |
| | | | | EP 2831111 B1 | | 20 March 2019 |
| | | | | ES 2729165 T3 | | 30 October 2019 |
| | | | | CN 104321344 B | | 21 November 2017 |
| | | | | AU 2013241769 A1 | | 28 August 2014 |
| | | | | JP 2015516805 A | | 18 June 2015 |
| | | | | EA 201401065 A1 | | 31 March 2015 |
| | | | | CA 2865464 A1 | | 03 October 2013 |
| | | | | CL 2014002393 A1 | | 23 January 2015 |
| | | | | DK 2831111 T3 | | 29 April 2019 |
| | | | | LT 2831111 T | | 10 May 2019 |
| | | | | RS 58732 B1 | | 28 June 2019 |
| | | | | EA 031182 B1 | | 30 November 2018 |
| | | | | NZ 628584 A | | 29 April 2016 |

Form PCT/ISA/210 (patent family annex) (January 2015)

**INTERNATIONAL SEARCH REPORT**
Information on patent family members

International application No.

**PCT/CN2020/079690**

| Patent document cited in search report | | | Publication date (day/month/year) | Patent family member(s) | | | Publication date (day/month/year) |
|---|---|---|---|---|---|---|---|
| | | | | KR | 20140146606 | A | 26 December 2014 |
| | | | | SI | 2831111 | T1 | 28 June 2019 |
| | | | | US | 2013259859 | A1 | 03 October 2013 |
| | | | | PH | 12014502179 | B1 | 10 December 2014 |
| | | | | AU | 2013241769 | B2 | 11 May 2017 |
| | | | | KR | 102020255 | B1 | 10 September 2019 |
| | | | | EP | 2831111 | A1 | 04 February 2015 |
| | | | | MX | 2014011171 | A | 06 March 2015 |
| | | | | PT | 2831111 | T | 31 May 2019 |
| | | | | TR | 201908638 | T4 | 22 July 2019 |
| CN | 102753577 | A | 24 October 2012 | HK | 1172354 | A1 | 24 October 2014 |
| | | | | US | 2010111967 | A1 | 06 May 2010 |
| | | | | EP | 2792687 | A1 | 22 October 2014 |
| | | | | RU | 2011117410 | A | 20 December 2012 |
| | | | | RU | 2014149553 | A | 27 June 2016 |
| | | | | RU | 2542382 | C2 | 20 February 2015 |
| | | | | DK | 2344537 | T3 | 10 February 2014 |
| | | | | EP | 2344537 | B1 | 29 January 2014 |
| | | | | CA | 2739122 | C | 15 May 2018 |
| | | | | JP | 2012504943 | A | 01 March 2012 |
| | | | | ES | 2455217 | T3 | 15 April 2014 |
| | | | | KR | 101345522 | B1 | 31 December 2013 |
| | | | | KR | 20110055726 | A | 25 May 2011 |
| | | | | SI | 2344537 | T1 | 30 May 2014 |
| | | | | HR | P20140376 | T1 | 23 May 2014 |
| | | | | CN | 103936860 | A | 23 July 2014 |
| | | | | CO | 6351794 | A2 | 20 December 2011 |
| | | | | AR | 073775 | A1 | 01 December 2010 |
| | | | | TW | I393571 | B | 21 April 2013 |
| | | | | WO | 2010040508 | A1 | 15 April 2010 |
| | | | | CN | 102753577 | B | 16 July 2014 |
| | | | | IL | 211729 | D0 | 30 June 2011 |
| | | | | AU | 2009301431 | B2 | 31 October 2013 |
| | | | | SG | 195536 | A1 | 30 December 2013 |
| | | | | KR | 20130118994 | A | 30 October 2013 |
| | | | | AU | 2009301431 | A8 | 14 November 2013 |
| | | | | US | 9708396 | B2 | 18 July 2017 |
| | | | | EP | 2781526 | A1 | 24 September 2014 |
| | | | | MX | 2011003190 | A | 27 April 2011 |
| | | | | EP | 2792687 | B1 | 16 May 2018 |
| | | | | PT | 2344537 | E | 09 May 2014 |
| | | | | EP | 2344537 | A1 | 20 July 2011 |
| | | | | HK | 1199265 | A1 | 26 June 2015 |
| | | | | TW | I458492 | B | 01 November 2014 |
| | | | | WO | 2010040508 | A9 | 21 April 2011 |
| | | | | PE | 04252011 | A1 | 01 July 2011 |
| | | | | US | 2017369566 | A1 | 28 December 2017 |
| | | | | CR | 20110166 | A | 03 June 2011 |
| | | | | WO | 2010040508 | A8 | 24 February 2011 |
| | | | | CA | 2739122 | A1 | 15 April 2010 |

Form PCT/ISA/210 (patent family annex) (January 2015)

INTERNATIONAL SEARCH REPORT
Information on patent family members

International application No.

**PCT/CN2020/079690**

| Patent document cited in search report | | | Publication date (day/month/year) | Patent family member(s) | | | Publication date (day/month/year) |
|---|---|---|---|---|---|---|---|
| | | | | RU | 2640253 | C2 | 27 December 2017 |
| | | | | JP | 5368570 | B2 | 18 December 2013 |
| | | | | CY | 1115030 | T1 | 14 December 2016 |
| | | | | ZA | 201102203 | B | 28 December 2011 |
| | | | | US | 8268314 | B2 | 18 September 2012 |
| | | | | MA | 32712 | B1 | 02 October 2011 |
| | | | | SI | EP2344537 | T1 | 30 May 2014 |
| | | | | EC | SP11010969 | A | 31 May 2011 |
| | | | | TW | 201018485 | A | 16 May 2010 |
| CN | 102906114 | A | 30 January 2013 | RU | 2597973 | C2 | 20 September 2016 |
| | | | | NZ | 602320 | A | 28 June 2013 |
| | | | | CL | 2012002662 | A1 | 25 January 2013 |
| | | | | JP | 2013526848 | A | 27 June 2013 |
| | | | | US | 2011236388 | A1 | 29 September 2011 |
| | | | | US | 8945552 | B2 | 03 February 2015 |
| | | | | KR | 20130001291 | A | 03 January 2013 |
| | | | | UA | 110932 | C2 | 10 March 2016 |
| | | | | EP | 2552960 | B1 | 18 April 2018 |
| | | | | BR | 112012024287 | A2 | 01 March 2017 |
| | | | | AU | 2011231580 | A1 | 27 September 2012 |
| | | | | KR | 101484383 | B1 | 22 January 2015 |
| | | | | PE | 20130560 | A1 | 05 May 2013 |
| | | | | PE | 05602013 | A1 | 05 May 2013 |
| | | | | CA | 2793402 | A1 | 29 September 2011 |
| | | | | WO | 2011117329 | A1 | 29 September 2011 |
| | | | | IL | 222116 | A | 28 September 2017 |
| | | | | CA | 2793402 | C | 03 April 2018 |
| | | | | MA | 34172 | B1 | 03 April 2013 |
| | | | | US | 2017240626 | A1 | 24 August 2017 |
| | | | | US | 2015191535 | A1 | 09 July 2015 |
| | | | | TW | 201138820 | A | 16 November 2011 |
| | | | | EC | SP12012177 | A | 30 October 2012 |
| | | | | EP | 2552960 | A1 | 06 February 2013 |
| | | | | CN | 102906114 | B | 15 March 2017 |
| | | | | MX | 2012010937 | A | 15 October 2012 |
| | | | | CR | 20120464 | A | 05 October 2012 |
| | | | | SG | 184295 | A1 | 29 November 2012 |
| | | | | AR | 080794 | A1 | 09 May 2012 |
| | | | | TW | I426920 | B | 21 February 2014 |
| | | | | JP | 5707482 | B2 | 30 April 2015 |
| | | | | RU | 2012143793 | A | 10 May 2014 |
| | | | | CO | 6630087 | A2 | 01 March 2013 |
| | | | | AU | 2011231580 | B2 | 29 January 2015 |
| WO | 2018037000 | A1 | 01 March 2018 | IL | 264962 | D0 | 30 May 2019 |
| | | | | US | 2019194308 | A1 | 27 June 2019 |
| | | | | KR | 20190039577 | A | 12 April 2019 |
| | | | | CA | 3034574 | A1 | 01 March 2018 |
| | | | | EP | 3504237 | A1 | 03 July 2019 |
| | | | | AU | 2017315075 | A1 | 04 April 2019 |
| | | | | CN | 109863171 | A | 07 June 2019 |

Form PCT/ISA/210 (patent family annex) (January 2015)

**INTERNATIONAL SEARCH REPORT**
Information on patent family members

International application No.

**PCT/CN2020/079690**

| Patent document cited in search report | Publication date (day/month/year) | Patent family member(s) | | Publication date (day/month/year) |
|---|---|---|---|---|
| | | JP 2019528694 A | | 17 October 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1998045332 A
- WO 2007095338 A2
- WO 201004058 A
- CN 102250247 A
- WO 2011117329 A
- US 5641870 A
- US 20080014196 A
- CN 105143262 A

### Non-patent literature cited in the description

- *J. biol. chem,* 1968, vol. 243, 3558
- **MAISONPIERRE, P.C. et al.** *Science,* 1997, vol. 277, 55-60
- **CHEUNG, A.H. et al.** *Genomics,* 1998, vol. 48, 389-91
- **YANCOPOULOS, G.D. et al.** *Nature,* 2000, vol. 407, 242-48
- **KIM, I. et al.** *FEBS Let,* 1999, vol. 443, 353-56
- **KIM, I. et al.** *J Biol Chem,* 1999, vol. 274, 26523-28
- **DAVIS, S. et al.** *Cell,* 1996, vol. 87, 1161-69
- **LEUNG, D.W. et al.** *Science,* 1989, vol. 246, 1306-9
- **KECK, P.J. et al.** *Science,* 1989, vol. 246, 1309-12
- **CONNOLLY, D.T. et al.** *J. Biol. Chem.,* 1989, vol. 264, 20017-24
- **FERRARA, N.** *Endocr. Rev.,* 1997, vol. 18, 4-25
- **BERKMAN, R.A.** *J. Clin. Invest.,* 1993, vol. 91, 153-159
- **BROWN, L.F. et al.** *Human Pathol,* 1995, vol. 26, 86-91
- **BROWN, L.F. et al.** *Cancer Res,* 1993, vol. 53, 4727-4735
- **MATTERN, J. et al.** *Brit. J. Cancer,* 1996, vol. 73, 931-934
- **DVORAK, H.F. et al.** *Am. J. Pathol.,* 1995, vol. 146, 1029-1039
- **HAMERS-CASTERMAN C ; ATARHOUCH T ; MUYLDERMANS S ; ROBINSON G ; HAMERS C ; SONGA EB ; BENDAHMAN N ; HAMERS R.** Naturally occurring antibodies devoid of light chains. *Nature,* 1993, vol. 363, 446-448
- **KABAT, E. A. et al.** Sequences of Proteins of Immunological Interest. 1991
- **BIRD et al.** *Science,* 1988, vol. 242, 423-426
- **HUSTON et al.** *Proc. Natl. Acad. Sci USA,* 1988, vol. 85, 5879-5883
- **ZAPATA et al.** *Protein Eng,* 1995, vol. 8 (10), 1057-1062
- **HOLLIGER et al.** *Proc. Natl. Acad. Sci. USA,* 1993, vol. 90, 6444-6448
- **ALFTHAN et al.** *Protein Eng,* 1995, vol. 8, 725-731
- **CHOI et al.** *Eur. J. Immunol.,* 2001, vol. 31, 94-106
- **HU et al.** *Cancer Res,* 1996, vol. 56, 3055-3061
- **KIPRIYANOV et al.** *J. Mol. Biol.,* 1999, vol. 293, 41-56
- **ROOVERS et al.** *Cancer Immunol. Immunother.,* 2001, vol. 50, 51-59
- *Protein Engineering,* 1994, vol. 7, 697
- **KABAT et al.** Sequences of Proteins of Immunological Interest. Public Health Service, National Institutes of Health, 1991
- **KABAT et al.** Sequences of Proteins of Immunological Interest. National Institutes of Health, 1991
- **AL-LAZIKANI et al.** *J. Molec. Biol.,* 1997, vol. 273, 927-948
- **AL-LAZIKANI et al.** *JMB,* 1997, vol. 273, 927-948
- **LEFRANC M. P.** *Immunologist,* 1999, vol. 7, 132-136
- **LEFRANC, M. P. et al.** *Dev. Comp. Immunol.,* 2003, vol. 27, 55-77
- Epitope Mapping Protocols in Methods in Molecular Biology. 1996, vol. 66
- **STAHLI et al.** *Methods in Enzymology,* 1983, vol. 9, 242-253
- **KIRKLAND et al.** *J. Immunol.,* 1986, vol. 137, 3614-3619
- **HARLOW ; LANE.** Antibodies, A Laboratory Manual. Cold Spring Harbor Press, 1988
- **MOREL et al.** *Molec. Immunol.,* 1988, vol. 25, 7-15
- **CHEUNG et al.** *Virology,* 1990, vol. 176, 546-552
- **MOLDENHAUER et al.** *Scand. J. Immunol.,* 1990, vol. 32, 77-82

- Antibody Experiment Technical Guide
- The Immunoglobulin Facts Book. 2001
- **WATSON et al.** Molecular Biology of the Gene. The Benjamin/Cummings Pub. Co, 1987, 224
- **ALTSCHUL, S.F. et al.** *J. Mol. Biol.,* 1990, vol. 215, 403-410
- **GISH, W. et al.** *Nature Genet,* 1993, vol. 3, 266-272
- **MADDEN, T.L. et al.** *Meth. Enzymol.,* 1996, vol. 266, 131-141
- **ALTSCHUL, S.F. et al.** *Nucleic Acids Res.,* 1997, vol. 25, 3389-3402
- **ZHANG, J. et al.** *Genome Res,* 1997, vol. 7, 649-656
- *CHEMICAL ABSTRACTS,* 347396-82-1
- *WHO Drug Information,* 2015, vol. 29 (1
- *CHEMICAL ABSTRACTS,* 216974-75-3